# EUROPEAN PATENT APPLICATION

(11) **EP 4 534 147 A1**
(43) Date of publication of application: **09.04.2025**
(21) Application number: 23201876.2
(22) Date of filing: 05.10.2023
(51) Int. Cl.: A61P 31/00, A61P 35/00, A61P 37/00, C07D 403/14, A61K 31/4184

(54) **STING ANTAGONIST COMPOUNDS**

(71) Applicant: Sulis Therapeutics ApS, 8000 Aarhus C (DK)
(72) Inventor: SENSFUSS, Ulrich, 2720 Vanløse (DK); BETHELL, Richard Charles, 11230 Stockholm (SE)
(74) Representative: Mewburn Ellis LLP

(57) **Abstract**

The present invention relates to STING antagonists comprising a benzimidazole structure, which can be used to treat diseases or conditions associated with overactivation of STING.

## Description

### Field of the Invention

The present invention relates to benzimidazole compounds which act as STING antagonists. The benzimidazole compounds can be used to treat diseases or conditions associated with overactivation of STING.

### Background

Stimulator of Interferon Genes (STING) is an intracellular adaptor protein which plays an important role in innate immunity. STING induces type I interferon (IFN) production in addition to other proinflammatory mediators such as cytokines in response to infection or cellular damage. Cytosolic STING is activated upon binding cyclic dinucleotides (CDNs), which can be derived from bacterial sources or 2',3'-cyclic GMP-AMP (2',3'-cGAMP) generated by cyclic GMP-AMP synthase (cGAS), an enzyme that is activated by binding to double-stranded DNA in cytoplasm. In addition to the canonical CDN-dependent activation, STING can also be activated constitutively in a non-canonical CDN-independent manner, for example through gain-of-function mutations (Decout et al., 2021).

Activation of STING leads to up-regulation of type I interferon production. Activation of type I interferon production is an approach which has been explored in the treatment of some cancers, and numerous STING agonists have been designed for this purpose. Examples of STING agonists include benzimidazoles, for example as described in WO2017/175147, WO2019/069270, WO2020/132582, WO2020/132566, and WO2020/132549 the content of each of which is hereby incorporated by reference.

In contrast, many monogenic autoinflammatory syndromes, autoimmune diseases, inflammatory diseases, neurological disorders, metabolic diseases, cardiovascular diseases, and cancers have been linked to the overexpression of STING and activation of the cyclic GMP-AMP synthase pathway.

Monogenic autoinflammatory diseases are associated with mutations in genes that participate in innate immunity or that control innate immune homeostasis. STING-associated vasculopathy with onset in infancy (SAVI) is one such disease. SAVI is characterized by recurrent fevers, ulcerative skin lesions, vasculitis and interstitial lung disease and is mostly caused by autosomal dominant mutations in STING1 (Decout et al., 2021).

Another monogenic autoinflammatory disease is COPA syndrome, a rare early-onset autosomal dominant disease which can affect the lungs, kidneys, and joints, caused by missense mutations in the COPA gene, which encodes the COPα protein subunit of the COPI complex. This mutation impairs the binding and sorting of proteins targeted for ER retrieval, and promotes ligand-independent activation of STING-mediated signaling (Decout et al., 2021).

Aicarrdi-Goutières Syndrome (AGS) is a further example of a monogenic autoinflammatory disease, which is characterized by early onset of progressive encephalopathy and skin lesions (known as chilblains). Mutations in a number of genes linked to this condition and associated with nucleic acid sensing or metabolism - including TREX1, the genes encoding the three RNase H2 endonuclease subunits, RNASEH2A, RNASEH2B and RNASEH2C, and the deoxynucleoside triphosphate triphosphohydrolase SAMHD1 - have been associated with disturbed self-DNA metabolism and constitutive activation of the cGAS-STING pathway. TREX1 deficiency is therefore another disease linked to STING and activation of the cyclic GMP-AMP synthase pathway (Decout et al., 2021). Similarly, DNase II deficiency is a further autoinflammatory disease linked to STING and activation of the cyclic GMP-AMP synthase pathway (Rodero et al., 2017). Familial chilblain lupus is a further monogenic autoinflammatory disease which is a form of cutaneous lupus erythematosus caused by a gain-of-function in STING, or loss-of-function mutations in the nuclease genes TREX1 and SAMHD1 (Konig et al., 2017).

The autoimmune disease systemic lupus erythematosus (SLE) has also been linked with cGAS pathway activity and type I interferon induction via STING, providing a mechanism for how cGAS-STING may amplify or accelerate disease symptoms. Sjögren's syndrome is a further autoimmune disorder characterized by an increased type 1 interferon gene signature (Decout et al., 2021).

Inflammation is also a prominent hallmark of several neurodegenerative diseases, and elevated levels of type I interferons and contributions of STING have also been observed in models and disease samples of Huntington disease (Decout et al., 2021).

Other diseases and conditions linked to STING and activation of the cyclic GMP-AMP synthase pathway include rheumatoid arthritis, systemic sclerosis, ischaemic brain injury, Parkinson's disease, general neurodegeneration, amyotrophic lateral sclerosis and frontotemporal dementia, age-dependent macular degeneration, traumatic brain injury, non-alcoholic steatohepatitis, alcoholic liver disease, acute pancreatitis, silica-induced fibrosis, sepsis, cardiovascular diseases, myocardial infarction, chronic heart failure, colorectal cancer, skin cancer, metastases, senescence, and aging (Decout et al., 2021).

The identification of the link to these diseases and conditions has led to an increased focus on STING as a potential therapeutic target. The search for STING inhibitors has yielded several small molecules targeting STING which have anti-inflammatory effects. So far there have been two main categories of STING inhibitor design. The first uses molecules which bind non-covalently STING, putatively to the cyclic dinucleotide binding site of STING, to act as competitive antagonists of STING activators. Such compounds have generally modest biological activities. Secondly, inhibitors have been designed that bind covalently to cysteine residues in the transmembrane domain of STING in order to prevent their palmitoylation, which is linked to STING activation. However, cysteine-reactive molecules are prone to being non-specific, which is due to covalent binding to cysteine in other non-target proteins, meaning STING inhibitors which bind covalently have a higher risk of off-target activities when used therapeutically.

An ongoing need exists in the art for effective treatments of disease associated with unwanted or uncontrolled activation of STING. However, STING antagonists rely on high systemic drug exposures to maintain sufficient STING inhibition, which increases the risk of unwanted off-target effects. Furthermore, existing STING antagonists are less effective in the treatment of diseases driven by constitutive (agonist-free) activation of STING.

Until now, the ability to inhibit STING in a targeted manner using small molecule therapeutic agents which do not bind STING covalently has yet to be achieved.

Transforming agonists into potent selective antagonists is more challenging than would be expected and there is a need to identify novel low molecular weight compounds which can have this unexpected mode of action. Hence, there is an unmet need for potent selective STING inhibitors that do not activate the STING pathway and that do not cause adverse effects by interacting with other biomolecules than STING, e.g. due to chemical reactivity.

STING agonists activate the immune response through the activation of certain immune cells which induces the expression of pro-inflammatory cytokines and chemokines, promotes tumor-associated antigen (TAA) processing and presentation by dendritic cells (DCs) and leads to an antigen-specific T-cell mediated immune response against cancer cells.

Benzimidazoles have been investigated for their use as modulators of STING, however, such compounds sometimes act as STING agonists and sometimes as antagonists.

There are different sites or targets at which a low molecular weight inhibitor could modulate the cGAS STING pathway. Preventing cGAMP transport might overlap, in some cases, with the effect of inhibition of STING or cGAS. There have been large efforts to develop small molecule STING inhibitors , which is, in fact, the "only" site in this pathway for which both antagonists and agonists have been identified.

The present invention has been devised in light of the above considerations.

### Summary of the Invention

A first aspect provides a compound of Formula (I-b): 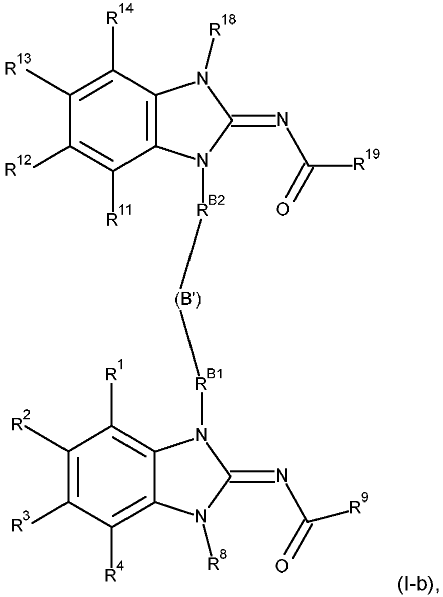 or a tautomer, prodrug or pharmaceutically acceptable salt thereof,
wherein R⁹ and R¹⁹ are each independently selected from an optionally substituted C₅₋₁₀ cycloalkyl, optionally substituted C₆₋₁₀ carboaryl, optionally substituted C₅₋₁₀ heteroaryl or optionally substituted C₅₋₁₀ heterocyclyl, wherein the optional substituents are independently selected from H, halogen, C₃₋₅ cycloalkyl, optionally substituted C₁₋₄ alkyl wherein said optionally substituted C₁₋₄ alkyl is optionally substituted by one or more substituents independently selected from halogen, halo(C₁₋₄ alkyl), OH, NH₂, C₁₋₄ alkoxy, COzH, CO₂(C₁₋₆ alkyl), N(C₁₋₆ alkyl)₂, OC(=O)NH₂ and C(=O)NH₂;
R¹ and R¹¹ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are independently selected from OH, -O-P(O)(OH)₂, C₁₋₄ alkoxy, NH₂, N(C₁₋₆ alkyl)₂, CO₂(C₁₋₆ alkyl) and halogen;
R³ and R¹³ are each independently selected from H, C(=O)NH₂; optionally substituted -C(=O)NH(C₁₋₆ alkyl), optionally substituted C₁₋₆ alkoxy or optionally substituted CO₂C₁₋₆ alkyl, wherein the optional substituents are independently selected from OH, -O-P(O)(OH)₂, C₁₋₄ alkoxy, N(C₁₋₆ alkyl)₂, NH₂, CO₂(C₁₋₆ alkyl) and halogen;
R⁴ and R¹⁴ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are selected from OH, -O-P(O)(OH)₂, C₁₋₄alkoxy, N(C₁₋₆ alkyl)₂, NH₂, CO₂(C₁₋₆ alkyl) and halogen;
R² and R¹² are each independently selected from H, halogen, OH, optionally substituted C₁₋₆ alkyl, optionally substituted C₁₋₆ alkoxy, optionally substituted CO₂(C₁₋₆ alkyl), and optionally substituted C₁₋₆ alkylamino wherein the optional substituents are selected from OH, -O-P(O)(OH)₂, C₁₋₄ alkoxy, N(C₁₋₆ alkyl)₂, NH₂, CO₂(C₁₋₆ alkyl) and halogen;
R⁸ and R¹⁸ are each independently selected from H, and C₁₋₄ alkyl;
R^{B1} and R^{B2} are each independently selected from (-CH₂-)₁₋₂ or a bond and B' taken together with R^{B1} and R^{B2} forms a linking group, wherein B' is: 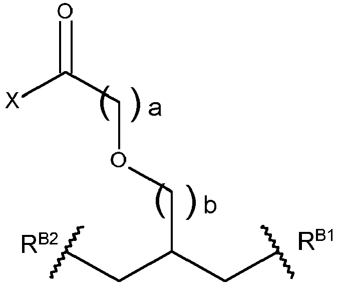
   wherein a is 0, 1, or 2;
   b is 0 or 1;
   wherein X is -NR^{B3}R^{B4} or X is -OR^{B5},
   wherein R^{B3}, R^{B4} and R^{B5} are each independently selected from:
      i) hydrogen;
      ii) optionally substituted C₁₋₈ alkyl;
      iii) -(CH₂-(CH₂)₁₋₂₋O)₁₋₃-(CH₂)₁₋₃-H;
      iv) optionally substituted C₂₋₈ alkenyl;
      v) optionally substituted C₂₋₈ alkynyl;
      vi) optionally substituted C₆₋₁₀ carboaryl;
      vii) optionally substituted C₃₋₁₀ cycloalkyl;
      viii) optionally substituted C₅₋₁₂ heteroaryl;
      ix) optionally substituted C₄₋₁₂ heterocyclyl;
      x) optionally substituted C₃₋₁₀ heteroalkyl;
      xi) optionally substituted C₃₋₁₀ heteroalkenyl;
      xii) optionally substituted C₃₋₁₀ heteroalkynyl;
   or wherein R^{B3} and R^{B4} together with N form an optionally substituted C₄₋₁₂ heterocyclyl or C₅₋₁₂ heteroaryl;
   wherein the optional substituents at R^{B3}, R^{B4} and R^{B5} are each R^{B6},
   wherein each R^{B6} is independently selected from:
      i) hydroxy;
      ii) oxo;
      iii) carboxy;
      iv) halogen;
      v) trifluoromethyl;
      vi) trifluoromethoxy;
      vii) -NH₂;
      viii) optionally substituted C₆₋₁₀ aryl;
      ix) optionally substituted C₃₋₆ cycloalkyl;
      x) optionally substituted C₄₋₁₀ heterocyclyl;
      xi) optionally substituted C₅₋₁₂ heteroaryl;
      xii) optionally substituted C₁₋₄ alkyl;
      xiii) optionally substituted C₂₋₄ alkenyl;
      xiv) optionally substituted C₂₋₄ alkynyl;
      xv) optionally substituted C₁₋₄ alkoxy;
      xvi) optionally substituted C₃₋₄ alkenoxy;
      xvii) optionally substituted C₃₋₄ alkynoxy;
      xviii) optionally substituted -N(C₁₋₄ alkyl)₂,
      xix) optionally substituted -NH(C₁₋₄alkyl);
      xx) optionally substituted C₃₋₆ heteroalkyl;
      xxi) optionally substituted C₄₋₆ heteroalkenyl;
      xxii) optionally substituted C₄₋₆ heteroalkynyl;
      xxiii) optionally substituted -C(=O)-(C₆₋₁₀ aryl);
      xxiv) optionally substituted -C(=O)-(C₃₋₆ cycloalkyl);
      xxv) optionally substituted -C(=O)-(C₄₋₁₀ heterocyclyl);
      xxvi) optionally substituted -C(=O)-(C₄₋₁₀ heteroaryl);
      xxvii) optionally substituted -C(=O)-(C₁₋₄ alkyl);
      xxviii) optionally substituted -C(=O)-(C₂₋₄ alkenyl);
      xxix) optionally substituted -C(=O)-(C₂₋₄ alkynyl);
      xxx) optionally substituted -C(=O)-(C₁₋₄ alkoxy);
      xxxi) optionally substituted -C(=O)-(C₃₋₄ alkenoxy);
      xxxii) optionally substituted -C(=O)-(C₃₋₄ alkynoxy);
      xxxiii) optionally substituted -C(=O)-N(C₁₋₄ alkyl)₂,
      xxxiv) optionally substituted -C(=O)-NH(C₁₋₄ alkyl);
      xxxv) optionally substituted -C(=O)-(C₃₋₆ heteroalkyl);
      xxxvi) optionally substituted -C(=O)-(C₄₋₆ heteroalkenyl);
      xxxvii) optionally substituted -C(=O)-(C₄₋₆ heteroalkynyl);
      xxxviii) optionally substituted -CH_{Z}-(C₆₋₁₀ aryl);
      xxxix) optionally substituted -CH₂-(C₄₋₁₀ heterocyclyl);
      xi) optionally substituted -(C=O)NH-CH₂-(C₆₋₁₀ aryl); and
      xii) optionally substituted -(C=O)NH-CH₂-(C₅₋₁₂ heteroaryl);
   wherein the optional substituents at R^{B6} are each independently selected from hydroxy, oxo, carboxy, halogen, trifluoromethyl, trifluoromethoxy, -NH₂, -NH(C₁₋₂ alkyl), -N(C₁₋₂ alkyl)₂, methyl, ethyl, CH₂OCH₃, methoxy, and ethoxy.

The compounds of the invention are STING binders that exhibit STING antagonist activity. STING binding can be determined by a time-resolved FRET assay. For instance, a STING protein having a His-tag can be used. A D2-labeled active site probe can be used to accept 620 nm emission from Anti-HIS-Tb-STING. Increased fluorescence is measured at 665 nm. Molecules that compete for the probe binding site will result in a lower 665 nm signal. The assay can be performed in low-volume white 96-well plates containing 5 µL compounds in an appropriate diluent buffer. Following 60 minutes incubation at room temperature in the dark, fluorescence emission at 665 nm is measured following 330 nm xenon flash excitation. pIC₅₀ values can be determined using a standard four parameter curve fit. Preferably, the compounds of the invention have an IC₅₀ of less than 3 nM, less than 2 nM, less than 1.5 nM, less than 1.2 nM or less than 1.0 nM, as measured by the FRET assay.

STING antagonist activity can be determined by a human fibroblast antagonist assay. For this, human skin fibroblasts are pre-treated for 2h with a serial dilution of the compound, and are then stimulated for 20 hours with 300 nM STING agonist (e.g. from GSK, CAS number: 2138299-34-8). Supernatants are then harvested and assessed for IFN-a/b release, which indicates STING activation. IFN-a/b concentration in the supernatant is determined, e.g. using an IFN-α/β bioassay. IC₅₀ values are calculated using a non-linear regression fitting, variable slope (4 parameters). Preferably, the compounds of the invention have an IC₅₀ of less than 20 µM, less than 15 µM, less than 13 µM, less than 10 µM, less than 8 µM, less than 7 µM, as measured by the human fibroblast antagonist assay.

A second aspect provides a pharmaceutical composition comprising the compound of the first aspect and a pharmaceutically acceptable diluent, carrier or excipient.

The third aspect provides the compound of the first aspect for use in a method of therapy. The third aspect provides the compound of the first aspect for use in a method of therapy wherein the disorder or condition is associated with overactivation of STING. The disorder or condition can be monogenic autoinflammatory syndrome, STING-associated vasculopathy with onset in infancy (SAVI), Aicardi-Goutieres Syndrome, COPA Syndrome, Familial Chilblain Lupus, DNase II deficiency, or TREX1 deficiency, an autoimmune disease, systemic lupus erythematosus (SLE) or Sjögren's syndrome or a cardiovascular disease. The third aspect also provides the use of a compound of the first aspect in the manufacture of a medicament for treating a disorder or condition is associated with overactivation of STING. The third aspect also provides a compound of the first aspect for use in the treatment of a disorder or condition is associated with overactivation of STING. The third aspect also provides a method of treating a disorder or condition is associated with overactivation of STING comprising administering a therapeutically effective amount of a compound of the first aspect or a composition according to the second aspect to a patient in need thereof. The disorder or condition can be monogenic autoinflammatory syndrome, STING-associated vasculopathy with onset in infancy (SAVI), Aicardi-Goutieres Syndrome, COPA Syndrome, Familial Chilblain Lupus, DNase II deficiency, or TREX1 deficiency, an autoimmune disease, systemic lupus erythematosus (SLE), Sjögren's syndrome or a cardiovascular disease.

It has been surprisingly found that the compounds of the present invention are STING antagonists. The present compounds have shown surprisingly good STING binding and no STING agonist effect. Prior art compounds have not been shown to only have antagonist activity. It is therefore surprising that the present compounds have STING antagonist activity and not agonist activity. The fully symmetrical structure makes the chemical synthesis of the compounds easier.

The invention includes the combination of the aspects and preferred features described except where such a combination is clearly impermissible or expressly avoided.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

### Definitions

### Substituents

The phrase "optionally substituted" as used herein, pertains to a parent group which may be un substituted or which may be substituted.

Unless otherwise specified, the term "substituted" as used herein, pertains to a parent group which bears one or more substituents. The term "substituent" is used herein in the conventional sense and refers to a chemical moiety which is covalently attached to, or if appropriate, fused to, a parent group. A wide variety of substituents are well known, and methods for their formation and introduction into a variety of parent groups are also well known.

Examples of substituents are described in more detail below.

Unless otherwise stated, halogen or halo is selected from chloro (Cl), fluoro (F), bromo (Br) and iodo (I), such as fluoro.

Hydroxy: -OH.

Oxo: =O (oxygen double bonded to the rest of the molecule).

C₁₋₁₂ hydrocarbon: The term "C₁₋₁₂ hydrocarbon" as used herein pertains to a monovalent moiety obtained by removing a hydrogen atom from a carbon atom of a hydrocarbon compound having from 1 to 12 carbon atoms, which may be aliphatic or alicyclic, which may be saturated or unsaturated (e.g. partially unsaturated, fully unsaturated) and may also be branched. Thus, the term "hydrocarbon" includes the terms alkyl, alkenyl, alkynyl, cycloalkyl, etc., discussed below.

C₁₋₈ alkyl: The term "C₁₋₈ alkyl" as used herein, pertains to a monovalent moiety obtained by removing a hydrogen atom from a carbon atom of a hydrocarbon compound having from 1 to 8 carbon atoms, which may be aliphatic or alicyclic, and which are saturated and may also be branched. The term "C₁₋₆ alkyl" as used herein, pertains to a monovalent moiety obtained by removing a hydrogen atom from a carbon atom of a hydrocarbon compound having from 1 to 6 carbon atoms, which may be aliphatic or alicyclic, and which are saturated. The term "C₁₋₄ alkyl" as used herein, pertains to a monovalent moiety obtained by removing a hydrogen atom from a carbon atom of a hydrocarbon compound having from 1 to 4 carbon atoms, which may be aliphatic or alicyclic, and which are saturated. The term "C₁₋₈ alkyl" as used herein encompasses both linear and branched alkyl groups. Thus, the term "alkyl" includes the sub-classes cycloalkyl, discussed below.

Examples of saturated alkyl groups include, but are not limited to, methyl (C₁), ethyl (C₂), propyl (C₃), butyl (C₄), pentyl (C₅) hexyl (C₆), heptyl (C₇), octyl (C₈).

Examples of saturated linear alkyl groups include, but are not limited to, methyl (C₁), ethyl (C₂), n-propyl (C₃), n-butyl (C₄), n-pentyl (amyl) (C₃) and n-hexyl (C₆).

Examples of saturated branched alkyl groups include iso-propyl (C₃), iso-butyl (C₄), sec-butyl (C₄), tert-butyl (C₄), iso-pentyl (C₅), and neo-pentyl (C₅).

C₃₋₁₀ cycloalkyl: The term "C₃₋₁₀ cycloalkyl" as used herein, pertains to an alkyl group which is also a cyclyl group; that is, a monovalent moiety obtained by removing a hydrogen atom from a ring atom of a cyclic hydrocarbon (carbocyclic) compound, which moiety has from 3 to 10 carbon atoms, including from 3 to 10 ring atoms. The carbocyclic ring may be saturated or unsaturated and may be bridged or unbridged. The ring may be a fused ring or a single ring.

Examples of cycloalkyl groups include, but are not limited to, those derived from:
saturated monocyclic hydrocarbon compounds:
   cyclopropane (C₃), cyclobutane (C₄), cyclopentane (C₅), cyclohexane (C₆), cycloheptane (C₇), methylcyclopropane (C₄), dimethylcyclopropane (C₅), methylcyclobutane (C₅), dimethylcyclobutane (C₆), methylcyclopentane (C₆), dimethylcyclopentane (C₇) methylcyclohexane (C₇), cyclooctane (C₈), cyclononane (C₉) and cyclodecane (C₁₀);
unsaturated monocyclic hydrocarbon compounds:
   cyclopropene (C₃), cyclobutene (C₄), cyclopentene (C₅), cyclohexene (C₆), methylcyclopropene (C₄), dimethylcyclopropene (C₅), methylcyclobutene (C₅), dimethylcyclobutene (C₆), methylcyclopentene (C₆), dimethylcyclopentene (C₇) and methylcyclohexene (C₇); and
saturated polycyclic hydrocarbon compounds:
   norcarane (C₇), norpinane (C₇), norbornane (C₇).

C₂₋₈ alkenyl: The term "C₂₋₈ alkenyl" as used herein, pertains to a non-aromatic hydrocarbon group having one or more carbon-carbon double bonds.

Examples of unsaturated alkenyl groups include, but are not limited to, ethenyl (vinyl, -CH=CH₂), 1-propenyl (-CH=CH-CH₃), 2-propenyl (allyl, -CH-CH=CH₂), isopropenyl (1-methylvinyl, -C(CH₃)=CH₂), butenyl (C₄), pentenyl (C₅), and hexenyl (C₆).

C₂₋₈alkynyl: C₂₋₈ alkynyl: The term "C₂₋₈ alkynyl" as used herein, pertains to a hydrocarbon group having one or more carbon-carbon triple bonds.

Examples of unsaturated alkynyl groups include, but are not limited to, ethynyl (-C≡CH), 1-propynyl (-C≡CCH₃) and 2-propynyl (propargyl, -CH₂C≡CH).

Ether: -OR, or -R-O-R- wherein R is an ether substituent, for example, a C₁₋₁₂ hydrocarbon group (also referred to as a C₁₋₁₂ alkoxy group, discussed below), a C₃₋₂₀ heterocyclyl group (also referred to as a C₃₋₂₀ heterocyclyloxy group), or a C₅₋₂₀ aryl group (also referred to as a C₅₋₂₀ aryloxy group), preferably a C₁₋₇alkyl group.

C₁₋₆ alkoxy: The term C₁₋₆ alkoxy as used herein, pertains to an OR group, wherein R is an C₁₋₆ alkyl group. Examples of C₁₋₆ alkoxy groups include, but are not limited to, OMe, OEt (ethoxy), -O(nPr) (n-propoxy), -O(iPr) (isopropoxy), O(nBu) (n-butoxy), O(sBu) (sec-butoxy), O(iBu) (isobutoxy), and O(tBu) (tert-butoxy).

C₃₋₄ alkenoxy: The term C₃₋₄ alkenoxy as used herein, pertains to an OR group, wherein R is an C₃₋₄ alkenyl group. Examples of C₃₋₄ alkenoxy include O-CH=CH-CH₃, O-C(CH₃)=CH₂, or O- butenyl.

C₃₋₄ alkynoxy: The term C₃₋₄ alkynoxy as used herein, pertains to an OR group, wherein R is a C₃₋₄alkynyl group. Examples of C₃₋₄ alkynoxy groups include O-CHzC=CH, OCH₂CH₂C≡CH or O-C≡CCH₃.

C₃₋₁₀ heteroalkyl; The term C₃₋₁₀ heteroalkyl as used herein pertains to a C₃₋₁₀ alkyl group which chain is interrupted by 1 to 3 heteroatoms selected from O, S and N. In other words, a C₃₋₁₀ alkyl chain, which chain includes 1 to 3 heteroatoms selected from O, S and N. Examples of C₃₋₁₀ heteroalkyl groups include but are not limited to CH₂CH₂OCH_{3,} CH₂CH₂OCH₂CH_{3,} CH₂CH₂OCH₂CH₂CH_{3,} CH₂OCH₂CH_{3,} CH₂OCH₂CH₂CH_{3,} CH₂OCH₂CH₂CH₂CH_{3,} CH₂CH₂OCH₂CH₂CH₂CH₂CH_{3,} CH₂OCH₂CH₂CH₂CH₂CH_{3,} CH₂CH₂CH₂OCH_{3,} CH₂CH₂CH₂CH₂CH₂CH₂OCH_{3,} CH₂CH₂SCH_{3,} CH₂CH₂SCH₂CH_{3,} CH₂CH₂SCH₂CH₂CH_{3,} CH₂SCH₂CH_{3,} CH₂SCH₂CH₂CH_{3,} CH₂SCH₂CH₂CH₂CH_{3,} CH₂CH₂SCH₂CH₂CH₂CH₂CH_{3,} CH₂SCH₂CH₂CH₂CH₂CH_{3,} CH₂CH₂CH₂SCH_{3,} CH₂CH₂CH₂CH₂CH₂CH₂SCH_{3,} CH₂CH₂NHCH_{3,} CH₂CH₂NHCH₂CH_{3,} CH₂CH₂NHCH₂CH₂CH_{3,} CH₂NHCH₂CH_{3,} CH₂NHCH₂CH₂CH_{3,} CH₂NHCH₂CH₂CH₂CH_{3,} CH₂CH₂NHCH₂CH₂CH₂CH₂CH_{3,} CH₂NHCH₂CH₂CH₂CH₂CH_{3,} CH₂CH₂CH₂NHCH_{3,} CH₂CH₂CH₂CH₂CH₂CH₂NHCH_{3,} CH₂CH₂N(CH₂)_{2,} CH₂N(CH₂CH₂)₂ CH₂N(CH₂CH₂CH₂)₂, CHzNHCHzCHzCHzOCHz, CH₂NHCH₂CH₂OCH_{3,} CH₂NHCH₂CH₂OCH₂CH_{3,} CH₂CH₂NHCH₂CH₂CH₂OCH₂CH_{3,} CH₂NHCH₂CH₂CH₂OCH₂CH_{3,} CH₂OCH₂CH₂NHCH_{3,} CH₂CH₂CH₂OCH₂CH₂CH₂NHCH_{3,} CHzOCHzN(CHz)z, CHzN(CHzCHzOCHz)z, CH₂NHCH₂SCH_{3,} CH₂NHCH₂CH₂SCH_{3,} CH₂NHCH₂CH₂SCH₂CH_{3,} CH₂CH₂NHCH₂CH₂CH₂SCH₂CH_{3,} CH₂NHCH₂CH₂SCH₂CH₂CH_{3,} CH₂SCH₂CH₂NHCH_{3,} CH₂CH₂SCH₂CH₂CH₂CH₂NHCH_{3,} CH₃NHCH₂CH₂CH₂OCH_{3.}

C₃₋₁₀ heteroalkenyl; The term C₃₋₁₀ heteroalkenyl as used herein pertains to a C₃₋₁₀ alkenyl group which chain is interrupted by 1 to 3 heteroatoms selected from O, S and N. In other words, a C₃₋₁₀ alkenyl chain, which chain includes 1 to 3 heteroatoms selected from O, S and N. Examples of C₃₋₁₀ heteroalkenyl groups include but are not limited to CH=CHOCH_{3,} CH₌CHOCH₂CH_{3,} CH₌CHOCH₂CH₂CH_{3,} CH₂OCH₌CH_{2,} CH₂OCH₂CH=CH_{2,} CH₂OCH₂CH=CHCH_{3,} CH₂CH₂OCH₂CH₌CHCH₂CH_{3,} CH₂OCH₂CH₂CH₂CH₌CH_{2,} CH=CHCH₂OCH_{3,} CH₂CH₂CH₌CHCH₂CH₂OCH_{3,} CH=CHSCH_{3,} CH₌CHSCH₂CH_{3,} CH₌CHSCH₂CH₂CH_{3,} CH₂SCH₌CH_{2,} CH₂SCH₂CH₌CH_{2,} CH₂SCH₂CH=CHCH_{3,} CH₂CH₂SCH₂CH=CHCH₂CH_{3,} CH₂SCH₂CH₂CH₂CH₌CH_{2,} CH=CHCH₂SCH_{3,} CH₂CH₂CH₌CHCH₂CH₂SCH_{3,} CH₌CHNHCH_{3,} CH₌CHNHCH₂CH_{3,} CH=CHNHCH₂CH₂CH_{3,} CH₂NHCH=CH_{2,} CH₂NHCH₂CH₌CH_{2,} CH₂NHCH₂CH₂CH=CH_{2,} CH₂CH₂NHCH₂CH₂CH=CHCH_{3,} CH₂NHCH₂CH₂CH=CHCH_{3,} CH₂CH₌CHNHCH₃, CH₂CH₌CHCH₂CH₂CH₂NHCH_{3,} CH₌CHN(CH₂)₂, CH₂N(CH=CH)₂ CH₂N(CH₂CH₌CH)₂, CH₂NHCH₂OCH₂CH=CH_{2,} CH₂OCH₂NHCH₂CH₂CH₌CHCH_{3,} CH₂NHCH₂OCH₂CH=CHCH_{3,} CH₂CH₌CHNHOCH_{3,} CH₂CH₌CHCH₂OCH₂CH₂NHCH_{3.}

C₃₋₁₀ heteroalkynyl; The term C₃₋₁₀ heteroalkynyl as used herein pertains to a C₃₋₁₀ alkynyl group which chain is interrupted by 1 to 3 heteroatoms selected from O, S and N. In other words, a C₃₋₁₀ alkynyl chain, which chain includes 1 to 3 heteroatoms selected from O, S and N. Examples of C₃₋₁₀ heteroalkynyl groups include but are not limited to CH≡COCH_{3,} CH≡COCH₂CH_{3,} CH≡COCH₂CH₂CH_{3,} CH₂OC≡CH_{,} CH₂OCH₂C≡CH_{,} CH₂OCH₂C≡CCH_{3,} CH₂CH₂OCH₂C≡CCH₂CH_{3,} CH₂OCH₂CH₂CH₂C≡CH_{,} CH≡CCH₂OCH_{3,} CH₂CH₂C≡CCH₂CH₂OCH_{3,} CH≡CSCH_{3,} CH≡CSCH₂CH_{3,} CH≡CSCH₂CH₂CH_{3,} CH₂SC≡CH_{,} CH₂SCH₂C≡CH_{,} CH₂SCH₂C≡CCH_{3,} CH₂CH₂SCH₂C≡CCH₂CH_{3,} CH₂SCH₂CH₂CH₂C≡CH_{,} CH≡CCH₂SCH_{3,} CH₂CH₂C≡CCH₂CH₂SCH_{3,} CH**≡**CNHCH_{3,} CH≡CNHCH₂CH_{3,} CH≡CNHCH₂CH₂CH_{3,} CH₂NHC≡CH_{,} CH₂NHCH₂C≡CH_{,} CH₂NHCH₂CH₂C≡CH_{,} CH₂CH₂NHCH₂CH₂C≡CCH_{3,} CH₂NHCH₂CH₂C≡CCH_{3,} CH₂C≡CNHCH_{3,} CH₂C≡CCH₂CH₂CH₂NHCH_{3,} CH≡CN(CH₂)₂, CH₂N(C≡CH)₂ CHzN(CHzC=CH)z.

Amino: -NR¹R², wherein R¹ and R² are independently amino substituents, for example, hydrogen, a C₁₋₆ hydrocarbon group (also referred to as C₁₋₆ alkylamino or C₁₋₆dialkylamino), or, in the case of a "cyclic" amino group, R¹ and R², taken together with the nitrogen atom to which they are attached, form a heterocyclic ring having from 4 to 6 ring atoms. Amino groups may be primary (-NH₂), secondary (-NHR¹), or tertiary (-NHR¹R²), and in cationic form, may be quaternary (-⁺NR¹R²R³). Examples of amino groups include, but are not limited to -NH₂, -NHCH₃, -NHC(CH₃)₂, -N(CH₃)₂, -N(CH₂CH₃)₂, N(C₁₋₆ alkyl)₂ and -NHPh. Examples of cyclic amino groups include, but are not limited to, aziridino, azetidino, pyrrolidino, piperidino, piperazino, morpholino, and thiomorpholino.

C₁₋₆ alkyl amido: The term C₁₋₆ alkyl amido (also referred to as carbamoyl, carbamyl, aminocarbonyl, carboxamide) has the structure -C(=O)NR¹R², wherein R¹ and R² are independently amino substituents, as defined for amino groups for example, hydrogen, a C₁₋₆ hydrocarbon group (also referred to as C₁₋₆ alkylamido or C₁₋₆dialkylamido), or, in the case of a "cyclic" amido group, R¹ and R², taken together with the nitrogen atom to which they are attached, form a heterocyclic ring having from 4 to 6 ring atoms. Examples of amido groups include, but are not limited to, -C(=O)NH₂, -C(=O)N(C₁₋₆alkyl)₂, C(=O)NH(C₁₋₆ alkyl), such as -C(=O)NHCH₃, -C(=O)N(CH₃)₂, -C(=O)NHCH₂CH₃, and -C(=O)N(CH₂CH₃)₂, as well as amido groups in which R¹ and R², together with the nitrogen atom to which they are attached, form a heterocyclic structure as in, for example, piperidinocarbonyl, morpholinocarbonyl, thiomorpholinocarbonyl, and piperazinocarbonyl.

Carbonyl: a carbonyl functional group refers to a C=O bond such as the following structure R-C(=O)-R' wherein R and R' are independently carbonyl substituents which can be organic groups or H atoms for example a C₁₋₆hydrocarbon group , C₆₋₁₀ aryl, C₃₋₆ cycloalkyl, C₄₋₁₀ heterocyclyl, C₄₋₁₀ heterocyclyl or H. For example carbonyl groups include C(=O)-(C₁₋₄ alkyl), -C(=O)-(C₂₋₄ alkenyl), -C(=O)-(C₂₋₄ alkynyl), - C(=O)-(C₆₋₁₀ aryl), -C(=O)-(C₃₋₆ cycloalkyl), -C(=O)-(C₄₋₁₀ heterocyclyl), -C(=O)-( C₄₋₁₀ heterocyclyl), - C(=O)-(C₁₋₄ alkoxy), -C(=O)-(C₃₋₄ alkenoxy), -C(=O)-(C₃₋₄ alkynoxy), -C(=O)-(C₃₋₆ heteroalkyl), -C(=O)-(C₄₋₆ heteroalkenyl), or -C(=O)-(C₄₋₆ heteroalkynyl).

Carboxy (carboxylic acid): -C(=O)OH (COzH).

Ester: If not stated otherwise, the term "ester" in this document refers to a carboxylic ester functional group with the formula R-C(=O)-OR' wherein R and R' are independently substituents which are organic groups. for example a C₁₋₆ hydrocarbon group. R can also be hydrogen. For example an ester group can be -CO₂(C₁₋₆ alkyl).

C₁₋₆ alkyl ester: The term C₁₋₆ alkyl ester (carboxylate, carboxylic acid ester, oxycarbonyl) has the structure -C(=O)OR, wherein R is a C₁₋₆ hydrocarbon group. For example, CO₂(C₁₋₆ alkyl) or CO₂C₁₋₄ alkyl.

Examples of ester groups include, but are not limited to, -C(=O)OCH₃, -C(=O)OCH₂CH₃ and -C(=O)OC(CH₃)₃.

Amide: an amide functional group which has the structure R-C(=O)N(R')(R") wherein R, R' and R" are independently amide substituents which can be organic groups or H atoms for example a C₁₋₆hydrocarbon group or H.

Aminocarbonyloxy: -OC(=O)NR¹R², wherein R¹ and R² are independently amino substituents, as defined for amino groups. Examples of aminocarbonyloxy groups include, but are not limited to, -OC(=O)NH₂, - OC(=O)NH-CH₃, -OC(=O)N(CH₃)₂, and -OC(=O)N(CH₂-CH₃)₂.

Phosphoric acid (phosphonooxy): -OP(=O)(OH)₂.

C₄₋₁₂ heterocyclyl: The term "C₄₋₁₂ heterocyclyl" as used herein, pertains to a monovalent moiety obtained by removing a hydrogen atom from a ring atom of a heterocyclic compound, which moiety has from 4 to 12 ring atoms, of which from 1 to 5 are ring heteroatoms. In certain embodiments, each ring has from 5 to 7 ring atoms, of which from 1 to 4 are ring heteroatoms. The ring may be saturated or unsaturated and may be bridged or unbridged. The ring may be a fused ring or a single ring. For the avoidance of doubt, substituents on the heterocyclyl ring may be linked via either a carbon atom or a heteroatom.

In this context, the prefixes (e.g. C₅₋₁₀ C₅₋₇, C₅₋₆, etc.) denote the number of ring atoms, or range of number of ring atoms, whether carbon atoms or heteroatoms. For example, the term "C₅₋₆ heterocyclyl", as used herein, pertains to a heterocyclyl group having 5 or 6 ring atoms.

Examples of monocyclic heterocyclyl groups include, but are not limited to, those derived from:
N₁: aziridine (C₃), azetidine (C₄), pyrrolidine (tetrahydropyrrole) (C₅), pyrrolidine (C₅), pyrroline (e.g., 3-pyrroline, 2,5-dihydropyrrole) (C₅), 2H-pyrrole or 3H-pyrrole (C₅), piperidine (C₆), dihydropyridine (C₆), tetrahydropyridine (C₆), azepine (C₇), 1,2,3,4,-tetrahydroquinolone (C₁₀);
0₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃), thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), pyrazole (C₅), piperazine (C₆), pyrimidine (C₆);
N₃: triazole (C₅)
N₄: tetrazole (C₅)
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂S₁: 6,7-dihydro-4H-thiazolo[5,4-c]pyridine-5-yl (C₉);
N₂O₁: oxadiazine (C₆);
O₁S₁: oxathiole (C₅) and oxathiane (thioxane) (C₆); and,
N₁O₁S₁: oxathiazine (C₆).

Examples of bicyclic heterocyclyl groups include, but are not limited to those derived from:

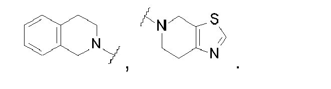

C₆₋₁₀ carboaryl: The term "C₆₋₁₀ carboaryl", as used herein, pertains to a monovalent moiety obtained by removing a hydrogen atom from an aromatic ring atom of an aromatic compound, which moiety has from 6 to 10 ring atoms and the ring atoms are all carbon atoms, as in "carboaryl groups". The ring may be a fused ring or a single ring. Examples of carboaryl groups include, but are not limited to, those derived from benzene (i.e. phenyl) (C₆), naphthalene (C₁₀) and azulene (C₁₀).

In this context, the prefixes (e.g. C₅₋₇, C₅₋₆, C₅₋₁₀, etc.) denote the number of ring atoms, or range of number of ring atoms. For example, the term "C₅₋₆ aryl" as used herein, pertains to an aryl group having 5 or 6 ring atoms.

Examples of carboaryl groups which comprise fused rings, at least one of which is an aromatic ring, include, but are not limited to, groups derived from indane (e.g. 2,3-dihydro-1H-indene) (C₉), indene (C₉), isoindene (C₉) and tetraline (1,2,3,4-tetrahydronaphthalene) (C₁₀).

C₅₋₁₂ heteroaryl: The term "C₅₋₁₂ heteroaryl", as used herein, pertains to a monovalent moiety obtained by removing a hydrogen atom from an aromatic ring atom of an aromatic compound, which moiety has from 5 to 12 ring atoms of which from 1 to 5 are ring heteroatoms. In certain embodiments, each ring has from 5 to 7 ring atoms, of which from 1 to 4 are ring heteroatoms. For the avoidance of doubt, substituents on the heteroaryl ring may be linked via either a carbon atom or a heteroatom. The ring may be a fused ring or a single ring.

Examples of monocyclic heteroaryl groups include, but are not limited to, those derived from:
N₁: pyrrole (C₅), pyridine (C₆);
O₁: furan ( (C₅);
S₁: thiophene (C₅);
N₁O₁: oxazole (C₅), isoxazole (C₅)
N₂O₁: oxadiazole, such as 1,2,5-oxadiazole (furazan) (C₅), ;
N₃O₁: oxatriazole (C₅);
N₁S₁: 1,3-thiazole (C₅), 1,2-thiazole (isothiazole) (C₅);
N₂: imidazole (C₅), pyrazole (C₅), pyridazine (1,2-diazine) (C₆), pyrimidine (1,3-diazine) (C₆) (e.g., cytosine, thymine, uracil), pyrazine (1,4-diazine) (C₆);
N₃: triazole (C₅), triazine (C₆); and,
N₄: tetrazole (C₅).

### Pharmaceutically acceptable salt

The term "pharmaceutically acceptable" is used to specify that an object (for example a salt, dosage form or excipient) is suitable for use in patients. An example list of pharmaceutically acceptable salts can be found in the Handbook of Pharmaceutical Salts: Properties, Selection and Use*,* P. H. Stahl and C. G.

Wermuth, editors, Weinheim/Zürich: Wiley-VCH/VHCA, 2002. A suitable pharmaceutically acceptable salt of a compound of Formula (I-b) is, for example, an acid-addition salt. An acid addition salt of a compound of Formula (I-b) may be formed by bringing the compound into contact with a suitable inorganic or organic acid under conditions known to the skilled person. An acid addition salt may for example be formed using an inorganic acid selected from the group consisting of hydrochloric acid, hydrobromic acid, sulphuric acid and phosphoric acid. An acid addition salt may also be formed using an organic acid selected from the group consisting of trifluoroacetic acid, citric acid, maleic acid, oxalic acid, acetic acid, formic acid, benzoic acid, fumaric acid, succinic acid, tartaric acid, lactic acid, pyruvic acid, methanesulfonic acid, benzenesulfonic acid and para-toluenesulfonic acid.

Therefore, in one embodiment there is provided a compound of Formula (I-b), (I-b1) or (I-2b) or a pharmaceutically acceptable salt thereof, where the pharmaceutically acceptable salt is a hydrochloric acid, hydrobromic acid, sulphuric acid, phosphoric acid, trifluoroacetic acid, citric acid, maleic acid, oxalic acid, acetic acid, formic acid, benzoic acid, fumaric acid, succinic acid, tartaric acid, lactic acid, pyruvic acid, methanesulfonic acid, benzenesulfonic acid or para-toluenesulfonic acid salt. In one embodiment there is provided a compound of Formula (I-b), (I-b1) or (I-2b) or a pharmaceutically acceptable salt thereof, where the pharmaceutically acceptable salt is a methanesulfonic acid salt.

### Other forms

Compounds and salts described in this specification may exist in solvated forms and unsolvated forms. For example, a solvated form may be a hydrated form, such as a hemihydrate, a monohydrate, a dihydrate, a trihydrate or an alternative quantity thereof. The compounds of Formula (I-b) encompass all such solvated and unsolvated forms of compounds of Formula (I-b), particularly to the extent that such forms possess STING antagonist activity, as for example measured using the tests described herein.

Compounds and salts described in this specification include one or more chiral (i.e. asymmetric) centres. To the extent a structure or chemical name in this specification does not indicate the chirality, the structure or name is intended to encompass any single stereoisomer (i.e. any single chiral isomer) corresponding to that structure or name, as well as any mixture of stereoisomers (e.g. a racemate). In some embodiments, a single stereoisomer is obtained by isolating it from a mixture of isomers (e.g. a racemate) using, for example, chiral chromatographic separation. In other embodiments, a single stereoisomer is obtained through direct synthesis from, for example, a chiral starting material.

A particular enantiomer of a compound described herein may be more active than other enantiomers of the same compound.

According to one embodiment there is provided a compound of Formula (I-b), or a pharmaceutically acceptable salt thereof, which is a single enantiomer being in an enantiomeric excess (%ee) of ≥ 95, ≥ 98% or ≥ 99%. Conveniently, the single enantiomer is present in an enantiomeric excess (%ee) of ≥ 99%.

According to another embodiment there is provided a pharmaceutical composition, which comprises a compound of Formula (I-b), which is a single enantiomer being in an enantiomeric excess (%ee) of ≥ 95, ≥ 98% or ≥ 99% or a pharmaceutically acceptable salt thereof, in association with one or more pharmaceutically acceptable excipients. Conveniently, the single enantiomer is present in an enantiomeric excess (%ee) of ≥ 99%.

### Isotopes

Atoms of the compounds and salts described in this specification may exist as their isotopes. The compound of Formula (I-b) encompasses all compounds of Formula (I-b) where an atom is replaced by one or more of its isotopes (for example a compound of Formula (I-b) where one or more carbon atom is an ¹¹C or ¹³C carbon isotope, or where one or more hydrogen atoms is a ²H or ³H isotope).

### Tautomers

Compounds and salts described in this specification may exist as a mixture of tautomers. "Tautomers" are structural isomers that exist in equilibrium resulting from the migration of a hydrogen atom. The compound of Formula (I-b) includes all tautomers of compounds of Formula (I-b) particularly to the extent that such tautomers possess STING antagonist activity.

For example, the tautomeric form of compound (I-b), when R⁸ and R¹⁸ are hydrogen, can be shown as Formula (I-a):

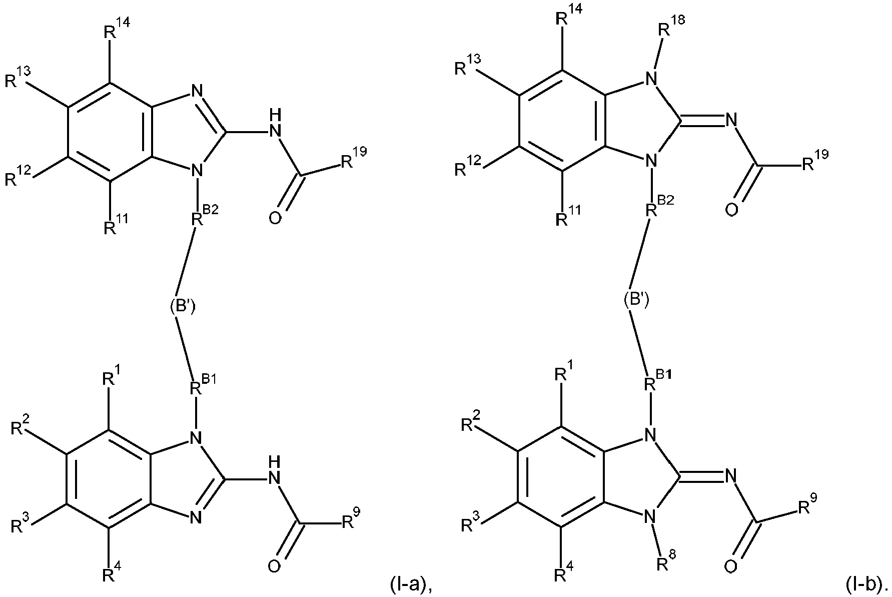

The tautomeric form of compound (I-b1), when R⁸ and R¹⁸ are hydrogen, can be shown as (I-a1): 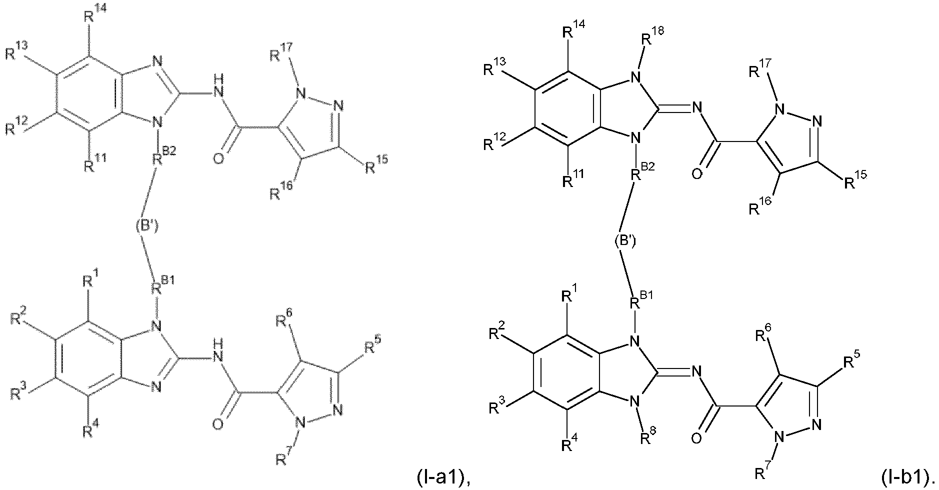 Where R⁸ and R¹⁸ are H the tautomeric forms (I-a) and (I-a1) might form.

### Crystalline forms

Compounds and salts described in this specification may be crystalline and may exhibit one or more crystalline forms. The compound of Formula (I-b) encompasses any crystalline or amorphous form of a compound of Formula (I-b), or mixture of such forms, which possesses STING antagonist activity.

It is generally known that crystalline materials may be characterised using conventional techniques such as X-Ray Powder Diffraction (XRPD), Differential Scanning Calorimetry (DSC), Thermal Gravimetric Analysis (TGA), Diffuse Reflectance Infrared Fourier Transform (DRIFT) spectroscopy, Near Infrared (NIR) spectroscopy, solution and/or solid state nuclear magnetic resonance spectroscopy. The water content of crystalline materials may be determined by Karl Fischer analysis.

### Therapy, prophylaxis and related terms

The term "therapy" is intended to have its normal meaning of dealing with a disease in order to entirely or partially relieve one, some or all of its symptoms, or to correct or compensate for the underlying pathology. The term "therapy" also includes "prophylaxis" unless there are specific indications to the contrary. The terms "therapeutic" and "therapeutically" should be interpreted in a corresponding manner.

The term "prophylaxis" is intended to have its normal meaning and includes primary prophylaxis to prevent the development of the disease and secondary prophylaxis whereby the disease has already developed and the patient is temporarily or permanently protected against exacerbation or worsening of the disease or the development of new symptoms associated with the disease.

The term "treatment" is used synonymously with "therapy". Similarly the term "treat" can be regarded as "applying therapy" where "therapy" is as defined herein.

The term "subject" to which administration is contemplated includes, but is not limited to, humans (i.e., a male or female of any age group, e.g., a paediatric subject (e.g., infant, child, adolescent) or adult subject (e.g., young adult, middle-aged adult or senior adult)) and/or other primates (e.g., cynomolgus monkeys, rhesus monkeys); mammals, including commercially relevant mammals such as cattle, pigs, horses, sheep, goats, cats, and/or dogs; and/or birds, including commercially relevant birds such as chickens, ducks, geese, quail, and/or turkeys. Preferred subjects are humans.

An "effective amount", as used herein, refers to an amount that is sufficient to achieve a desired biological effect. A "therapeutically effective amount", as used herein refers to an amount that is sufficient to achieve a desired therapeutic effect. For example, a therapeutically effective amount can refer to an amount that is sufficient to improve at least one sign or symptom of the disease to be treated.

### Pharmaceutical compositions

The compounds of Formula (I-b), and pharmaceutically acceptable salts thereof, may be administered as pharmaceutical compositions, comprising one or more pharmaceutically acceptable excipients.

Therefore, in one embodiment there is provided a pharmaceutical composition comprising a compound of Formula (I-b), or a pharmaceutically acceptable salt thereof, and at least one pharmaceutically acceptable excipient.

The excipient(s) selected for inclusion in a particular composition will depend on factors such as the mode of administration and the form of the composition provided. Suitable pharmaceutically acceptable excipients are well known to persons skilled in the art and are described, for example, in the Handbook of Pharmaceutical Excipients, Sixth edition, Pharmaceutical Press, edited by Rowe, Ray C; Sheskey, Paul J; Quinn, Marian. Pharmaceutically acceptable excipients may function as, for example, adjuvants, diluents, carriers, stabilisers, flavourings, colorants, fillers, binders, disintegrants, lubricants, glidants, thickening agents and coating agents. As persons skilled in the art will appreciate, certain pharmaceutically acceptable excipients may serve more than one function and may serve alternative functions depending on how much of the excipient is present in the composition and what other excipients are present in the composition.

The pharmaceutical compositions may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous or intramuscular dosing), or as a suppository for rectal dosing. The compositions may be obtained by conventional procedures well known in the art. Compositions intended for oral use may contain additional components, for example, one or more colouring, sweetening, flavouring and/or preservative agents.

Suitable daily doses of the compounds disclosed herein, or a pharmaceutically acceptable salt thereof, in therapeutic treatment of humans are about 0.0001-100 mg/kg body weight.

Pharmaceutical formulations as described herein may be formulated by methods known to those skilled in the art to provide doses of the active compound in the range of 0.1 mg to 1000 mg. The daily dose will necessarily be varied depending upon the host treated, the particular route of administration, any therapies being co-administered, and the severity of the illness being treated. Accordingly, the practitioner who is treating any particular patient may determine the optimum dosage.

The pharmaceutical compositions described herein comprise compounds of Formula (I-b), or a pharmaceutically acceptable salt thereof, and are therefore expected to be useful in therapy.

As such, in one embodiment there is provided a pharmaceutical composition for use in therapy, comprising a compound of Formula (I-b), or a pharmaceutically acceptable salt thereof, and at least one pharmaceutically acceptable excipient.

In one embodiment there is provided a pharmaceutical composition for use in the treatment of a disease associated with overactivation of STING, comprising a compound of Formula (I-b), or a pharmaceutically acceptable salt thereof, and at least one pharmaceutically acceptable excipient. In one embodiment there is provided a pharmaceutical composition for use in the treatment of a disease, comprising a compound of Formula (I-b), or a pharmaceutically acceptable salt thereof, and at least one pharmaceutically acceptable excipient. In one embodiment there is provided a pharmaceutical composition for use in the treatment of a disease associated with overactivation of STING, comprising a compound of Formula (I-b), or a pharmaceutically acceptable salt thereof, and at least one pharmaceutically acceptable excipient.

### Methods of Use

The compounds described herein may be used in a method of therapy. Also provided is a method of treatment, comprising administering to a subject in need of treatment a therapeutically effective amount of a compound of Formula (I-b). The term "therapeutically effective amount" is an amount sufficient to show benefit to a patient. Such benefit may be at least amelioration of at least one symptom. The actual amount administered, and rate and time-course of administration, will depend on the nature and severity of what is being treated. Prescription of treatment, e.g. decisions on dosage, is within the responsibility of general practitioners and other medical doctors.

A compound may be administered alone or in combination with other treatments, either simultaneously or sequentially dependent upon the condition to be treated.

In one embodiment there is provided a compound of Formula (I-b), or a pharmaceutically acceptable salt thereof, or a pharmaceutical composition comprising the compound of Formula (I-b) for use in therapy. In one embodiment there is provided the use of the compound of Formula (I-b), or a pharmaceutically acceptable salt thereof, or a pharmaceutical composition comprising the compound of Formula (I-b) for the manufacture of a medicament. In another embodiment there is provided a method of treatment comprising administering to a subject the compound of Formula (I-b), or a pharmaceutically acceptable salt thereof, or a pharmaceutical composition comprising the compound of Formula (I-b).

In one embodiment there is provided a compound of Formula (I-b), or a pharmaceutically acceptable salt thereof, or a pharmaceutical composition comprising the compound of Formula (I-b) for use in treating a disorder. In some embodiments there is provided a method of treating a disorder or condition comprising administering to a subject the compound of Formula (I-b), or a pharmaceutically acceptable salt thereof, or a pharmaceutical composition comprising the compound of Formula (I-b).

In some embodiments, the disorder or condition is associated with overactivation of STING. In some embodiments, the disorder or condition is driven by constitutive STING activation. In some embodiments, the disorder or condition is driven by constitutive STING activation caused by a gain-of-function mutation.

In some embodiments, the disorder or condition is a monogenic autoinflammatory syndrome, autoimmune disease, inflammatory disease, neurological disorder, metabolic disease, cardiovascular disease, or cancer.

In some embodiments, the disorder or condition is a monogenic autoinflammatory syndrome. In some embodiments, the disorder or condition is STING-associated vasculopathy with onset in infancy (SAVI), Aicardi-Goutieres Syndrome, COPA Syndrome, Familial Chilblain Lupus, Dnase II deficiency, or TREX1 deficiency.

In some embodiments, the disorder or condition is an autoimmune disease. In some embodiments, the disorder or condition is systemic lupus erythematosus (SLE), Sjögren's syndrome, or rheumatoid arthritis.

In some embodiments, the disorder or condition is an inflammatory disease. In some embodiments, the disorder or condition is silica-induced fibrosis or sepsis.

In some embodiments, the disorder or condition is a neurological disorder. In some embodiments, the disorder or condition is ischaemic brain injury, Parkinson disease, general neurodegeneration, Huntington disease, amyotrophic lateral sclerosis and frontotemporal dementia, age-dependent macular degeneration, or traumatic brain injury.

In some embodiments, the disorder or condition is a metabolic disease. In some embodiments, the disease is non-alcoholic steatohepatitis, alcoholic liver disease, or acute pancreatitis.

In some embodiments, the disorder or condition is a cardiovascular disease. In some embodiments, the disorder or condition is myocardial infarction or chronic heart failure.

In some embodiments, the disorder or condition is cancer. In some embodiments, the disorder or condition is colorectal cancer, skin cancer, or metastases.

In some embodiments the disorder or condition is selected from monogenic autoinflammatory syndrome, STING-associated vasculopathy with onset in infancy (SAVI), Aicardi-Goutieres Syndrome, COPA Syndrome, Familial Chilblain Lupus, DNase II deficiency, or TREX1 deficiency, an autoimmune disease, systemic lupus erythematosus (SLE), Sjögren's syndrome or a cardiovascular disease.

In some embodiments, the disorder or condition is senescence or aging. In some embodiments, the subject is a mammal. In some embodiments, the subject is a human.

### Further embodiments /preferences

The following embodiments may apply to all aspects as described above or may relate to a single aspect. The embodiments may be combined together in any combination.

### R⁹ and R¹⁹

In some embodiments R⁹ and R¹⁹ are each independently selected from an optionally substituted C₅₋₁₀ cycloalkyl, optionally substituted C₆₋₁₀ carboaryl, optionally substituted C₅₋₁₀ heteroaryl or optionally substituted C₅₋₁₀ heterocyclyl, wherein the optional substituents are independently selected from H, halogen, C₃₋₅ cycloalkyl, optionally substituted C₁₋₄ alkyl wherein said optionally substituted C₁₋₄ alkyl is optionally substituted by one or more substituents independently selected from halogen, halo(C₁₋₄ alkyl), OH, NH₂, C₁₋₄ alkoxy, CO₂H, CO₂(C₁₋₆ alkyl), N(C₁₋₆ alkyl)₂, OC(=O)NH₂ and C(=O)NH₂.

In further embodiments R⁹ and R¹⁹ are each independently selected from an optionally substituted C₅₋₆ heteroaryl or optionally substituted C₅₋₆ heterocyclyl, wherein the optional substituents are independently selected from H, halogen, C₃₋₅ cycloalkyl, optionally substituted C₁₋₄ alkyl wherein said optionally substituted alkyl is optionally substituted by one or more substituents independently selected from halogen, halo(C₁₋₄ alkyl), OH, NH₂, C₁₋₄ alkoxy, CO₂H, CO₂C₁₋₆ alkyl, N(C₁₋₆ alkyl)₂, OCONH₂ and CONH₂.

When R⁹ or R¹⁹ is an optionally substituted C₅₋₆ heteroaryl or optionally substituted C₅₋₆ heterocyclyl the optional substituent is selected from optionally substituted methyl, optionally substituted ethyl, optionally substituted propyl wherein the optional substituents are OH, NH₂, halogen or C₁₋₄ alkoxy. In further embodiments when R⁹ or R¹⁹ is an optionally substituted C₅₋₆ heteroaryl or optionally substituted C₅₋₆ heterocyclyl the optional substituent is selected from methyl and ethyl.

In some embodiments Formula (I-b) is of Formula (I-b1):

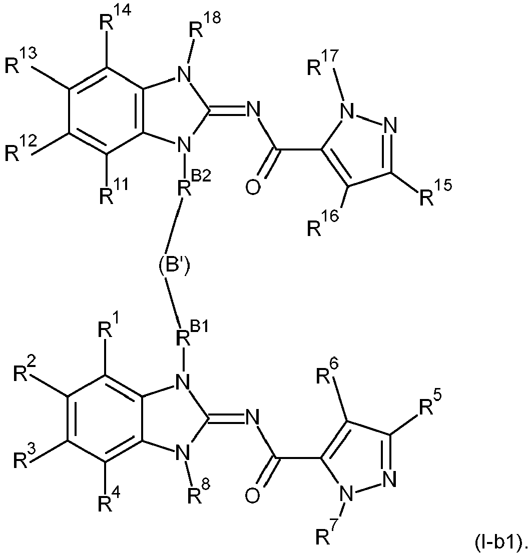

### R¹ and R¹¹

In some embodiments R¹ and R¹¹ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are independently selected from OH, -OP(O)(OH)₂, C₁₋₄ alkoxy, NH₂, N(C₁₋₆ alkyl)₂, CO₂(C₁₋₆ alkyl) and halogen.

When R¹ or R¹¹ are optionally substituted C₁₋₆ alkyl, the optional substituents are selected from OH, F, Br, methoxy and ethoxy. In some embodiments when R¹ or R¹¹ are optionally substituted C₁₋₆ alkyl, they are optionally substituted methyl or optionally substituted ethyl. In further embodiments, they are optionally substituted methyl. In further embodiments, they are unsubstituted methyl.

When R¹ or R¹¹ are halo, in some embodiments they are F, Br or CI.

In some embodiments R¹ and R¹¹ are independently selected from H, OH, F or methyl.

In some embodiments R¹ is identical to R¹¹.

In some embodiments R¹ and R¹¹ are both H.

### R² and R¹²

In some embodiments R² and R¹² are each independently selected from H, halogen, OH, optionally substituted C₁₋₆ alkyl, optionally substituted C₁₋₆ alkoxy, optionally substituted CO₂(C₁₋₆ alkyl), and optionally substituted C₁₋₆ alkylamino wherein the optional substituents are selected from OH, -OP(O)(OH)₂, C₁₋₄ alkoxy, N(C₁₋₆ alkyl)₂, NH₂, CO₂(C₁₋₆ alkyl) and halogen.

When R² or R¹² are optionally substituted C₁₋₆ alkyl, the optional substituents are selected from OH, F, Br, methoxy and ethoxy. In some embodiments when R² or R¹² are optionally substituted C₁₋₆ alkyl, they are optionally substituted methyl or optionally substituted ethyl. In further embodiments, they are optionally substituted methyl. In further embodiments, they are unsubstituted methyl.

When R² or R¹² are halo, in some embodiments they are F, Br or CI.

In some embodiments R² and R¹² are independently selected from H, OH, F or methyl.

In some embodiments R² is identical to R¹².

In some embodiments R² and R¹² are both H.

### R³ and R¹³

In some embodiments R³ and R¹³ are each independently selected from H, C(=O)NH₂; optionally substituted -C(=O)NH(C₁₋₆ alkyl), optionally substituted -C(=O)N(C₁₋₆ alkyl)₂, optionally substituted C₁₋₆ alkoxy or optionally substituted CO₂C₁₋₆ alkyl, wherein the optional substituents are independently selected from OH, -O-P(O)(OH)₂, C₁₋₄ alkoxy, N(C₁₋₆ alkyl)₂, NH₂, CO₂(C₁₋₆ alkyl) and halogen.

When R³ or R¹³ are optionally substituted -C(=O)NH(C₁₋₆ alkyl), optionally substituted C₁₋₆ alkoxy or optionally substituted CO₂C₁₋₆ alkyl, the optional substituents are selected from OH, -O-P(O)(OH)₂, methoxy, ethoxy, N(CH₃)₂, NH₂, CO₂(CH₃) and halogen.

When R³ or R¹³ are -C(=O)NH(C₁₋₆ alkyl) in some embodiments they are -C(=O)NHCH₃, - C(=O)NHCH₂CH₃, -C(=O)NHCH₂CH₂CH₃.

In some embodiments R³ and R¹³ are selected from H, -C(=O)NH₂-C(=O)NHCH₃, -C(=O)NHCH₂CH₃, or-C(=O)NHCH₂CH₂CH₃.

In some embodiments R³ and R¹³ are selected from -C(=O)NH(C₁₋₃ alkyl) and -C(=O)NH₂.

In some embodiments R³ is identical to R¹³.

In some embodiments R³ and R¹³ are both -C(=O)NH₂.

### R⁴ and R¹⁴

In some embodiments R⁴ and R¹⁴ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are selected from OH, -O-P(O)(OH)₂, C₁₋₄alkoxy, N(C₁₋₆ alkyl)₂, NH₂, CO₂(C₁₋₆ alkyl) and halogen.

When R⁴ or R¹⁴ are optionally substituted C₁₋₆ alkyl, the optional substituents are selected from OH, F, Br, methoxy and ethoxy. In some embodiments when R⁴ or R¹⁴ are optionally substituted C₁₋₆ alkyl, they are optionally substituted methyl or optionally substituted ethyl. In further embodiments, they are optionally substituted methyl. In further embodiments they are unsubstituted methyl.

When R⁴ or R¹⁴ are halo, in some embodiments they are F, Br or CI.

In some embodiments R⁴ and R¹⁴ are independently selected from H, OH, F or methyl.

In some embodiments R⁴ is identical to R¹⁴.

In some embodiments R⁴ and R¹⁴ are both H.

### R⁵ and R¹⁵

In some embodiments R⁵ and R¹⁵ are each independently selected from H, cyclopropyl and C₁₋₄ alkyl.

In some embodiments R⁵ and R¹⁵ are both H.

In some embodiments R⁵ and R¹⁵ are both cyclopropyl.

When R⁵ or R¹⁵ are each independently C₁₋₄ alkyl they are methyl, ethyl, propyl or butyl. In some embodiments they are methyl or ethyl. In further embodiments they are methyl.

In some embodiments R⁵ is identical to R¹⁵.

In some embodiments R⁵ and R¹⁵ are both methyl.

### R⁶ and R¹⁶

In some embodiments R⁶ and R¹⁶ are each independently selected from H, halogen or optionally substituted C₁₋₄ alkyl or C₁₋₄ alkoxy wherein the optional substituents are independently selected from halogen, halo(C₁₋₄ alkyl), OH, and C₁₋₄ alkoxy.

When R⁶ or R¹⁶ are optionally substituted C₁₋₆ alkyl, the optional substituents are selected from OH, F, Br, methoxy and ethoxy. In some embodiments when R⁶ or R¹⁶ are optionally substituted C₁₋₆ alkyl, they are optionally substituted methyl or optionally substituted ethyl. In further embodiments, they are optionally substituted methyl. In further embodiments, they are unsubstituted methyl.

When R⁶ or R¹⁶ are halo, in some embodiments they are F, Br or CI.

In some embodiments R⁶ and R¹⁶ are independently selected from H, OH, F or methyl.

In some embodiments R⁶ is identical to R¹⁶.

In some embodiments R⁶ and R¹⁶ are both H.

### R⁷ and R¹⁷

In some embodiments R⁷ and R¹⁷ are each independently selected from optionally substituted C₁₋₄ alkyl wherein the optional substituents are independently selected from halogen, -halo(C₁₋₄ alkyl), OH, and C₁₋₄ alkoxy.

When R⁷ or R¹⁷ are each independently optionally substituted C₁₋₄ alkyl it is an optionally substituted methyl, ethyl, propyl or butyl. When R⁷ or R¹⁷ are each independently selected from optionally substituted C₁₋₄ alkyl the optional substituents are selected from OH, halogen, methoxy and ethoxy. When R⁷ and R¹⁷ are each independently optionally substituted C₁₋₄ alkyl, in some embodiments they are methyl or ethyl.

In some embodiments R⁷ is identical to R¹⁷.

In some embodiments R⁷ and R¹⁷ are both ethyl.

### R⁸ and R¹⁸

In some embodiments R⁸ and R¹⁸ are each independently selected from H, and C₁₋₄ alkyl.

In some embodiments when R⁸ and R¹⁸ are C₁₋₄ alkyl they are selected from methyl, ethyl, propyl or butyl.

In some embodiments R⁸ and R¹⁸ are methyl, ethyl or H. In further embodiments R⁸ and R¹⁸ are methyl or H.

In some embodiments R⁸ is identical R¹⁸.

In some embodiments R⁸ and R¹⁸ are both H.

In some embodiments R⁸ and R¹⁸ are both methyl.

In some embodiments R⁸ and R¹⁸ are both ethyl.

### R^{B1} and R^{B2}

In some embodiments R^{B1} and R^{B2} are each independently selected from (-CH₂-)₁₋₂ or a bond.

When R^{B1} or R^{B2} are a bond this is a bond connected directly from the N to B'.

When R^{B1} or R^{B2} are (-CH₂-)₁₋₂ they can each independently be -CH₂- or -CH₂CH₂-.

In some embodiments R^{B1} is a bond and R^{B2} is -CH₂- or -CH₂CH₂-. In some embodiments R^{B1} is a bond and R^{B2} is -CH₂- or -CH₂CH₂-. In other embodiments R^{B2} is a bond and R^{B1} is -CH₂-. In other embodiments R^{B1} is a bond and R^{B2} is -CH₂-.

In some embodiments R^{B1} and R^{B2} are both a bond.

In some embodiments R^{B1} and R^{B2} are both -CH₂- or both -CH₂-CH₂- or R^{B1} is -CH₂- and R^{B2} is -CH₂-CH₂-In some embodiments R^{B1} is identical to R^{B2}.

In some embodiments R^{B1} and R^{B2} are both -CH₂-.

### B'

In some embodiments B' is: 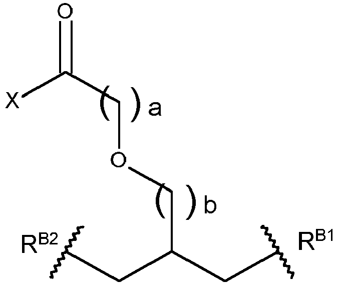
wherein a is 0, 1, or 2;
b is 0 or 1;
wherein X is -NR^{B3}R^{B4} or X is -OR^{B5},
wherein R^{B3}, R^{B4} and R^{B5} are each independently selected from:
   i) hydrogen;
   ii) optionally substituted C₁₋₈ alkyl;
   iii) -(CH₂-(CH₂)₁₋₂₋O)₁₋₃-(CH₂)₁₋₃-H;
   iv) optionally substituted C₂₋₈ alkenyl;
   v) optionally substituted C₂₋₈ alkynyl;
   vi) optionally substituted C₆₋₁₀ carboaryl;
   vii) optionally substituted C₃₋₁₀ cycloalkyl;
   viii) optionally substituted C₅₋₁₂ heteroaryl;
   ix) optionally substituted C₄₋₁₂ heterocyclyl;
   x) optionally substituted C₃₋₁₀ heteroalkyl;
   xi) optionally substituted C₃₋₁₀ heteroalkenyl;
   xii) optionally substituted C₃₋₁₀ heteroalkynyl;
or wherein R^{B3} and R^{B4} together with N form an optionally substituted C₄₋₁₂ heterocyclyl or C₅₋₁₂ heteroaryl;
wherein the optional substituents at R^{B3}, R^{B4} and R^{B5} are each R^{B6},
wherein each R^{B6} is independently selected from:
   i) hydroxy;
   ii) oxo;
   iii) carboxy;
   iv) halogen;
   v) trifluoromethyl;
   vi) trifluoromethoxy;
   vii) -NH₂;
   viii) optionally substituted C₆₋₁₀ aryl;
   ix) optionally substituted C₃₋₆ cycloalkyl;
   x) optionally substituted C₄₋₁₀ heterocyclyl;
   xi) optionally substituted C₅₋₁₂ heteroaryl;
   xii) optionally substituted C₁₋₄ alkyl;
   xiii) optionally substituted C₂₋₄ alkenyl;
   xiv) optionally substituted C₂₋₄ alkynyl;
   xv) optionally substituted C₁₋₄ alkoxy;
   xvi) optionally substituted C₃₋₄ alkenoxy;
   xvii) optionally substituted C₃₋₄ alkynoxy;
   xviii) optionally substituted -N(C₁₋₄ alkyl)₂,
   xix) optionally substituted -NH(C₁₋₄ alkyl);
   xx) optionally substituted C₃₋₆ heteroalkyl;
   xxi) optionally substituted C₄₋₆ heteroalkenyl;
   xxii) optionally substituted C₄₋₆ heteroalkynyl;
   xxiii) optionally substituted -C(=O)-(C₆₋₁₀ aryl);
   xxiv) optionally substituted -C(=O)-(C₃₋₆ cycloalkyl);
   xxv) optionally substituted -C(=O)-(C₄₋₁₀ heterocyclyl);
   xxvi) optionally substituted -C(=O)-(C₄₋₁₀ heteroaryl);
   xxvii) optionally substituted -C(=O)-(C₁₋₄ alkyl);
   xxviii) optionally substituted -C(=O)-(C₂₋₄ alkenyl);
   xxix) optionally substituted -C(=O)-(C₂₋₄ alkynyl);
   xxx) optionally substituted -C(=O)-(C₁₋₄ alkoxy);
   xxxi) optionally substituted -C(=O)-(C₃₋₄ alkenoxy);
   xxxii) optionally substituted -C(=O)-(C₃₋₄ alkynoxy);
   xxxiii) optionally substituted -C(=O)-N(C₁₋₄ alkyl)₂,
   xxxiv) optionally substituted -C(=O)-NH(C₁₋₄ alkyl);
   xxxv) optionally substituted -C(=O)-(C₃₋₆ heteroalkyl);
   xxxvi) optionally substituted -C(=O)-(C₄₋₆ heteroalkenyl);
   xxxvii) optionally substituted -C(=O)-(C₄₋₆ heteroalkynyl);
   xxxviii) optionally substituted -CH₂-(C₆₁₀ aryl);
   xxxix) optionally substituted -CH₂-(C₄₁₀ heterocyclyl);
   xl) optionally substituted -(C=O)NH-CH₂-(C₆₋₁₀ aryl); and
   xii) optionally substituted -(C=O)NH-CH₂-(C₅₋₁₂ heteroaryl);
wherein the optional substituents at R^{B6} are each independently selected from hydroxy, oxo, carboxy, halogen, trifluoromethyl, trifluoromethoxy, -NH₂, -NH(C₁₋₂ alkyl), -N(C₁₋₂ alkyl)₂, methyl, ethyl, CH₂OCH₃, methoxy, and ethoxy.

In some embodiments a is 1.

In some embodiments b is 0.

In some embodiments X is -NR^{B3}R^{B4}.

In some embodiments X is -OR^{B5}.

### R^{B3} and R^{B4}

In some embodiments B' has the formula:

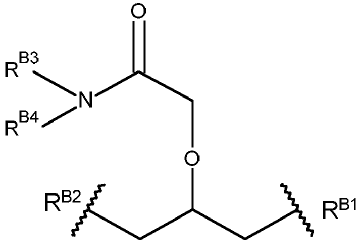

In some embodiments R^{B3} and R^{B4} are each independently selected from:
i) hydrogen;
ii) optionally substituted C₁₋₈ alkyl;
iii) -(CH₂CH₂O)₁₋₅-(CH₂)₁₋₃-H;
iv) optionally substituted C₆₋₁₀ carboaryl;
v) optionally substituted C₃₋₁₀ cycloalkyl;
vi) optionally substituted C₅₋₁₂ heteroaryl;
vii) optionally substituted C₄₋₁₂ heterocyclyl;
viii) optionally substituted C₃₋₁₀ heteroalkyl;
wherein the optional substituents are R^{B6} as defined above.

In some embodiments when R^{B3} and R^{B4} are each independently selected from -(CH₂CH₂O)₁₋₅-(CH₂)₁₋₃-H they are CH₂CH₂OCH₂CH₂OCH₂CH₂OCH₃.

In some embodiments when R^{B3} and R^{B4} are each independently selected from optionally substituted C₁₋₈ alkyl they are methyl, ethyl, propyl, butyl or isopropyl. In further embodiments when R^{B3} and R^{B4} are optionally substituted C₁₋₈ alkyl the optional substituents are R^{B6} and are selected from optionally substituted C₁₋₄ alkoxy, optionally substituted C₆₋₁₀ aryl, optionally substituted C₄₋₁₀ heterocyclyl; optionally substituted C₅₋₁₂ heteroaryl; optionally substituted -(C=O)NH-CH₂-(C₆₋₁₀ aryl); and optionally substituted - (C=O)NH-CH₂-(C₅₋₁₂ heteroaryl) wherein the optional substituents on R^{B6} are selected from methyl, or methoxy.

In some embodiments when R^{B3} or R^{B4} are selected from optionally substituted C₁₋₈ alkyl they are optionally substituted C₁₋₆ alkyl. In further embodiments the optional substituents on the C₁₋₆ alkyl is R^{B6} and R^{B6} is selected from methoxy, -(C=O)NH-CH₂-phenyl, CH₂OCH₃, C(=O)NH-phenyl, phenyl optionally substituted by methoxy, or a C₅₋₆ heteroaryl optionally substituted by methoxy or methyl.

In some embodiments R^{B3} and R^{B4} are each independently selected from a C₁₋₃ alkyl optionally substituted by methoxy, phenyl optionally substituted by methoxy, -(C=O)NH-CH₂-phenyl, CH₂OCH₃, pyridine, pyrimidine, piperidine optionally substituted by methyl or a pyrazole optionally substituted by methyl.

In some embodiments when R^{B3} and R^{B4} are each independently selected from a C₁₋₃ alkyl they are both ethyl, ethyl substituted with methoxy or isopropyl. In some embodiments when R^{B3} or R^{B4} are selected from a C₁₋₃ alkyl one of R^{B3} and R^{B4} are H and the other is isopropyl, ethyl or methyl which is substituted by CH₂OCH₃ and C(=O)NHCH₂-phenyl.

In some embodiments when R^{B3} and R^{B4} are each independently selected from optionally substituted C₆₋₁₀ carboaryl they are phenyl. In some embodiments when R^{B3} and R^{B4} are each independently optionally substituted phenyl the optional substituents are selected from methoxy and methyl. In further embodiments R^{B3} and R^{B4} are each independently selected from phenyl optionally substituted by methoxy. In some embodiments only one of R^{B3} and R^{B4} are optionally substituted C₆₋₁₀ carboaryl.

In some embodiments when R^{B3} or R^{B4} are selected from optionally substituted C₆₋₁₀ cycloalkyl they are selected from cyclopropyl, cyclobutyl or cyclopentyl. In some embodiments R^{B3} and R^{B4} are each independently selected from cyclopropyl. In some embodiments when R^{B3} or R^{B4} are selected from optionally substituted C₆₋₁₀ cycloalkyl the optional substituents are selected from methyl, ethyl, methoxy or NH₂. In some embodiments when R^{B3} or R^{B4} are selected from optionally substituted C₆₋₁₀ cycloalkyl they are unsubstituted. In some embodiments only one of R^{B3} and R^{B4} are optionally substituted C₆₋₁₀ cycloalkyl. In further embodiments only one of R^{B3} and R^{B4} are optionally substituted C₆₋₁₀ cycloalkyl and the other is methyl-phenyl-methoxy.

In some embodiments when R^{B3} or R^{B4} are selected from optionally substituted C₅₋₁₂ heteroaryl they are selected from pyrimidine or pyrazole. In some embodiments when R^{B3} or R^{B4} are selected from optionally substituted C₅₋₁₂ heteroaryl the optional substituents are R^{B6} and are selected from methoxy, ethoxy, methyl, ethyl, F, or NH₂. In further embodiments the optional substituents are methyl. In some embodiments R^{B3} and R^{B4} are each independently selected from an unsubstituted pyrimidine or a pyrazole substituted by methyl.

In some embodiments when R^{B3} or R^{B4} are selected from optionally substituted C₅₋₁₂ heterocyclyl they are a piperidine. In some embodiments when R^{B3} or R^{B4} are selected from optionally substituted C₅₋₁₂ heterocyclyl the optional substituents are R^{B6} and are selected from methoxy, ethoxy, methyl, ethyl, F, or NH₂. In further embodiments the optional substituents are NH₂, ethyl or methyl. In some embodiments R^{B3} and R^{B4} are each a piperidine substituted by methyl.

In some embodiments one of R^{B3} and R^{B4}is selected from:
i) optionally substituted C₁₋₈ alkyl;
ii) -(CH₂CH₂O)₁₋₅-(CH₂)₁₋₃-H;
iii) optionally substituted C₆₋₁₀ carboaryl;
iv) optionally substituted C₃₋₁₀ cycloalkyl;
v) optionally substituted C₅₋₁₂ heteroaryl;
vi) optionally substituted C₄₋₁₂ heterocyclyl;
vii) optionally substituted C₃₋₁₀ heteroalkyl;

wherein the optional substituents are R^{B6} as defined above;
and the other is selected from H and C₁₋₃ alkyl which C₁₋₃ alkyl is optionally substituted by optionally substituted C₆₋₁₀ aryl, optionally substituted C₅₋₁₂ heteroaryl, methoxy or CHzOCHz wherein the optional substituents on the C₆₋₁₀ aryl or C₅₋₁₂ heteroaryl are R^{B6} as defined above.

In some embodiments one of R^{B3} and R^{B4}is selected from H and C₁₋₃ alkyl optionally substituted with methoxy and the other is independently selected from:
i) C₁₋₃ alkyl optionally substituted with methoxy, C(=O)NHCH₂-phenyl, CHzOCHz, phenyl optionally substituted with OMe or pyridine;
ii) CH₂CH₂OCH₂CH₂OCH₂CH₂OCH₃;
iii) phenyl;
iv) optionally substituted C₅₋₆ heteroaryl wherein the optional substituents are C₁₋₃ alkyl; or
v) optionally substituted C₅₋₆ heterocyclyl wherein the optional substituents are C₁₋₃ alkyl.

In some embodiments one of R^{B3} and R^{B4}is H and the other is independently selected from:
i) isopropyl;
ii) optionally substituted ethyl;
iii) phenyl;
iv) optionally substituted C₂₋₇ heteroalkyl;
v) optionally substituted C₅₋₆ heteroaryl;
vi) optionally substituted C₅₋₆ heterocyclyl;
vii) -CH₂CH₂OCH₂CH₂OCH₂CH₂OCH₃.
wherein the optional substituents are R^{B6} as defined above.

In some embodiments one of R^{B3} and R^{B4}is H and the other is independently selected from:
i) isopropyl;
ii) optionally substituted ethyl wherein the optional substituent is methoxy;
iii) phenyl;
iv) optionally substituted piperidine wherein the optional substituent is methyl;
v) optionally substituted C₂₋₇ heteroalkyl wherein the optional substituent is alkoxy, C=O, or phenyl;
vi) optionally substituted C₅₋₆ heteroaryl wherein the optional substituent is methyl or NH₂;
vii) optionally substituted C₅₋₆ heterocyclyl wherein the optional substituent is methyl or NH₂;
viii) -CH₂CH₂OCH₂CH₂OCH₂CH₂OCH₃.

In further embodiments one of R^{B3} and R^{B4} is H and the other is independently selected from isopropyl, ethyl optionally substituted by methoxy, CH₂CH₂OCH₂CH₂OCH₂CH₂OCH₃, phenyl, pyrimidine, pyrazole substituted by methyl or a piperidine substituted by methyl.

In some embodiments both R^{B3} and R^{B4} are CH₂CH₂OCH₃, isopropyl, or CH₂CH₃. In another embodiment one of R^{B3} and R^{B4} is CH₂-phenyl-OMe and the other is CH₂-pyridine. In another embodiment one of R^{B3} and R^{B4} is CH₂OCH₃ and the other is C(=O)NH-CH₂-phenyl.

In some embodiments R^{B3} and R^{B4}together with N form an optionally substituted C₄₋₁₂ heterocyclyl or C₅₋₁₂ heteroaryl. In some embodiments the optional substituents are R^{B6} and are selected from C(=O)C₁₋₄ alkyl, C₁₋₄alkoxy, C₁₋₄ alkyl, NH₂, C₅₋₆ heteroaryl, C₄₋₆ heterocyclyl, or optionally substituted phenyl wherein the optional substituents on the phenyl are F and OMe.

In some embodiments when R^{B3} and R^{B4}together with N form an optionally substituted C₄₋₁₂ heterocyclyl or C₅₋₁₂ heteroaryl they form an optionally substituted C₅₋₁₀ heterocyclyl or a C₅₋₁₀ heteroaryl. In some embodiments they form a C₅ heterocyclyl containing a single N atom. In some embodiments they form a C₆ heterocylyl comprising one nitrogen atom and one oxygen atom, or two nitrogen atoms. In some embodiments they form a C₅ heteroaryl comprising 2 N atoms. In some embodiments they form fused rings with 9 or 10 atoms containing one or two nitrogen atoms and optionally one sulphur atom. In some embodiments they form a pyrazole.

In further embodiments R^{B3} and R^{B4} together with N form an optionally substituted morpholine, an optionally substituted pyrazole, an optionally substituted pyrrolidine, an optionally substituted piperazine, an optionally substituted oxazine, an optionally substituted 1,2,3,4,-tetrahydroquinolone, or an optionally substituted 6,7-dihydro-4H-thiazolo[5,4-c]pyridin-5-yl.

In some embodiments when R^{B3} and R^{B4}together with N form an optionally substituted C₄₋₁₂ heterocyclyl or C₅₋₁₂ heteroaryl the optional substituents are R^{B6} and are selected from C(=O)C₁₋₄ alkyl, C₁₋₄ alkyl, NH₂, C₁₋₄ alkoxy, C₅₋₆ heteroaryl, C₄₋₆ heterocyclyl, or optionally substituted phenyl wherein the optional substituents on the phenyl are F and methoxy.

In some embodiments when R^{B3} and R^{B4}together with N form an optionally substituted C₄₋₁₂ heterocyclyl or C₅₋₁₂ heteroaryl the optional substituents are R^{B6} and are selected from C₁₋₃ alkyl, C(=O)CH₃, CH₂OCH₃, OCH₃, NH₂, halogen, C₅₋₆ heteroaryl or phenyl wherein said phenyl is optionally substituted by methoxy or halogen.

In some embodiments R^{B3} and R^{B4} together form an optionally substituted piperazine, an optionally substituted morpholine, an optionally substituted pyrazole, an optionally substituted pyrrolidine, an optionally substituted piperidine, an optionally substituted oxazine, an optionally substituted pyrimidine, an optionally substituted 1,2,3,4,-tetrahydroquinolone, an optionally substituted 6,7-dihydro-4H-thiazolo[5,4-c]pyridin-5-yl, or an optionally substituted 1,2,3,4-tetrahydroisoquinoline, and the optional substituents are R^{B6} and R^{B6} and are selected from C₁₋₃ alkyl, C(=O)CH₃, CH₂OCH₃, OCH₃, NH₂, halogen, C₅₋₆ heteroaryl or phenyl wherein said phenyl is optionally substituted by methoxy or halogen.

In some embodiments the optional substituents are R^{B6} and are selected from methoxy, CH₂OCH₃, C(=O)Me, NH₂, methyl, phenyl optionally substituted with methoxy or F, a tetrazole or C(=O)-1,2,3,4,-tetrahydroquinolone substituted with OMe.

In some embodiments R^{B3} and R^{B4} together form a C₅ heterocyclic ring optionally substituted by phenyl which phenyl is optionally substituted by methoxy or F, C(=O)-1,2,3,4-tetrahydroisoquinoline substituted by methoxy, CH₂OCH₃ or a tetrazole. In some embodiments the C₅ heterocyclic ring is a pyrrolidine.

In some embodiments R^{B3} and R^{B4} together form a pyrazole which is optionally substituted with NH₂ and methyl. In some embodiments R^{B3} and R^{B4} together form an optionally substituted morpholine wherein the optional substituents are methyl, or phenyl. In some embodiments R^{B3} and R^{B4} together form a piperazine substituted by C(=O)CH₃. In some embodiments R^{B3} and R^{B4} together form a 6,7-dihydro-4H-thiazolo[5,4-c]pyridin-5-yl substituted by NH₂. In some embodiments R^{B3} and R^{B4} together form 1,2,3,4-tetrahydroisoquinoline substituted by methoxy.

In some embodiments the number of R^{B6}substituents on R^{B3} or R^{B4} is 0, 1, 2 or 3. In further embodiments the number of R^{B6} substituents on R^{B3} or R^{B4} is 1 or 2.

In some embodiments R^{B3} or R^{B4}are unsubstituted.

### R^{B5}

In some embodiments B' is of the formula: 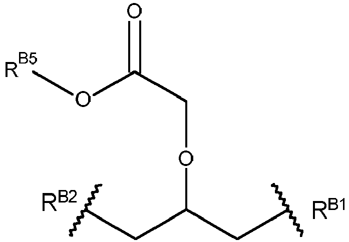 wherein R^{B5} is selected from H, C₁₋₆ alkyl, C₂₋₆ alkenyl or C₂₋₆ alkynyl.

In some embodiments R^{B5} is selected from H, C₁₋₃ alkyl or C₂₋₃ alkenyl.

In some embodiments R^{B5} is selected from H or C₁₋₃ alkyl.

In some embodiments R^{B5} is H, isopropyl or ethyl.

In some embodiments R^{B5} is H. In some embodiments R^{B5} is isopropyl. In some embodiments R^{B5} is ethyl.

### R^{B6}

In some embodiments *R*^{B6} is independently selected from:
i) hydroxy;
ii) oxo;
iii) carboxy;
iv) halogen;
v) trifluoromethyl;
vi) trifluoromethoxy;
vii) -NH₂;
viii) optionally substituted C₆₋₁₀ aryl;
ix) optionally substituted C₃₋₆ cycloalkyl;
x) optionally substituted C₄₋₁₀ heterocyclyl;
xi) optionally substituted C₅₋₁₂ heteroaryl;
xii) optionally substituted C₁₋₄ alkyl;
xiii) optionally substituted C₂₋₄ alkenyl;
xiv) optionally substituted C₂₋₄ alkynyl;
xv) optionally substituted C₁₋₄ alkoxy;
xvi) optionally substituted -C(=O)-(C₆₋₁₀ aryl);
xvii) optionally substituted -C(=O)-(C₃₋₆ cycloalkyl);
xviii) optionally substituted -C(=O)-(C₄₋₁₀ heterocyclyl);
xix) optionally substituted -C(=O)-(C₄₋₁₀ heteroaryl);
xx) optionally substituted -C(=O)-(C₁₋₄ alkyl);
xxi) optionally substituted -CH₂-(C₆₋₁₀ aryl);
xxii) optionally substituted -CH₂-(C₄₋₁₀ heterocyclyl);
xxiii) optionally substituted -(C=O)NH-CH₂-(C₆₋₁₀ aryl); and
xxiv) optionally substituted -(C=O)NH-CH₂-(C₅₋₁₂ heteroaryl);
wherein the optional substituents at R^{B6} are each independently selected from hydroxy, oxo, carboxy, halogen, trifluoromethyl, trifluoromethoxy, -NH₂, -NH(C₁₋₂ alkyl), -N(C₁₋₂ alkyl)₂, methyl, ethyl, CH₂OCH₃, methoxy, and ethoxy.

In some embodiments each R^{B6} is independently selected from:
i) hydroxy;
ii) oxo;
iii) carboxy;
iv) halogen;
v) trifluoromethyl;
vi) trifluoromethoxy;
vii) -NH₂;
viii) optionally substituted C₆₋₁₀ aryl;
ix) optionally substituted C₃₋₆ cycloalkyl;
x) optionally substituted C₄₋₁₀ heterocyclyl;
xi) optionally substituted C₅₋₁₂ heteroaryl;
xii) optionally substituted C₁₋₄ alkyl;
xiii) optionally substituted C₁₋₄ alkoxy;
xiv) optionally substituted -C(=O)-(C₁₋₄ alkyl);
xv) optionally substituted -C(=O)-(C₄₋₁₀ heterocyclyl);
xvi) optionally substituted -CH₂-(C₆₋₁₀ aryl);
xvii) optionally substituted -CH₂-(C₄₋₁₀ heterocyclyl);
xviii) optionally substituted -(C=O)NH-CH₂-(C₆₋₁₀ aryl); and
xix) optionally substituted -(C=O)NH-CH₂-(C₅₋₁₂ heteroaryl);
wherein the optional substituents at R^{B6} are each independently selected from hydroxy, oxo, carboxy, halogen, trifluoromethyl, trifluoromethoxy, -NH₂, -NH(C₁₋₂ alkyl), -N(C₁₋₂ alkyl)₂, methyl, ethyl, CH₂OCH₃, methoxy, and ethoxy.

In some embodiments each R^{B6} is independently selected from:
i) -NH₂;
ii) optionally substituted C₆₋₁₀ aryl;
iii) optionally substituted C₅₋₁₂ heteroaryl;
iv) optionally substituted C₁₋₄ alkyl;
v) optionally substituted C₁₋₄ alkoxy;
vi) optionally substituted -C(=O)-(C₁₋₄ alkyl);
vii) optionally substituted -(C=O)NH-CH₂-(C₆₋₁₀ aryl);
viii) optionally substituted -C(=O)-(C₄₋₁₀ heterocyclyl); and
wherein the optional substituents at R^{B6} are each independently selected from hydroxy, oxo, carboxy, halogen, trifluoromethyl, trifluoromethoxy, -NH₂, -NH(C₁₋₂ alkyl), -N(C₁₋₂ alkyl)₂, methyl, ethyl, CH₂OCH₃, methoxy, and ethoxy.

In some embodiments each R^{B6} is independently selected from:
i) -NH₂;
ii) C₁₋₃ alkyl, optionally substituted with -OMe;
iii) C(=O)CH₃;
iv) OCH₃,
v) halogen;
vi) C₅₋₆ heterocyclyl;
vii) C₅₋₆ heteroaryl;
viii) phenyl which phenyl is optionally substituted by 1-2 substituents selected from -OCH₃ or halogen;
ix) optionally substituted -(C=O)NH-CH₂-(C₆₋₁₀ aryl); or
x) C(=O)-1 ,2,3,4-tetrahydroisoquinoline substituted by methoxy.

In some embodiments when R^{B6} is halogen it is Cl, Br or F. In some embodiments R^{B6} is F.

In some embodiments when R^{B6} is optionally substituted C₆₋₁₀ aryl it is optionally substituted phenyl. In some embodiments the optional substituents are selected from halogen, trifluoromethyl, trifluoromethoxy, -NH₂, -NH(C₁₋₂ alkyl), -N(C₁₋₂ alkyl)₂, methyl, ethyl, methoxy, and ethoxy. In further embodiments when R^{B6} is phenyl the optional substituents are selected from halogen or methoxy. In further embodiments when R^{B6} is phenyl the optional substituents are selected from F or methoxy. In some embodiments the phenyl is substituted by either one methoxy group or two F atoms.

In some embodiments when R^{B6} is optionally substituted C₃₋₆ cycloalkyl it is cyclopropyl, cyclobutyl or cyclopentyl.

In some embodiments when R^{B6} is optionally substituted C₄₋₁₀ heterocyclyl it is a C₅ or C₆ heterocyclyl containing one or two heteroatoms selected form O, S and N. In some embodiments when R^{B6} is optionally substituted C₄₋₁₀ heterocyclyl it is a tetrahydrofuran, 1,3-dioxolane, tetrahydrothiophene, an oxazole, thiazole, pyrrolidine, a pyrroline, a pyrrole, a pyrazolidine, imidazoline, a pyrazoline, a pyrazole, an imidazole, a triazole, piperidine, piperazine, tetrahydropyran, a pyran, a dioxane, a dioxine, thiane, a dithiane, a thiomorpholine, a thiazine, a thiopyran, morpholine or an oxazine.

In some embodiments when R^{B6} is optionally substituted C₅₋₁₂ heteroaryl it is selected from an optionally substituted pyrrole, pyridine, furan, thiophene, oxazole, isoxazole, isoxazine, oxadiazole, oxatriazole, thiazole, isothiazole, imidazole, pyrazole, pyridazine, pyrimidine, pyrazine, triazole, triazine or a tetrazole.

In some embodiments when R^{B6} is optionally substituted C₁₋₄alkyl, it is an optionally substituted methyl, ethyl, propyl or butyl. In some embodiments R^{B6} is optionally substituted methyl or ethyl. In some embodiments the optional substituents are selected from OH, halogen or C₁₋₂alkoxy. In some embodiments the optional substituents are selected from OH and OCH₃.

In some embodiments when R^{B6} is optionally substituted C₁₋₄alkoxy it is optionally substituted methoxy or ethoxy. In some embodiments R^{B6} is methoxy.

In some embodiments when R^{B6} is optionally substituted -C(=O)-(C₁₋₄ alkyl) it is C(=O)CH₃, C(=O)CH₂CH₃ or C(=O)CH₂CH₂CH₃. In some embodiments R^{B6} is C(=O)CH₃.

In some embodiments when R^{B6} is optionally substituted -C(=O)-(C₄₋₁₀ heterocyclyl) it is an optionally substituted -C(=O)C₆ heterocyclyl or a -C(=O)C₁₀ heterocyclyl. In some embodiments it is a C(=O)-fused C₁₀ heterocyclyl containing one to three heteroatoms selected from O, S and N. In some embodiments it is a C(=O)-fused C₁₀ heterocyclyl containing one or two nitrogen atoms. In some embodiments it is a C(=O)-1,2,3,4-tetrahydroisoquinoline. In some embodiments the optional substituents are selected from hydroxy, halogen, methyl, ethyl, methoxy, and ethoxy. In further embodiments the optional substituent is methoxy. In some embodiments R^{B6} is C(=O)-1,2,3,4-tetrahydroisoquinoline substituted by methoxy.

In some embodiments R^{B6} is optionally substituted -CH₂-(C₆₋₁₀ aryl);

In some embodiments R^{B6} is optionally substituted -CH₂-(C₄₋₁₀ heterocyclyl);

In some embodiments when R^{B6} is optionally substituted -(C=O)NH-CH₂-(C₆₋₁₀ aryl) it is -(C=O)NH-CH₂-(phenyl) or -(C=O)NH-CH₂-(naphthalene). In further embodiments when R^{B6} is optionally substituted -(C=O)NH-CH₂-(C₆₋₁₀ aryl) it is -(C=O)NH-CH₂-(phenyl).

In some embodiments R^{B6} is optionally substituted -(C=O)NH-CH₂-(C₅₋₁₂ heteroaryl);

In some embodiments R^{B6} is selected from: -NH₂; optionally substituted C₆₋₁₀ aryl; optionally substituted C₅₋₁₂ heteroaryl; optionally substituted C₁₋₄ alkyl; optionally substituted C₁₋₄alkoxy; optionally substituted - C(=O)-(Ci-₄alkyl); optionally substituted C(=O)C₁₀ heterocyclyl, optionally substituted -(C=O)NH-CH₂-(C₆₋₁₀ aryl); wherein the optional substituents at R^{B6} are each independently selected from hydroxy, oxo, carboxy, halogen, trifluoromethyl, trifluoromethoxy, -NH₂, methyl, ethyl, CH₂OCH₃, methoxy, and ethoxy.

In some embodiments R^{B6} is selected from phenyl which phenyl is optionally substituted by methoxy or one or more F atoms, methoxy, -(C=O)NH-CH₂-(phenyl), CH₂OCH₃, pyridyl, methyl, NH₂, C(=O)Me, C(=O)-1,2,3,4-tetrahydroisoquinoline substituted by methoxy, tetrazole.

### R^{B6} optional substituents

In some embodiments the optional substituents at R^{B6} are selected from OH, =O, C(=O)OH, Cl, F, Br, trifluoromethyl, trifluoromethoxy, -NH₂, -NH(CH₃), -N(CH₃)₂, methyl, ethyl, CH₂OCH₃, methoxy, and ethoxy.

In some embodiments the optional substituents at R^{B6} are selected from OH, Cl, F, methyl, ethyl, CH₂OCH₃, methoxy, and ethoxy.

In some embodiments the optional substituents at R^{B6} are selected from F and methoxy.

In some embodiments the optional substituent at R^{B6} is one or two F atoms.

In some embodiments the optional substituent at R^{B6} is methoxy.

In some embodiments the number of optional substituents on R^{B6} is 1 or 2.

In some embodiments the number of optional substituents on R^{B6} is 1.

In some embodiments B' is selected from one of the following groups:

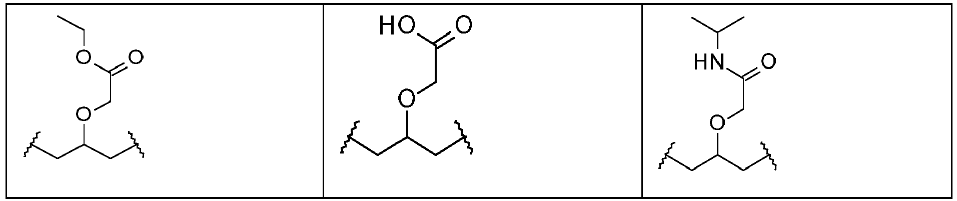

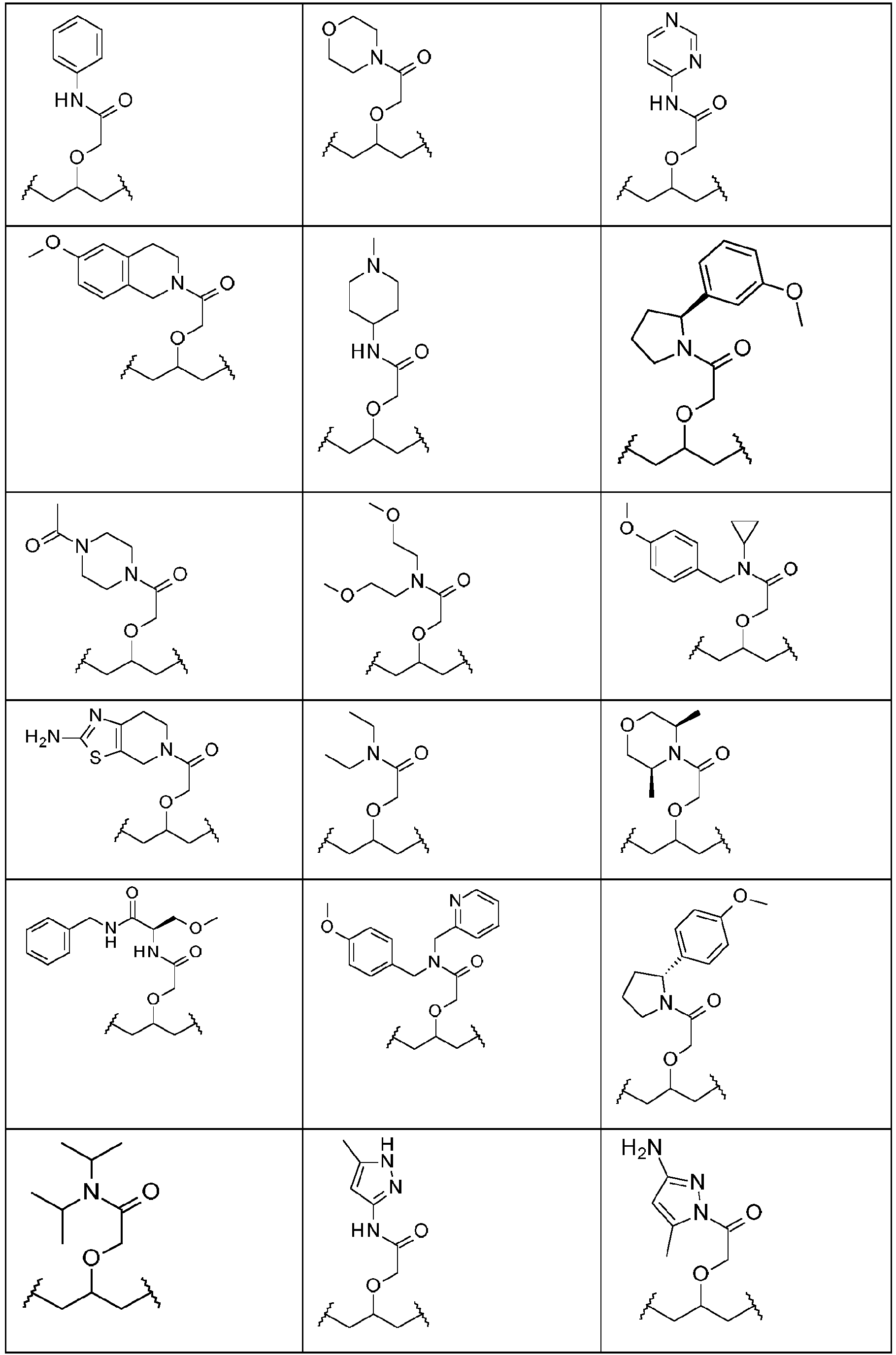

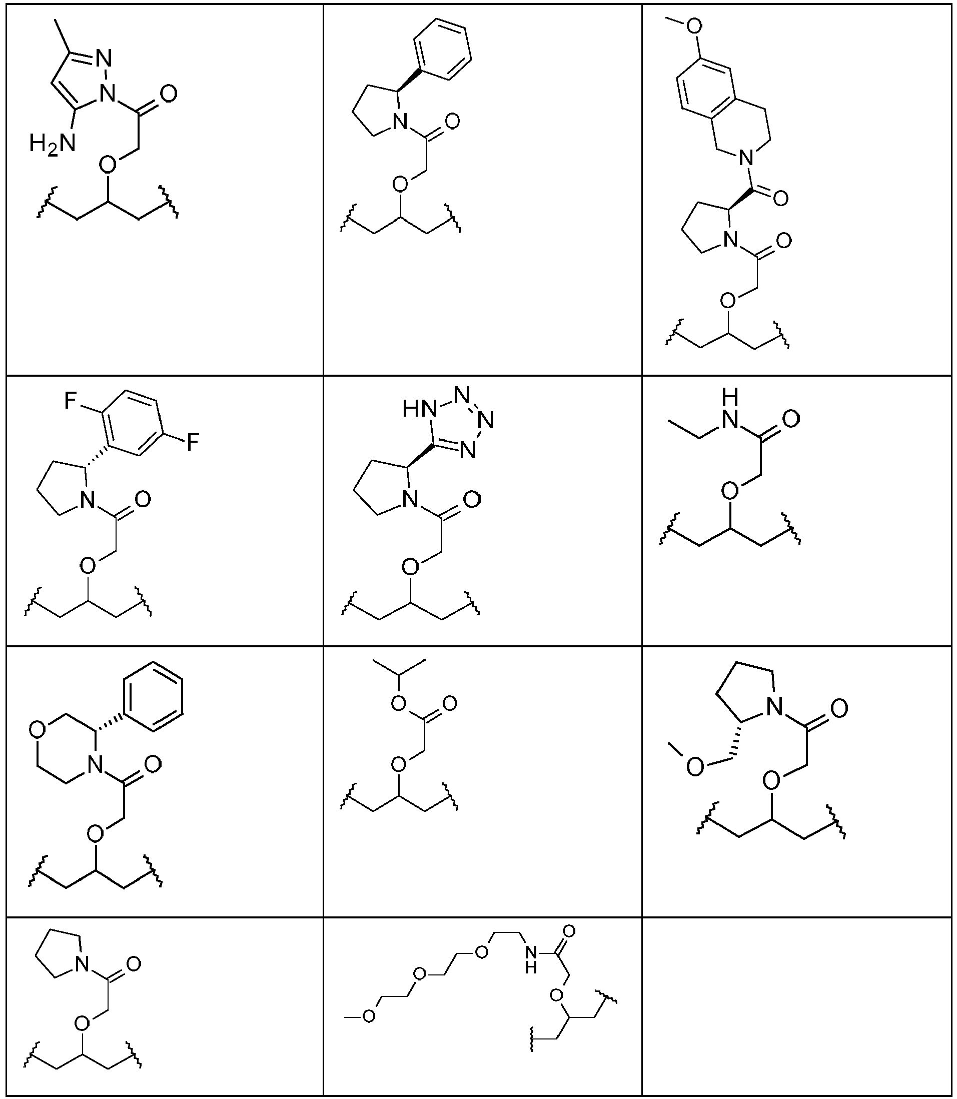

In further embodiments B' is selected from one of the following groups:

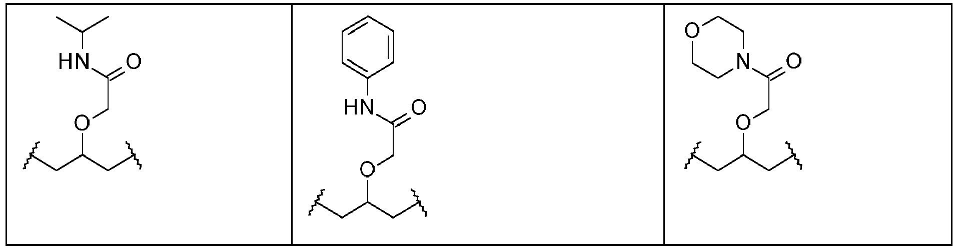

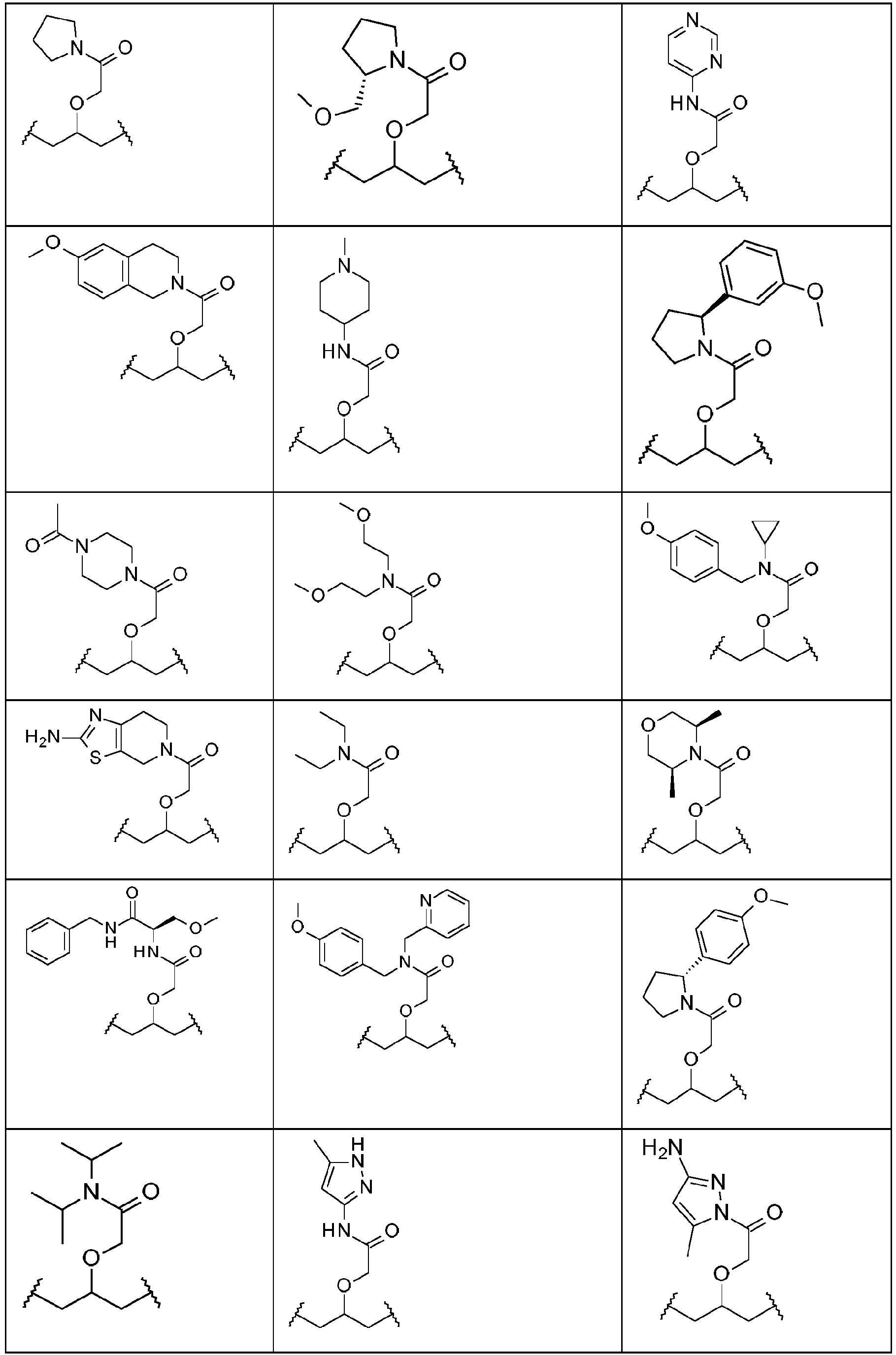

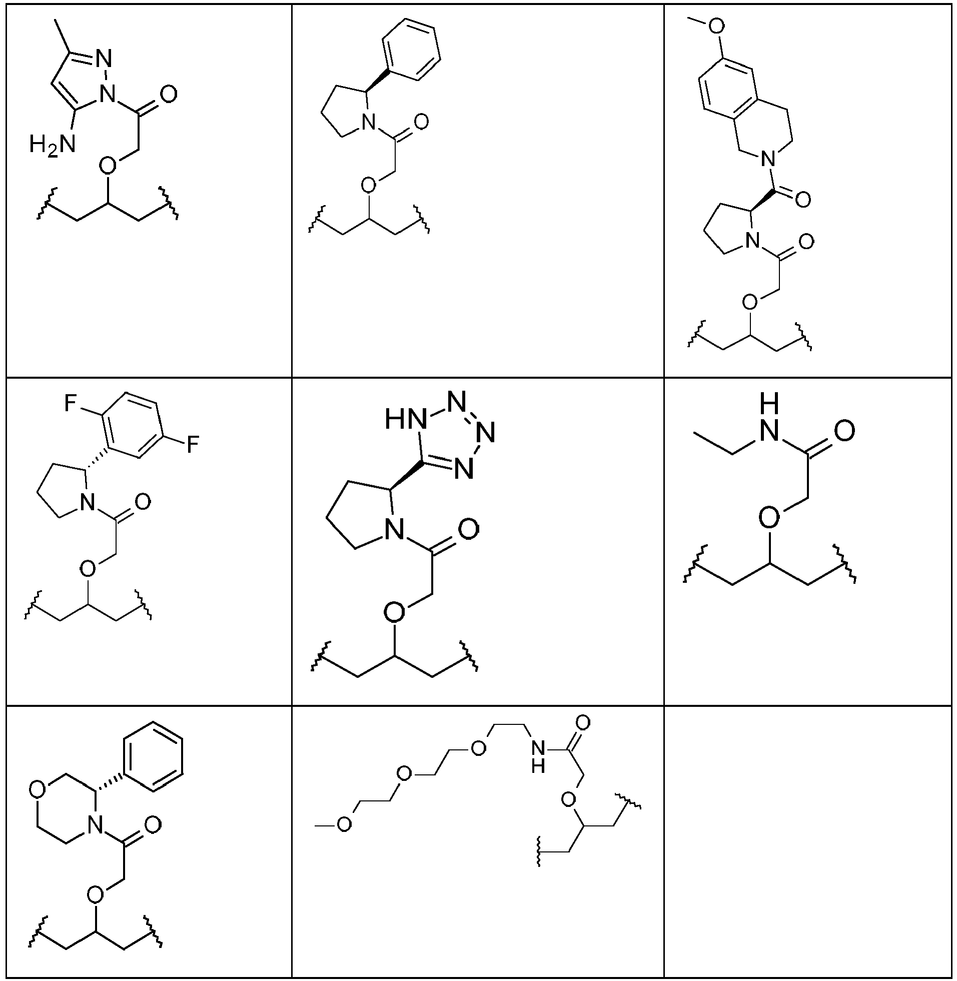

### Symmetrical compounds

In some embodiments R¹ is identical to R¹¹, R² is identical to R¹², R³ is identical to R¹³, R⁴ is identical to R¹⁴, R^{B1} is identical to R^{B2}, R⁸ is identical to R¹⁸ and R⁹ is identical to R¹⁹. In further embodiments R¹ is identical to R¹¹, R² is identical to R¹², R³ is identical to R¹³, R⁴ is identical to R¹⁴, R⁵ is identical to R¹⁵ , R^{B1} is identical to R^{B2}, R⁸ is identical to R¹⁸, R⁶ is identical to R¹⁶and R⁷ is identical to R¹⁷. Therefore, in some embodiments the compounds are symmetrical.

### Further embodiments

In some embodiments, R¹ and R¹¹ are selected from H, -OH or C₁₋₃ alkoxy;
R³ and R¹³ are selected from -C(=O)N(H)(C₁₋₃alkyl) or -C(=O)NH₂;
R⁴ and R¹⁴ are selected from H, halogen, OH or C₁₋₄ alkyl;
R⁷ and R¹⁷ are C₁₋₄ alkyl;
R⁵ and R¹⁵ are selected from H or C₁₋₄ alkyl;
R⁶ and R¹⁶ are H; and
R⁸ and R¹⁸ are methyl.

In further embodiments R¹ and R¹¹ are H;
R³ and R¹³ are -C(=O)NH₂ or -C(=O)NH-CH₃;
R⁷ and R¹⁷ are ethyl; and
R⁵ and R¹⁵ are methyl.

In some embodiments R¹, R¹¹, R⁶, R¹⁶, R⁴, R¹⁴, R² and R¹² are H;
R⁷ and R¹⁷ are ethyl;
R⁵, R¹⁵, R⁸ and R¹⁸ are methyl;
R³ and R¹³ are -C(=O)NH₂;
R^{B1} and R^{B2} are both -CH₂-;
b is 0;
a is 1;
X is -NR^{B3}R^{B4} or X is -OR^{B5};
R^{B5} is ethyl;
R^{B3} and R^{B4} are selected from H, ethyl, phenyl, pyrimidine, CH₂CH₂-O-CH₃, pyrazole substituted by methyl,
or R^{B3} and R^{B4} together form a group selected from a morpholine which is optionally substituted by phenyl, a 1,2,3,4-tetrahydroquinoline substituted with methoxy, a pyrrolidine substituted with a tetrazole or phenyl which phenyl is substituted with methoxy , or a 2-[(1,2,3,4-tetrahydro-2-isoquinolyl)carbonyl]pyrrolidine substituted with methoxy or a piperazine substituted with C(=O)CH₃.

In some embodiments compound (I-b) is of the Formula (I-2b): 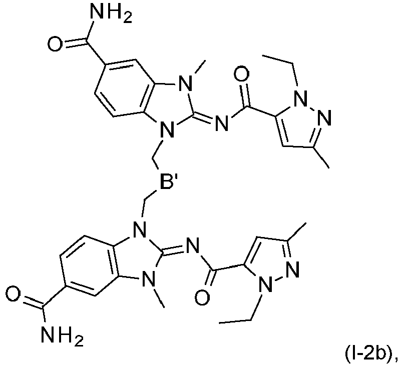 wherein B' is as defined above.

In some embodiments the compound is selected from the following compounds listed in Table 1:

**Table 1**

| **Example number** | **Structure** | **IUPAC name** |
|---|---|---|
| 1 | 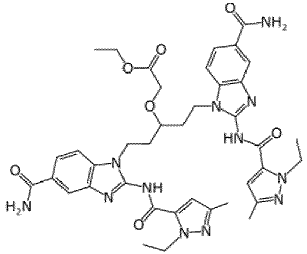 | Ethyl {3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetate |
| 2 | 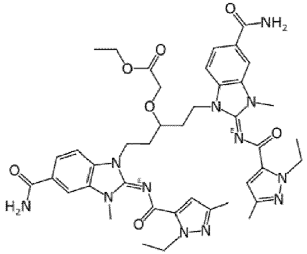 | Ethyl (3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetate |
| 3 | 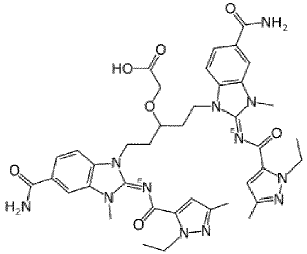 | (3-{5-Carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetic acid |
| 4 | 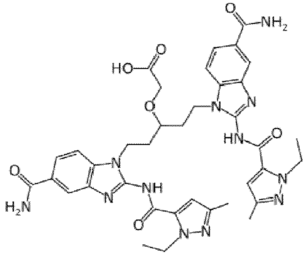 | {3-[5-Carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetic acid |
| 5 | 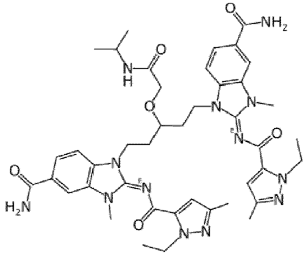 | 1-{3-[(N-lsopropylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 6 | 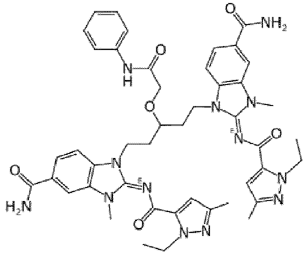 | 1-{3-[(N-Phenylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 7 | 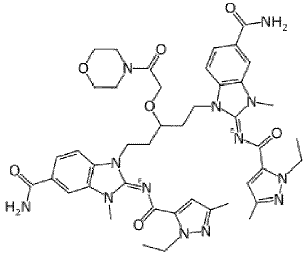 | 1-(5-{5-Carbamoyl-2-[(1-ethyl-3-methyl-5-pyrazolyl)carbonylimino]-3-methyl-2,3-dihydro-1H-1,3-benzimidazol-1-yl}-3-(2-morpholino-2-oxoethoxy)pentyl)-2-[(1-ethyl-3-methyl-5-pyrazolyl)carbonylimino]-3-methyl-2,3-dihydro-1H-1,3-benzimidazole-5-carboxamide |
| 8 | 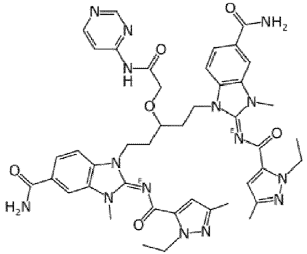 | 1-{3-[(N-4-Pyrimidinylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 9 | 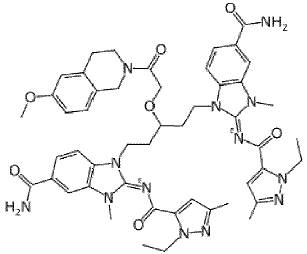 | 1-(5-{5-Carbamoyl-2-[(1-ethyl-3-methyl-5-pyrazolyl)carbonylimino]-3-methyl-2,3-dihydro-1H-1,3-benzimidazol-1-yl}-3-[2-(6-methoxy-1,2,3,4-tetrahydro-2-isoquinolyl)-2-oxoethoxy]pentyl)-2-[(1-ethyl-3-methyl-5-pyrazolyl)carbonylimino]-3-methyl-2,3-dihydro-1H-1 ,3-benzimidazole-5-carboxamide |
| 10 | 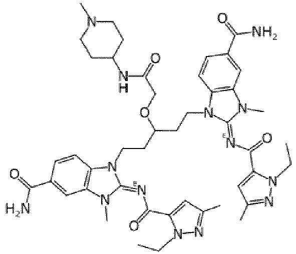 | 1-{3-[(N-1-Methyl-4-piperidylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 11 | 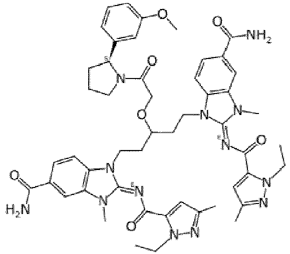 | 1-(3-{2-[(S)-2-(3-Methoxyphenyl)-1-pyrrolidinyl]-2-oxoethoxy}-5-{5-carbamoyl-2-[(1-ethyl-3-methyl-5-pyrazolyl)carbonylimino]-3-methyl-2,3-dihydro-1H-1 ,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 12 | 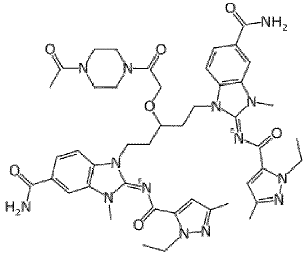 | 1-{3-[2-(4-Acetyl-1-piperazinyl)-2-oxoethoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 13 | 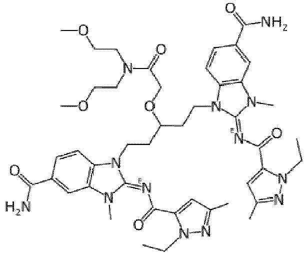 | 1-(3-{[Bis(2-methoxyethyl)carbamoyl]methoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 14 | 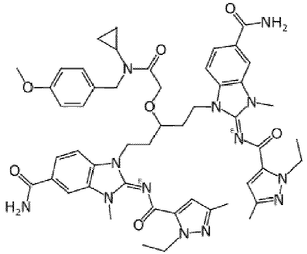 | 1-[3-({N-Cyclopropyl[(p-methoxyphenyl)methyl]carbamoyl}methoxy)-5-{5-carbamoyl-2-[(1-ethyl-3-methyl-5-pyrazolyl)carbonylimino]-3-methyl-2,3-dihydro-1H-1,3-benzimidazol-1-yl}pentyl]-2-[(1-ethyl-3-methyl-5-pyrazolyl)carbonylimino]-3-methyl-2,3-dihydro-1H-1,3-benzimidazole-5-carboxamide |
| 15 | 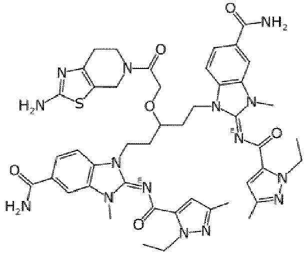 | 1-{3-[2-(2-Amino-4,5,6,7-tetrahydro-3-thia-1,5-diaza-5-indenyl)-2-oxoethoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 16 | 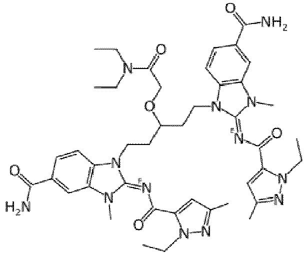 | 1-{3-[(N,N-Diethylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 17 | 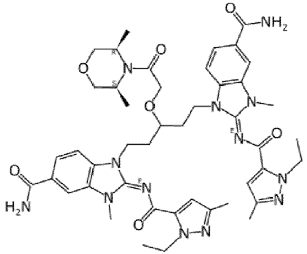 | 1-(3-{2-[(3R,5S)-3,5-Dimethyl-4-morpholinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 18 | 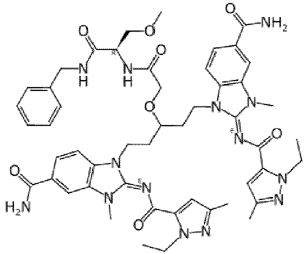 | 1-(3-{[N-(R)-1-(N-Benzylcarbamoyl)-2-methoxyethylcarbamoyl]methoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 19 | 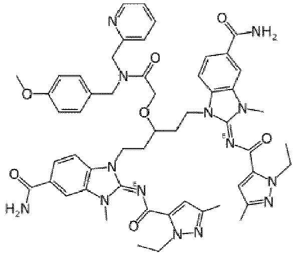 | 1-(5-{5-Carbamoyl-2-[(1-ethyl-3-methyl-5-pyrazolyl)carbonylimino]-3-methyl-2,3-dihydro-1H-1,3-benzimidazol-1-yl}-3-({[(p-methoxyphenyl)methyl][(2-pyridyl)methyl]carbamoyl}methoxy)pentyl)-2-[(1-ethyl-3-methyl-5-pyrazolyl)carbonylimino]-3-methyl-2,3-dihydro-1H-1,3-benzimidazole-5-carboxamide |
| 20 | 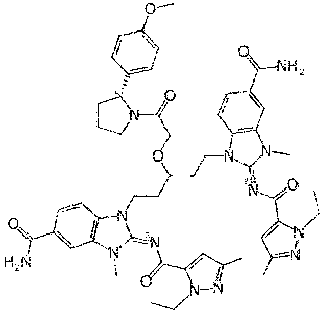 | 1-(3-{2-[(R)-2-(p-Methoxyphenyl)-1-pyrrolidinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 21 | 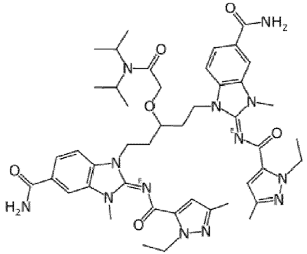 | 1-(3-{[Bis(isopropyl)carbamoyl]methoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 22 | 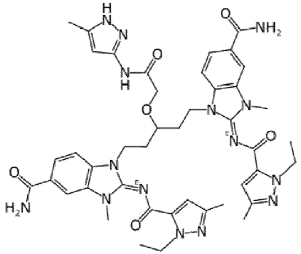 | 1-{3-[(N-5-Methyl-3-pyrazolylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 23 | 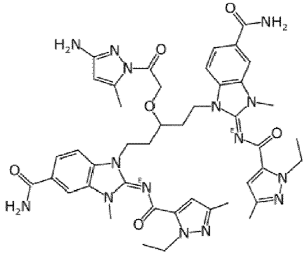 | 1-{3-[2-(3-Amino-5-methyl-1-pyrazolyl)-2-oxoethoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 24 | 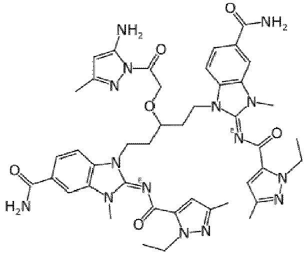 | 1-{3-[2-(5-Amino-3-methyl-1-pyrazolyl)-2-oxoethoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 25 | 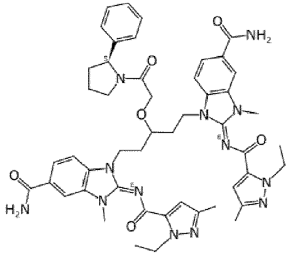 | 1-(3-{2-[(S)-2-Phenyl-1-pyrrolid inyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 26 | 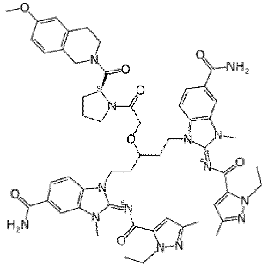 | 1-[3-(2-{(S)-2-[(6-Methoxy-1,2,3,4-tetrahydro-2-isoquinolyl)carbonyl]-1-pyrrolidinyl}-2-oxoethoxy)-5-{5-carbamoyl-2-[(1-ethyl-3-methyl-5-pyrazolyl)carbonylimino]-3-methyl-2,3-dihydro-1H-1,3-benzimidazol-1-yl}pentyl]-2-[(1-ethyl-3-methyl-5-pyrazolyl)carbonylimino]-3-methyl-2,3-dihydro-1H-1,3-benzimidazole-5-carboxamide |
| 27 | 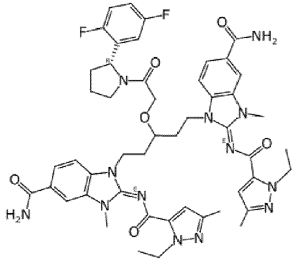 | 1-(3-{2-[(R)-2-(2,5-Difluorophenyl)-1-pyrrolidinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 28 | 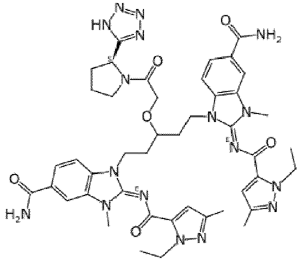 | 1-(3-{2-[(S)-2-(1H-Tetrazol-5-yl)-1-pyrrolidinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 29 | 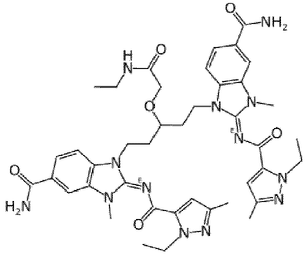 | 1-{3-[(N-Ethylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 30 | 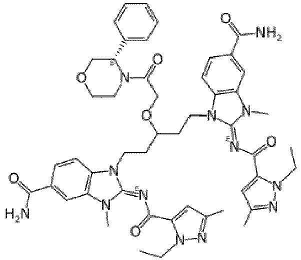 | 1-(3-{2-[(S)-3-Phenyl-4-morpholinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 31 | 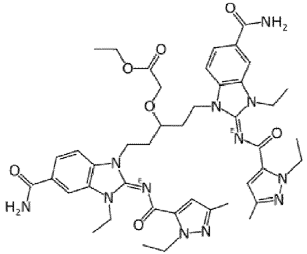 | Ethyl (3-{5-carbamoyl-3-ethyl-2-[(1-ethyl-3-methyl-5-pyrazolyl)carbonylimino]-2,3-dihydro-1H-1,3-benzimidazol-1-yl}-1-(2-{5-carbamoyl-3-ethyl-2-[(1-ethyl-3-methyl-5-pyrazolyl)carbonylimino]-2,3-dihydro-1H-1,3-benzimidazol-1-yl}ethyl)propoxy)acetate |
| 32 | 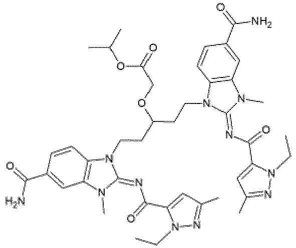 | Isopropyl (3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetate |
| 33 | 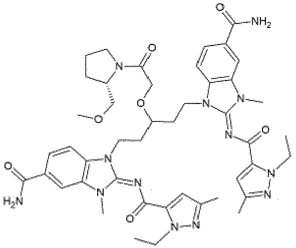 | 1-(3-{2-[(S)-2-(Methoxymethyl)-1-pyrrolidinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| 34 | 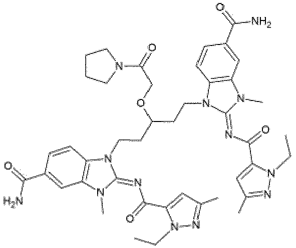 | 1-(5-{5-Carbamoyl-2-[(1-ethyl-3-methyl-5-pyrazolyl)carbonylimino]-3-methyl-2,3-dihydro-1H-1,3-benzimidazol-1-yl}-3-[2-oxo-2-(1-pyrrolidinyl)ethoxy]pentyl)-2-[(1-ethyl-3-methyl-5-pyrazolyl)carbonylimino]-3-methyl-2,3-dihydro-1H-1,3-benzimidazole-5-carboxamide |
| 35 | 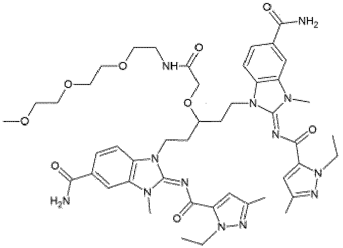 | N-3,6,9-Trioxadecyl(3-{5-carbamoyl-2-[(1 - ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetamide |
| 36 | 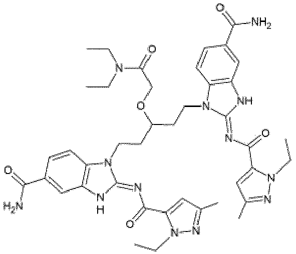 | 1-{3-[(N,N-Diethylcarbamoyl)methoxy]-5-{5-carbamoyl-2-[(1-ethyl-3-methyl-5-pyrazolyl)carbonylimino]-2,3-dihydro-1H-1,3-benzimidazol-1-yl}pentyl}-2-[(1-ethyl-3-methyl-5-pyrazolyl)carbonylimino]-2,3-dihydro-1H-1,3-benzimidazole-5-carboxamide |

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

All compounds of the present invention can be synthesized by those skilled in the art of organic synthesis. Non-standard procedures and syntheses of special building blocks and intermediates are described below.

### Materials and methods

### List of abbreviations

| | |
|---|---|
| aq | aqueous |
| Boc | *tert*-butyloxycarbonyl |
| BuOH | butanol |
| cat.# | catalogue number |
| CV | column volumes |
| DCM | dichloromethane |
| DIAD | diisopropyl azodicarboxylate |
| DIPEA | diisopropylethylamine; N-ethyl-N-(propan-2-yl)propan-2-aminel |
| DMAP | 4-(dimethylamino)pyridine |
| DMF | N,N-dimethylformamide |
| DMSO | dimethylsulfoxide |
| EDC | 1-(3-dimethylaminopropyl)-3-ethyl-carbodiimide hydrochloride |
| ESi | electrospray ionization |
| ESI | electrospray ionization |
| EtOAc | ethylacetate |
| EtOH | ethanol |
| FA | formic acid |
| HATU | 1-[bis(dimethylamino)methylene]-1H-1,2,3-triazolo[4,5-b]pyridinium 3-oxide hexafluorophosphate |
| HPLC | high-performance liquid chromatography |
| LCMS | liquid chromatography mass spectrometry |
| MeCN | acetonitrile |
| MeOH | methanol |
| MS | mass spectrometry; mass spectrum |
| NEt₃ | triethylamine |
| NMR | nuclear magnetic resonance spectroscopy |
| PDA | photo diode array |
| Ph | phenyl |
| PPh₃ | triphenylphosphine |
| rt | room temperature |
| Rt | retention time |
| SCX | strong cation exchange |
| SFC | supercritical fluid chromatography |
| TBAF | tetra-n-butylammonium fluoride |
| TBDMS | *tert*-butyldimethylsilyl |
| TBS | *tert*-butyldimethylsilyl |
| TLC | thin-layer chromatography |
| tBu | *tert*-butyl |
| TFA | trifluoroacetic acid |
| THF | tetrahydrofuran |
| TMS | tetramethylsilane or tetramethylsilyl |
| Ts | p-toluenesulfonyl |
| TsCl | p-toluenesulfonyl chloride |
| UPLC | ultra-performance liquid chromatography |

### LCMS methods

Analysis of products and intermediates was carried out using reverse phase analytical HPLC-MS using the parameters set out below.

**AnalpH2_MeCN_4min:** Agilent 1100 HPLC system with Waters ZQ mass detector; column: Waters Xbridge C18, 3.5 µm, 50 x 4.6 mm; column temperature 45 °C; A = water + 0.1% formic acid; B = MeCN + 0.1% formic acid; %B: 0.0 min 5%, 1.0 min 37.5%, 3.0 min 95%, 3.5 min 95%, 3.51 5%, 4.0 min 5%; flow 2.25 mL/min; PDA 210-400 nm and MS detection at 120-950 Da, ESi positive or negative.

**AnalpH9_MeCN_4min:** Agilent 1100 HPLC system with Waters ZQ mass detector; column: Waters Xbridge C18, 3.5 µm, 50 x 4.6 mm; column temperature 45 °C; A = 10 mM ammonium bicarbonate in water, pH 9; B = MeCN; %B: 0.0 min 5%, 1.0 min 37.5%, 3.0 min 95%, 3.5 min 95%, 3.51 5%, 4.0 min 5%; flow 2.25 mL/min; PDA 210-400 nm and MS detection at 120-950 Da, ESi positive or negative.

**UPLC_pH2_MeCN_2min:** Waters Acquity UPLC with Waters QDa mass detector; column: Waters BEH C18, 1.7 µm, 50 × 2.1 mm; A = water + 0.1% formic acid; B = MeCN + 0.1% formic acid; column temperature 50 °C; %B: 0.05 min 5%, 1.6 min 95%, 2.26min 5%; flow 0.6 mL/min; PDA 210-400 nm and MS detection at 120-1000 Da, ESi positive or negative.

**UPLC_pH9_MeCN_2min:** Waters Acquity UPLC with Waters QDa mass detector; column: Waters BEH C18, 1.7 µm, 50 x 2.1 mm; A = 10 mM ammonium bicarbonate in water, pH 9; B = MeCN; column temperature 50 °C; %B: 0.05 min 5%, 1.6 min 95%, 2.26 min 5%; flow 0.6 mL/min; PDA 210-400 nm and MS detection at 120-1000 Da, ESi positive or negative.

**UPLC_pH2_MeCN_QC_V1:** Waters Acquity UPLC with Waters QDa mass detector; column: Waters CSH C18, 1.7 µm, 100 x 2.1 mm; A = water + 0.1% formic acid; B = MeCN + 0.1% formic acid; column temperature 50 °C; %B: 0.05 min 5%, 5.00 min 95%, 6.60 min 5%; flow 0.35 mL/min; PDA 210-400 nm and MS detection at 120-1000 Da, ESi positive or negative.

**UPLC_pH2_MeCN_QC_V2:** Waters Acquity UPLC with Waters QDa mass detector; column: Waters CSH C18, 1.7 µm, 100 x 2.1 mm; A = water + 0.1% formic acid; B = MeCN + 0.1% formic acid; column temperature 50 °C; %B: 0.05 min 5%, 5.00 min 95%, 6.60 min 5%; flow 0.35 mL/min; PDA 210-400 nm and MS detection at 120-1250 Da, ESi positive or negative.

**UPLC_pH2_MeCN_QC_V3:** Waters Acquity UPLC with Waters QDa mass detector; column: Waters CSH C18, 1.7 µm, 100 x 2.1 mm; A = water + 0.1% formic acid; B = MeCN + 0.1% formic acid; column temperature 50 °C; %B: 0.05 min 5%, 5.00 min 95%, 6.60 min 5%; flow 0.35 mL/min; PDA 210-400 nm and MS detection at 250-2000 Da, ESi positive or negative.

**UPLC_pH9_MeCN_QC_V1:** Waters Acquity UPLC with Waters QDa mass detector; column: Waters CSH C18, 1.7 µm, 100 x 2.1 mm; A = 10 mM ammonium bicarbonate in water, pH 9; B = MeCN; column temperature 50 °C; %B: 0.05 min 5%, 5.00 min 95%, 6.60 min 5%; flow 0.35 mL/min; PDA 210-400 nm and MS detection at 120-1000 Da, ESi positive or negative.

**UPLC_pH9_MeCN_QC_V2:** Waters Acquity UPLC with Waters QDa mass detector; column: Waters CSH C18, 1.7 µm, 100 x 2.1 mm; A = 10 mM ammonium bicarbonate in water, pH 9; B = MeCN; column temperature 50 °C; %B: 0.05 min 5%, 5.00 min 95%, 6.60 min 5%; flow 0.35 mL/min; PDA 210-400 nm and MS detection at 120-1250 Da, ESi positive or negative.

**UPLC_pH9_MeCN_QC_V3:** Waters Acquity UPLC with Waters QDa mass detector; column: Waters CSH C18, 1.7 µm, 100 x 2.1 mm; A = 10 mM ammonium bicarbonate in water, pH 9; B = MeCN; column temperature 50 °C; %B: 0.05 min 5%, 5.00 min 95%, 6.60 min 5%; flow 0.35 mL/min; PDA 210-400 nm and MS detection at 250-2000, ESi positive or negative.

### ¹H-NMR spectroscopy

¹H NMR spectra were recorded at 400 MHz and 294 K if not stated otherwise. Chemical shifts (δ-values) are reported in parts per million (ppm), referenced to either tetramethylsilane (TMS) (0.0 ppm) or the corresponding solvent residual signal of DMSO (2.50 ppm in DMSO-d₆), chloroform (7.26 ppm in CDCl₃) or methanol (3.31 ppm in deuterated methanol). Coupling constants (J) are reported in Hertz (Hz), splitting patterns are designated as singlet (s), doublet (d), triplet (t), quadruplet (q), multiplet or more overlapping signals (m), broad signal (br); solvent is given in parentheses. ¹H-NMR integrations have been quantified when visible.

### Syntheses of building blocks, intermediates, probes and example compounds

Methods for preparing molecules similar to some of the compounds described below can be found in WO2017/175147, WO2019/069270 and J. M. Ramanjulu et al., Nature (2018), 564(7736), 439-443, Supplementary Information.

### Intermediate 3

### 1-Ethyl-3-methyl-1H-pyrazole-5-carbonyl isothiocyanate

### Synthesis:

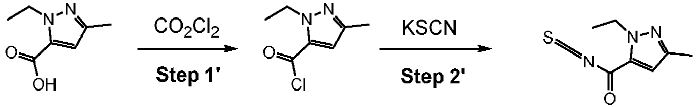

**Step 1':** To a stirred suspension of 1-ethyl-3-methyl-1H-pyrazole-5-carboxylic acid (4 g, 25.95 mmol) in anhydrous DCM (80 mL) at 0 °C were added oxalyl chloride (2.67 mL, 31.1 mmol) and DMF (0.2 mL, 2.59 mmol) dropwise. The reaction mixture was stirred at 0 °C for 15 min and then allowed to warm to rt (after 15 min the slurry turned into a light yellow solution). The reaction mixture was stirred for 1 h at rt. LCMS analysis of a sample dissolved in MeOH showed formation of the methyl ester and no starting material,
indicating completed formation of the acid chloride.
LCMS (AnalpH2_MeCN_4min): Rt = 2.19 min, m/z 169.2 ([M+H]⁺ of methyl ester)

Solvents were removed under reduced pressure. 20 mL of DCM was added and the mixture was concentrated again to dryness, affording 1-ethyl-3-methyl-1H-pyrazole-5-carbonyl chloride (4.50 g, 100%) as a yellow oil, which was taken forward to the next step without further purification.

**Step 2':** 1-Ethyl-3-methyl-1H-pyrazole-5-carbonyl chloride (4.48 g, 25.95 mmol) was placed under nitrogen and dry acetone (66 mL) was added. The reaction mixture was cooled down to 0 °C and potassium thiocyanate (3.03 g, 31.1 mmol) was added portionwise. The reaction mixture was stirred for 15 min at 0 °C and then for 1 h at rt. 50 mL of isohexane was added and the reaction mixture was concentrated in vacuo to dryness. To the black residue 100 mL of isohexane were added and the resulting dark brown solid was collected by filtration and washed with isohexane twice. The yellow filtrate was concentrated in vacuo affording 1-ethyl-3-methyl-1H-pyrazole-5-carbonyl isothiocyanate (Intermediate 3; 4.60 g, 91%) as a brown oil that was stored in a freezer to avoid degradation.
LCMS (AnalpH2_MeCN_4min): Rt = 2.06 min, m/z 196.2 [M+H]⁺ and Rt = 2.85 min, m/z 196.2 [M+H]⁺ (1:1 ratio of both peaks).
¹H-NMR (400 MHz, CDCl₃): δ 6.75 (s, 1H), 4.52 (q, J = 7.2 Hz, 2H), 2.31 (s, 4H), 1.42 (t, J = 7.1 Hz, 3H)

### Probe 1 (fluorescein-labelled STING ligand)

### N-{1-[4-(5-{N-3-(3',6'-Dihydroxy-3-oxospiro[isobenzofuran-1,9'-xanthen]-5-ylcarbonylamino)propylcarbamoyl}-2-(1-ethyl-3-methyl-5-pyrazolylcarbonylamino)-1H-1,3-benzimidazol-1-yl)butyl]-5-carbamoyl-1H-1,3-benzimidazol-2-yl}-1 -ethyl-3-methyl-5-pyrazolecarboxamide

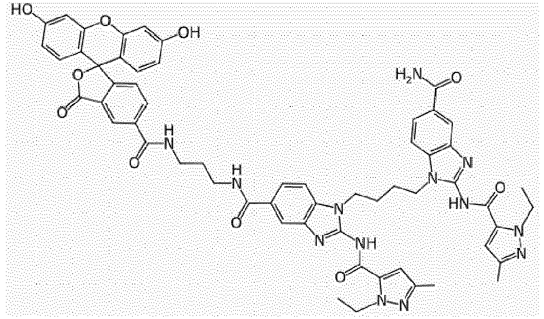

### Synthesis:

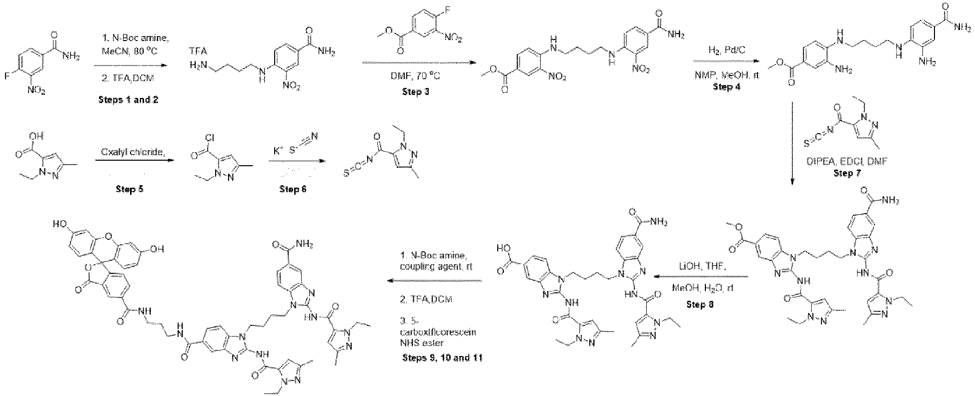

**Step 1:** To a suspension of 4-fluoro-3-nitrobenzamide (50 mg, 0.272 mmol) and 1-Boc-1 ,4-diaminobutane (51 mg, 0.272 mmol) in MeCN (1.4 mL) was added DIPEA (0.14 mL, 0.815 mmol). The reaction mixture was stirred at 80 °C for 6 h. The reaction mixture was concentrated under reduced pressure, affording 104 mg of crude tert-butyl (4-((4-carbamoyl-2-nitrophenyl)amino)butyl)carbamate (96 mg, 0.272 mmol, 100 %) as a yellow solid.
LCMS (UPLC_pH2_MeCN_2min): Rt = 1.57 min, m/z 353.1 [M+H]⁺

**Step 2:** To a stirred suspension of tert-butyl (4-((4-carbamoyl-2-nitrophenyl)amino)butyl)carbamate (96 mg, 0.272 mmol) in DCM (2.2 mL) was added TFA (0.54 mL) dropwise. The reaction mixture was stirred at rt for 4 h (slurry became a solution after TFA addition, but the compound precipitated again). The reaction mixture was concentrated under reduced pressure, affording the product as the TFA salt that was taken to the next step without further purification. Assumed quantitative, furnished 4-((4-aminobutyl)amino)-3-nitrobenzamide TFA salt as a yellow gum (100 mg, 0.272 mmol, 100 %).
LCMS (UPLC_pH9_MeCN_2min): Rt = 1.02 min, m/z 253.1 [M+H]⁺

**Step 3:** To a stirred solution of 4-(4-aminobutylamino)-3-nitro-benzamide;2,2,2-trifluoroacetic acid (796 mg, 2.17 mmol) in DMF (11 mL) were added methyl 4-fluoro-3-nitrobenzoate (433 mg, 2.17 mmol) and DIPEA (1.9 mL, 10.9 mmol). The reaction mixture was stirred at 70 °C for 6 h and then cooled down to rt and poured into ice-cold water. The resulting solid was isolated by filtration and dried under vacuum, affording methyl 4-((4-((4-carbamoyl-2-nitrophenyl)amino)butyl)amino)-3-nitrobenzoate (865 mg, 2.01 mmol, 92 %) as an orange solid.
LCMS (UPLC_pH9_MeCN_2min): Rt = 1.68 min, m/z 432.1 [M+H]⁺
¹H-NMR (400 MHz, DMSO-d₆): δ 8.64-8.57 (m, 3H), 8.40 (t, J = 5.7 Hz, 1H), 7.97 (ddd, J = 14.0, 9.2, 2.1 Hz, 3H), 7.27 (s, 1H), 7.14 (dd, J = 22.4, 9.2 Hz, 2H), 3.83 (s, 3H), 3.49-3.46 (m, 4H), 1.77-1.72 (m, 4H).

**Step 4:** Methyl 4-((4-((4-carbamoyl-2-nitrophenyl)amino)butyl)amino)-3-nitrobenzoate (300 mg, 0.695 mmol) was suspended in 1-methyl-2-pyrrolidinone (36 mL) and MeOH (2.2 mL). The reaction was vac/ filled with nitrogen three times. Then, palladium on carbon (74 mg, 0.695 mmol) was added under nitrogen and the reaction mixture was vac/ filled with hydrogen three times leaving a balloon of hydrogen. The reaction mixture was stirred at rt overnight. The reaction mixture was filtered through celite, eluting with MeOH. The filtrate was concentrated under reduced pressure. To the residue was added diethylether and the resulting solid was isolated by filtration and dried under vacuum, furnishing methyl 3-amino-4-((4-((2-amino-4-carbamoylphenyl)amino)butyl)amino)benzoate (224 mg, 0.603 mmol, 87 %) as a dark grey solid.
LCMS (UPLC_pH9_MeCN_2min): Rt = 1.37 min, m/z 372.2 [M+H]⁺

**Step 7:** To a stirred solution of methyl 3-amino-4-((4-((2-amino-4-carbamoylphenyl)amino)butyl)amino)benzoate (50 mg, 0.135 mmol) in DMF (0.67 mL) at 0 °C was added 1-ethyl-3-methyl-1H-pyrazole-5-carbonyl isothiocyanate (Intermediate 3; 53 mg, 0.269 mmol) dropwise. The reaction mixture was stirred at 0 °C for 20 min, and an LCMS sample was taken to confirm the formation of the thiourea intermediate. To the reaction mixture at 0 °C were then EDC (77 mg, 0.404 mmol) and DIPEA (0.14 mL, 0.808 mmol) dropwise. The reaction mixture was stirred at rt overnight. The reaction mixture was poured into ice-cold water dropwise. The resulting solid was collected by filtration, affording a gum that was redissolved in DCM/MeOH and concentrated under reduced pressure, affording methyl 1-(4-(5-carbamoyl-2-(1-ethyl-3-methyl-1H-pyrazole-5-carboxamido)-1H-benzo[d]imidazol-1 - yl)butyl)-2-(1-ethyl-3-methyl-1H-pyrazole-5-carboxamido)-1H-benzo[d]imidazole-5-carboxylate (30 mg, 0.0432 mmol, 32 %) as a yellow solid.
LCMS (UPLC_pH9_MeCN_2min): Rt = 1.60 min, m/z 694.3 [M+H]⁺

**Step 8:** To a stirred solution of methyl 1-(4-(5-carbamoyl-2-(1-ethyl-3-methyl-1H-pyrazole-5-carboxamido)-1H-benzo[d]imidazol-1-yl)butyl)-2-(1-ethyl-3-methyl-1H-pyrazole-5-carboxamido)-1H-benzo[d]imidazole-5-carboxylate (516 mg, 0.744 mmol) in MeOH (3.0 mL) and THF (3.0 mL) was added 1M aq LiOH (1.5 mL, 7.44 mmol) dropwise. The reaction mixture was stirred at rt for 48 h. THF and MeOH were removed under reduced pressure and the residue was acidified to pH 4 with aq 1M HCl. The resulting solid was collected by filtration, affording 1-(4-(5-carbamoyl-2-(1-ethyl-3-methyl-1H-pyrazole-5-carboxamido)-1H-benzo[d]imidazol-1-yl)butyl)-2-(1-ethyl-3-methyl-1H-pyrazole-5-carboxamido)-1H-benzo[d]imidazole-5-carboxylic acid (500 mg, 0.664 mmol, 89 %) as a grey solid.
LCMS (UPLC_pH9_MeCN_2min): Rt = 1.25 min, m/z 680.3 [M+H]⁺
¹H-NMR (400 MHz, DMSO-d₆): δ δ 8.07 (s, 1H), 7.97 (s, 1H), 7.94 (s, 1H), 7.82 (d, J = 6.9 Hz, 1H), 7.76 (d, J = 9.6 Hz, 1H), 7.57-7.53 (m, 2H), 7.30 (s, 1H), 6.59 (s, 1H), 6.58 (s, 1H), 4.56 (q, J = 6.9 Hz, 4H), 4.31-4.23 (s, 4H), 2.09 (s, 6H), 1.90-1.82 (s, 4H), 1.30 (t, J = 7.1 Hz, 6H).

**Step 9:** To a stirred solution of 1-(4-(5-carbamoyl-2-(1-ethyl-3-methyl-1H-pyrazole-5-carboxamido)-1H-benzo[d]imidazol-1-yl)butyl)-2-(1-ethyl-3-methyl-1H-pyrazole-5-carboxamido)-1H-benzo[d]imidazole-5-carboxylic acid (50 mg, 0.0736 mmol) and N-Boc-1,3-diaminopropane (15 mg, 0.0883 mmol) in DMSO (2.0 mL) was added DIPEA (0.077 mL, 0.441 mmol). The reaction mixture was stirred for 10 min and then HATU (34 mg, 0.0883 mmol) was added. The reaction mixture was diluted with DMSO and purified by preparative HPLC, affording tert-butyl (3-(1-(4-(5-carbamoyl-2-(1-ethyl-3-methyl-1H-pyrazole-5-carboxamido)-1H-benzo[d]imidazol-1-yl)butyl)-2-(1-ethyl-3-methyl-1H-pyrazole-5-carboxamido)-1H-benzo[d]imidazole-5-carboxamido)propyl)carbamate (36 mg, 0.0388 mmol, 53 %) as a white solid.
LCMS (UPLC_pH9_MeCN_2min): Rt = 1.62 min, m/z 836.4 [M+H]⁺

**Step 10:** To a stirred solution/suspension of tert-butyl (3-(1-(4-(5-carbamoyl-2-(1-ethyl-3-methyl-1H-pyrazole-5-carboxamido)-1H-benzo[d]imidazol-1-yl)butyl)-2-(1-ethyl-3-methyl-1H-pyrazole-5-carboxamido)-1H-benzo[d]imidazole-5-carboxamido)propyl)carbamate (36 mg, 0.0388 mmol) in DCM (7.1 mL) at rt was added TFA (0.71 mL) dropwise. The reaction mixture was stirred at rt for 1 h. The solvent was removed under reduced pressure, affording a crude containing N-(3-aminopropyl)-1-(4-(5-carbamoyl-2-(1-ethyl-3-methyl-1H-pyrazole-5-carboxamido)-1H-benzo[d]imidazol-1-yl)butyl)-2-(1-ethyl-3-methyl-1H-pyrazole-5-carboxamido)-1H-benzo[d]imidazole-5-carboxamide TFA salt (42 mg, 0.0388 mmol, assumed 100 %) as a light yellow gum.
UPLC-MS, pH9 MeCN, 2.6 min method:
LCMS (UPLC_pH9_MeCN_2min): Rt = 1.47 min, m/z 736.3 [M+H]⁺

**Step 11:** To N-(3-aminopropyl)-1-(4-(5-carbamoyl-2-(1-ethyl-3-methyl-1H-pyrazole-5-carboxamido)-1H-benzo[d]imidazol-1-yl)butyl)-2-(1-ethyl-3-methyl-1H-pyrazole-5-carboxamido)-1H-benzo[d]imidazole-5-carboxamide TFA salt (42 mg, 0.0388 mmol) in DMF (0.40 mL) was added triethylamine (0.032 mL, 0.233 mmol). Then, 5-carboxyfluorescein-N-hydroxysuccinimide ester (18 mg, 0.0388 mmol) in DMF (0.40 mL) was added dropwise, and the reaction mixture was stirred at rt for 1 h. The reaction mixture was purified by preparative HPLC, affording N-{1-[4-(5-{N-3-(3',6'-dihydroxy-3-oxospiro[isobenzofuran-1,9'-xanthen]-5-ylcarbonylamino)propylcarbamoyl}-2-(1-ethyl-3-methyl-5-pyrazolylcarbonylamino)-1H-1,3-benzimidazol-1-yl)butyl]-5-carbamoyl-1H-1,3-benzimidazol-2-yl}-1 -ethyl-3-methyl-5-pyrazolecarboxamide (Probe 1; 37 mg, 0.0334 mmol, 86 %) as an orange solid.
LCMS (UPLC_pH2_QC_V1): Rt = 4.20 min, m/z 1094.2 [M+H]⁺
¹H-NMR (400 MHz, DMSO-d₆): δ 8.85 (t, J = 5.6 Hz, 1H), 8.47-8.51 (m, 2H), 8.18 (d, J = 8.0 Hz, 1H), 7.95-7.96 (s with shoulder, 3H), 7.73 (t, J = 8.8 Hz, 2H), 7.53 (t, J = 9.2 Hz, 2H), 7.34 (d, J = 8.0 Hz with shoulder, 2H), 6.58-6.63 (m, 6H), 6.50 (d, J = 8.8 Hz, 2H), 4.56 (q, J = 7.0 Hz, 4H), 4.26 (br s, 4H), 3.25-3.45 (m, overlapping with H₂O peak at 3.30 ppm), 2.09 (s, 6H), 1.80-1.91 (m, 6H), 1.30 (t, J = 7.1 Hz, 6H).

### Example 1 (Building Block G-1)

### Ethyl {3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetate

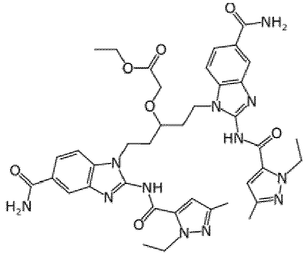

### Synthesis:

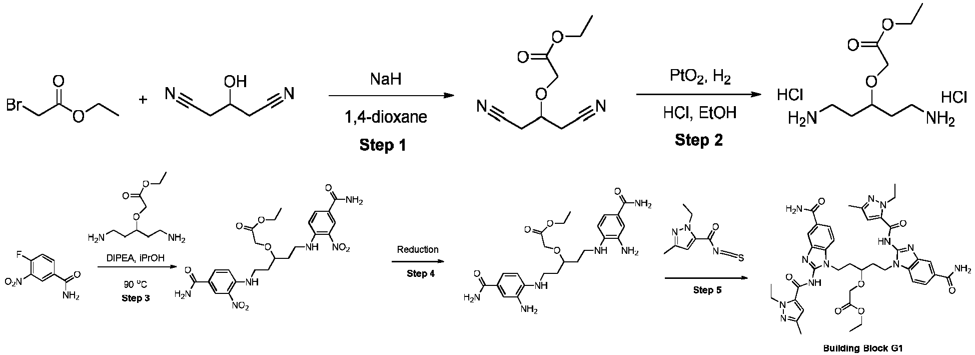

**Step 1:** To a stirred solution of 3-hydroxypentanedinitrile (5.00 g, 45.4 mmol) in dry 1,4-dioxane (200 mL) at 5 °C was added sodium hydride (60% dispersion in oil) (1816 mg, 45.4 mmol) portion-wise. The reaction mixture was stirred at rt for 1 h. Then, the reaction mixture was cooled down to 5 °C and ethyl bromoacetate (5.1 mL, 45.4 mmol) was added dropwise. The reaction mixture was stirred at rt overnight. The reaction mixture was cooled down to 5 °C and EtOH was added to quench any residual NaH. The reaction mixture was concentrated under reduced pressure and purified by column chromatography (SiO₂, Biotage, sfar, 350 g), eluting from 100% isohexane to 100% EtOAc, affording ethyl [2-cyano-1-(cyanomethyl)ethoxy]acetate (2.65 g, 13.5 mmol, 30%) as a light yellow oil.
¹H-NMR (400 MHz, CDCl₃): δ 4.33 (s, 2H), 4.26 (q, J = 7.2 Hz, 2H), 4.06-4.12 (m, 1H), 2.89 (s, 4H), 1.33 (t, J = 7.3 Hz, 3H).

**Step 2:** To a stirred solution of ethyl [2-cyano-1-(cyanomethyl)ethoxy]acetate (474 mg, 2.42 mmol) in ethanol (24 mL) was added hydrochloric acid (5 M solution in water, 1.4 mL, 7.0 mmol). The reaction mixture was vac / filled with nitrogen three times. Then, platinum (IV) oxide, Adam's catalyst (110 mg, 0.483 mmol) was added under N₂ and the reaction mixture was vac / filled with hydrogen three times. The reaction mixture was stirred at rt under 1 atm of hydrogen. Consumption of starting material was monitored by TLC. After reaction overnight, TLC showed complete consumption of starting material (EtOAc/isohexane 1:1) and the reaction mixture was filtered through a pad of celite eluting with EtOH and DCM. The filtrate was concentrated under reduced pressure, affording ethyl [3-amino-1-(2-aminoethyl)propoxy]acetate dihydrochloride (670 mg, 2.42 mmol, 100%) as a light yellow gum.
¹H-NMR (400 MHz, DMSO-d₆): δ 8.06 (4H, br s), 4.11-4.17 (m, 4H), 3.64-3.69 (m, 1H), 2.87 (t, J = 7.1 Hz, 4H), 1.81-1.73 (m, 4H), 1.22 (t, J = 7.1 Hz, 3H)

**Step 3:** To a stirred solution of 4-fluoro-3-nitrobenzamide (891 mg, 4.84 mmol) and ethyl [3-amino-1-(2-aminoethyl)propoxy]acetate dihydrochloride (670 mg, 2.42 mmol) in isopropanol (12 mL) was added DIPEA (4.2 mL, 24.2 mmol). The reaction mixture was stirred at 90 °C. After 1 h, the reaction mixture turned orange and a solid precipitate was observed. The reaction mixture was cooled down to 0 °C, diethylether was added and the solid was isolated by filtration, affording ethyl {3-(4-carbamoyl-2-nitrophenylamino)-1-[2-(4-carbamoyl-2-nitrophenylamino)ethyl]propoxy}acetate (626 mg, 1.18 mmol, 49%) as an orange solid.
LCMS (UPLC_pH9_MeCN_2min): Rt = 1.46 min, m/z 533.1 [M+H]⁺

**Step 4:** A stirred solution of ethyl {3-(4-carbamoyl-2-nitrophenylamino)-1-[2-(4-carbamoyl-2-nitrophenylamino)ethyl]propoxy}acetate (3.10 g, 5.82 mmol) in DMF (531 mL) was treated with 35% aq ammonia (9.7 mL) and then a solution of sodium dithionite (10.14 g, 58.2 mmol) in water (317 mL) was added dropwise at 0 °C. The reaction mixture was stirred at rt until consumption of the starting material was observed by LCMS. The resulting white precipitate was removed by filtration and discarded. The filtrate was concentrated under reduced pressure. MeOH and diethylether were added, the solid formed was collected by filtration and triturated further with DCM to give ethyl {3-(2-amino-4-carbamoylphenylamino)-1-[2-(2-amino-4-carbamoylphenylamino)ethyl]propoxy}acetate (2.75 g, 5.82 mmol, 99%) as a light brown solid.
LCMS (UPLC_pH9_MeCN_2min): Rt = 0.97-1.22 min, m/z 473.2 [M+H]⁺

**Step 5:** To a stirred solution of ethyl {3-(2-amino-4-carbamoylphenylamino)-1-[2-(2-amino-4-carbamoylphenylamino)ethyl]propoxy}acetate (2.75 g, 5.82 mmol) in DMF (29 mL) was added 1-ethyl-3-methyl-1H-pyrazole-5-carbonyl isothiocyanate (Intermediate 3; 2.5 g, 12.8 mmol) at 0 °C. The mixture was stirred for 30 min at 0 °C and formation of the thiourea intermediate was confirmed by LCMS. EDC (3.35 g, 17.46 mmol) and DIPEA (6.08mL, 34.92 mmol) were added and the mixture was stirred at rt overnight. The resulting mixture was dropped into half saturated aqueous NH₄CI solution and stirred for 1 h. The resulting precipitate was collected by filtration, washed with water and dried, affording ethyl {3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetate (Building Block G-1; 2.60 g, 3.27 mmol, 56%) as an off white solid.
LCMS (UPLC_pH2_MeCN_2min): Rt = 1.48 min, m/z 795.4 [M+H]⁺
¹H-NMR (400 MHz, DMSO-d₆): δ 8.00 (d, *J* = 1.6 Hz, 2H), 7.95 (s, 2H), 7.78 (dd, *J* = 8.5, 1.6 Hz, 2H), 7.52 (d, *J* = 8.2 Hz, 2H), 7.31 (s, 2H), 6.60 (s, 2H), 4.58 (q, *J* = 7.2 Hz, 4H), 4.25-4.40 (m, 4H), 4.17 (s, 2H), 4.10 (q, *J* = 7.2 Hz, 2H), 3.68-3.74 (m, 1H), 2.08-2.13 (m, 4H), 2.07 (s, 6H), 1.31 (t, *J* = 7.1 Hz, 6H), 1.16 (t, *J* = 7.1 Hz, 3H)

### Example 2 (Building Block G-2)

### Ethyl (3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetate

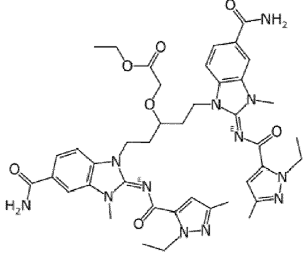

To a stirred solution of ethyl {3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetate (Building Block G-1; 700 mg, 0.881 mmol) in DMF (4.2 mL) were added cesium carbonate (1435 mg, 4.40 mmol) and iodomethane (0.14 mL, 2.20 mmol). The mixture was stirred overnight. The solid was filtered off and the filtrate was purified by preparative HPLC (column: Waters Xbridge C18 250 mm x 19 mm, 5 µm; method: increasing gradient of 20% to 60% MeCN in water containing 0.1% formic acid at pH 2), affording ethyl (3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetate (Building Block G-2; 307 mg, 42%), as an off white solid.
LCMS (UPLC_pH2_MeCN_2min): Rt = 1.40 min, m/z 823.4 [M+H]⁺
¹H-NMR (400 MHz, DMSO-d₆): δ 8.06 (d, *J* = 1.6 Hz, 2H), 8.02 (s, 2H), 7.84 (dd, *J* = 8.5, 1.4 Hz, 2H), 7.55 (d, *J* = 8.2 Hz, 2H), 7.42 (s, 2H), 6.47 (s, 2H), 4.49 (q, *J* = 7.2 Hz, 4H), 4.14-4.28 (m, 4H), 3.99-4.05 (m, 4H), 3.51-3.60 (m, 7H), 2.10 (s, 6H), 1.95-1.99 (m, 4H), 1.27 (t, *J* = 7.1 Hz, 6H), 1.13 (t, *J* = 7.1 Hz, 3H)

### Example 3 (Building Block G-4)

### (3-{5-Carbamoyl-2-[(1-ethyl-3-methyl-5-pyrazolyl)carbonylimino]-3-methyl-2,3-dihydro-1H-1,3-benzimidazol-1-yl)-1-(2-{5-carbamoyl-2-[(1-ethyl-3-methyl-5-pyrazolyl)carbonylimino]-3-methyl-2,3-dihydro-1H-1,3-benzimidazol-1-yl)ethyl)propoxy)acetic acid

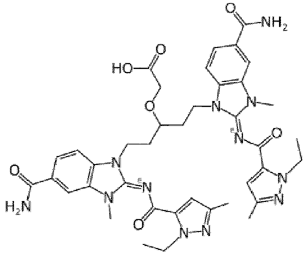

To a stirred solution of ethyl (3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetate (Building Block G-2; 35 mg, 0.0425 mmol) in EtOH (0.21 mL) and THF (0.21 mL) was added 1M aqueous lithium hydroxide (0.213 mL, 0.213 mmol) dropwise. The mixture was stirred overnight at rt. pH was adjusted to 4-6 by careful addition of 1M aq HCl and the reaction mixture was concentrated under reduced pressure, affording (3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetic acid (Building Block G-4; 33.8 mg) as an off-white gum.
LCMS (UPLC_pH2_MeCN_2min): Rt = 1.29 min, m/z 795.3 [M+H]⁺

Batch purified by preparative HPLC (column: SunFire C18 OBD 250 mm x 19 mm, 5 µm; method: 10-50% MeCN in water containing 0.1% formic acid at pH 2), affording an off-white solid.
¹H-NMR (400 MHz, DMSO-d₆): δ 8.05 (s, 2H), 8.02 (s, 2H), 7.84 (d, *J* = 8.4 Hz, 2H), 7.58 (d, *J* = 8.4 Hz, 2H), 7.41 (s, 2H), 6.48 (s, 2H), 4.49 (q, J = 6.9 Hz, 4H), 4.15-4.30 (m, 4H), 3.92 (s, 2H), 3.55-3.59 (m, 1H), 3.55 (s, 6H), 2.09 (s, 6H), 1.92-1.99 (m, 4H), 1.26 (t, *J* = 7.2 Hz, 6H)

### Example 4 (Building Block G-3)

### {3-[5-Carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetic acid

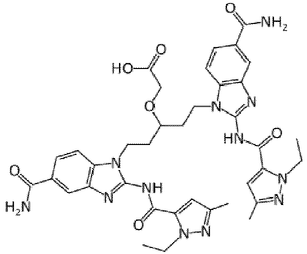

To a stirred solution of ethyl {3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetate (Building Block G-1; 200 mg, 0.252 mmol) in EtOH (0.63 mL) and THF (0.63 mL) was added was added 1M aqueous lithium hydroxide (1.26 mL, 1.26 mmol) dropwise. The mixture was stirred overnight at rt. pH was adjusted to 4-6 by careful addition of 1M aq HCI and the reaction mixture was concentrated under reduced pressure. One third of the mixture was purified by preparative HPLC (column: SunFire C18 OBD 250 mm x 19 mm, 5 µm; method: 10-50% MeCN in water containing 0.1% formic acid at pH 2). Fractions containing the desired product were combined and freeze-dried, affording {3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}acetic acid (Building Block G-3; 25 mg, 0.0326 mmol) as an off-white solid.
LCMS (UPLC_pH2_MeCN_QC_V2): Rt = 3.47 min, m/z+ 767.4 [M+H]⁺
¹H-NMR (400 MHz, DMSO-d₆): δ 7.99 (d, *J* = 1.6 Hz, 2H), 7.95 (s, 2H), 7.77 (dd, *J* = 8.2, 1.4 Hz, 2H), 7.54 (d, *J* = 8.2 Hz, 2H), 7.31 (s, 2H), 6.60 (s, 2H), 4.58 (q, *J* = 7.2 Hz, 4H), 4.25-4.41 (m, 4H), 4.10 (s, 2H), 3.69-3.75 (m, 1H), 2.07-2.11 (m, 4H), 2.07 (s, 3H), 1.31 (t, *J* = 7.1 Hz, 6H)

### Example 5

### 1-{3-[(N-Isopropylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1, 3-benzimidazole-5-carboxamide

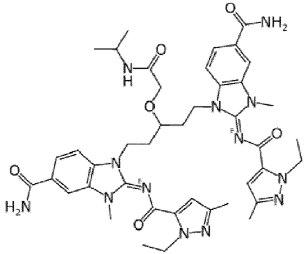

(3-{5-Carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetic acid (Building Block G-4; 30 mg, 0.0377 mmol) was dissolved in dry dimethyl sulfoxide (0.30 mL). Isopropylamine (0.0032 mL, 0.0377 mmol) was added followed by DIPEA (0.020 mL, 0.113 mmol) and HATU (18 mg, 0.0453 mmol). The mixture was stirred at rt for 1 h. The solution was diluted with DMSO (1 mL) and purified by preparative HPLC (column: SunFire C18 OBD 250 mm x 19 mm, 5 µm; method: 10-50% MeCN in water containing 0.1% formic acid at pH 2), affording 1-{3-[(N-isopropylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1 -ethyl-3-methyl-5-pyrazolyl)carbonylimino]-3-methyl-2,3-dihydro-1H-1,3-benzimidazole-5-carboxamide (17 mg, 54%) as a white solid.
LCMS (UPLC_pH2_MeCN_QC_V2): Rt = 3.51 min, m/z 836.5 [M+H]⁺
¹H-NMR (400 MHz, DMSO-d₆): δ 8.03 (d, *J* = 1.2 Hz, 2H), 8.03 (s, 2H), 7.83 (dd, *J* = 8.2, 1.4 Hz, 2H), 7.54 (d, *J* = 8.8 Hz, 2H), 7.42 (s, 2H), 7.32 (d, *J* = 8.4 Hz, 1H), 6.48 (s, 2H), 4.50 (q, *J* = 7.1 Hz, 4H), 4.15-4.26 (m, 4H), 3.82-3.91 (m, 1H), 3.67 (s, 2H), 3.55 (s, 6H), 3.48-3.53 (m, 1H), 2.09 (s, 6H), 1.98-2.03 (m, 4H), 1.26 (t, *J* = 7.0 Hz, 6H), 1.03 (d, *J* = 6.4 Hz, 6H)

### Example 6

### 1-{3-[(N-Phenylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1, 3-benzimidazole-5-carboxamide

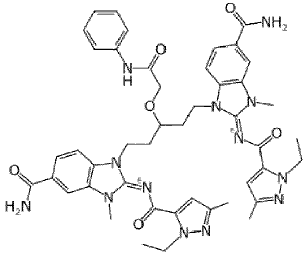

Prepared similarly to Example 5 from Building Block G-4 and the corresponding commercially available amine (aniline).
LCMS (UPLC_pH2_MeCN_QC_V2): Rt = 3.74 min, m/z 870.5 [M+H)]⁺
¹H-NMR (400 MHz, DMSO-d₆): δ 9.49 (s, 1H), 8.04 (d, J = 1.2 Hz, 2H), 8.01 (s, 2H), 7.83 (dd, J = 8.7, 1.2 Hz, 2H), 7.56-7.59 (m, 4H), 7.41 (s, 2H), 7.28-7.31 (m, 2H), 7.05-7.09 (m, 1H), 6.48 (s, 2H), 4.49 (q, J = 7.2 Hz, 4H), 4.24-4.28 (m, 4H), 3.88 (s, 2H), 3.60-3.65 (m, 1H), 3.51 (s, 6H), 2.00-2.08 (m) overlapping with 2.05 (s, together 10H), 1.26 (t, J = 7.2 Hz, 6H)

### Example 7

### 1-(5-{5-Carbamoyl-2-[(1-ethyl-3-methyl-5-pyrazolyl)carbonylimino]-3-methyl-2,3-dihydro-1H-1,3-benzimidazol-1-yl}-3-(2-morpholino-2-oxoethoxy)pentyl)-2-[(1-ethyl-3-methyl-5-pyrazolyl)carbonylimino]-3-methyl-2,3-dihydro-1H-1, 3-benzimidazole-5-carboxamide

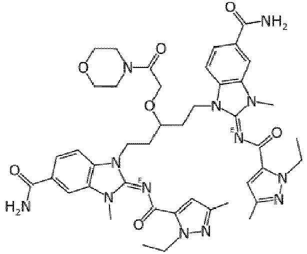

Prepared similarly to Example 5 from Building Block G-4 and the corresponding commercially available amine (morpholine).
LCMS (UPLC_pH2_MeCN_QC_V2): Rt = 3.28 min, m/z 864.4 [M+H]⁺
¹H-NMR (400 MHz, DMSO-d₆): δ 8.06 (d, J = 1.6 Hz, 2H), 8.03 (br s, 2H), 7.84 (dd, J = 8.2, 1.4 Hz, 2H), 7.57 (d, J = 8.2 Hz, 2H), 7.42 (br s, 2H), 6.49 (s, 2H), 4.50 (q, J = 7.2 Hz, 4H), 4.15-4.30 (m, 4H), 4.05 (s, 2H), 3.35-3.58 (m, 13H), 3.12 (br s, 2H), 2.10 (s, 6H), 1.95-2.00 (m, 4H), 1.27 (t, J = 7.2 Hz, 6H)

### Example 8

### 1-{3-[(N-4-Pyrimidinylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

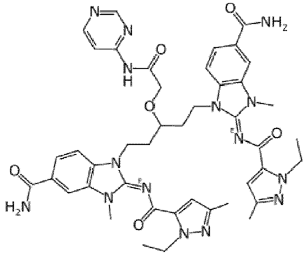

Prepared similarly to Example 5 from Building Block G-4 and the corresponding commercially available amine (4-pyrimidinylamine).
LCMS (UPLC_pH2_MeCN_QC_V2): Rt = 3.30 min, m/z 872.5 [M+H]+

### Example 9

### 1-(5-{5-Carbamoyl-2-[(1-ethyl-3-methyl-5-pyrazolyl)carbonylimino]-3-methyl-2,3-dihydro-1H-1,3-benzimidazol-1-yl)-3-[2-(6-methoxy-1,2,3, 4-tetrahydro-2-isoquinolyl)-2-oxoethoxyjpentyl)-2-[(1-ethyl-3-methyl-5-pyrazolyl)carbonylimino]-3-methyl-2,3-dihydro-1H-1,3-benzimidazole-5-carboxamide

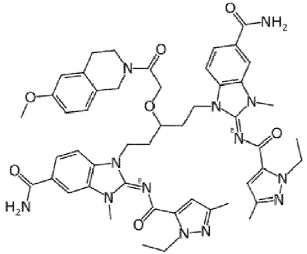

Prepared similarly to Example 5 from Building Block G-4 and the corresponding commercially available amine (6-methoxy-1,2,3,4-tetrahydroisoquinoline).
LCMS (UPLC_pH2_MeCN_QC_V2): Rt = 3.80 min, m/z 940.6 [M+H]+

### Example 10

### 1-{3-[(N-1-Methyl-4-piperidylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

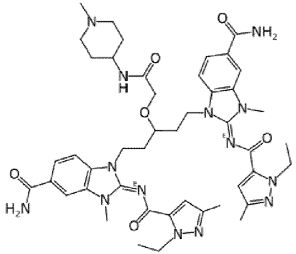

Prepared similarly to Example 5 from Building Block G-4 and the corresponding commercially available amine (1-methyl-4-piperidylamine).LCMS (UPLC_pH2_MeCN_QC_V2): Rt = 2.68 min, m/z 891.5 [M+H]+

### Example 11

### 1-(3-{2-[(S)-2-(3-Methoxyphenyl)-1-pyrrolidinyl}-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide

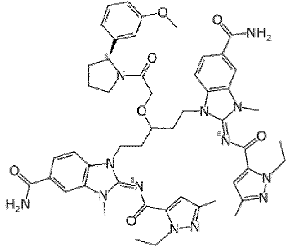

Prepared similarly to Example 5 from Building Block G-4 and the corresponding commercially available amine (S)-(2-(3-methoxyphenyl)pyrrolidine).
LCMS (UPLC_pH9_MeCN_QC_V2): Rt = 3.97 min, m/z 954.5 [M+H]⁺

### Example 12

### 1-{3-[2-(4-Acetyl-1-piperazinyl)-2-oxoethoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

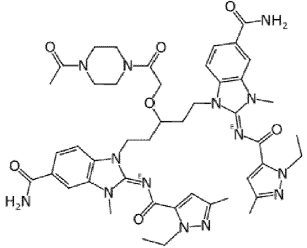

Prepared similarly to Example 5 from Building Block G-4 and the corresponding commercially available amine (1-(1-piperazinyl)-1-ethanone).
LCMS (UPLC_pH9_MeCN_QC_V2): Rt = 3.26 min, m/z 905.5 [M+H]⁺

### Example 13

### 1-(3-{[Bis(2-methoxyethyl)carbamoyl]methoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

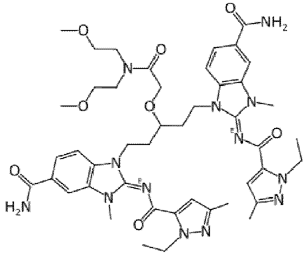

Prepared similarly to Example 5 from Building Block G-4 and the corresponding commercially available amine (2,8-dioxa-5-azanonane).
LCMS (UPLC_pH2_MeCN_QC_V2): Rt = 3.49 min, m/z 910.5 [M+H]⁺

### Example 14

### 1-[3-({N-Cyclopropyl[(p-methoxyphenyl)methyl]carbamoyl}methoxy)-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

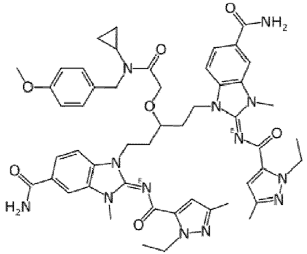

Prepared similarly to Example 5 from Building Block G-4 and the corresponding commercially available amine ([(p-methoxyphenyl)methyl]cyclopropylamine).
LCMS (UPLC_pH2_MeCN_QC_V3): Rt = 3.93 min, m/z 954.6 [M+H]⁺

### Example 15

### 1-{3-[2-(2-Amino-4,5,6,7-tetrahydro-3-thia-1,5-diaza-5-indenyl)-2-oxoethoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

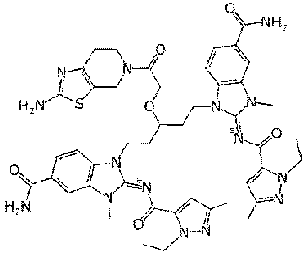

Prepared similarly to Example 5 from Building Block G-4 and the corresponding commercially available amine (4,5,6,7-tetrahydro-3-thia-1,5-diaza-2-indenylamine).
LCMS (UPLC_pH9_MeCN_QC_V3): Rt = 3.21 min, m/z 932.7 [M+H]⁺

### Example 16

### 1-{3-[(N,N-Diethylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

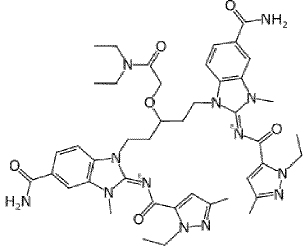

Prepared similarly to Example 5 from Building Block G-4 and the corresponding commercially available amine (N-ethylethanamine).
LCMS (UPLC_pH2_MeCN_QC_V2): Rt = 3.49 min, m/z 850.4 [M+H]⁺

### Example 17

### 1-(3-{2-[(3R,5S)-3,5-Dimethyl-4-morpholinyl}-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

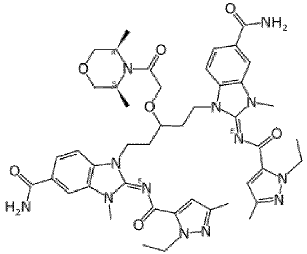

Prepared similarly to Example 5 from Building Block G-4 and the corresponding commercially available amine (3R,5S)-(3,5-dimethylmorpholine).
LCMS (UPLC_pH9_MeCN_QC_V2): Rt = 3.55 min, m/z 892.5 [M+H]+

### Example 18

### 1-(3-{[N-(R)-1-(N-Benzylcarbamoyl)-2-methoxyethylcarbamoyl]methoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

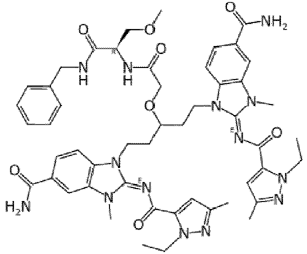

Prepared similarly to Example 5 from Building Block G-4 and the corresponding commercially available amine (N-benzyl-(R)-2-amino-3-methoxypropionamide).
LCMS (UPLC_pH2_MeCN_QC_V3): Rt = 3.68 min, m/z 985.7 [M+H]⁺

### Example 19

### 1-(5-{5-Carbamoyl-2-[(1-ethyl-3-methyl-5-pyrazolyl)carbonylimino]-3-methyl-2,3-dihydro-1H-1,3-benzimidazol-1-yl)-3-(([(p-methoxyphenyl)methyl][(2-pyridyl)methyl]carbamoyl)methoxy)pentyl)-2-[(1-ethyl-3-methyl-5-pyrazolyl)carbonylimino]-3-methyl-2,3-dihydro-1H-1,3-benzimidazole-5-carboxamide

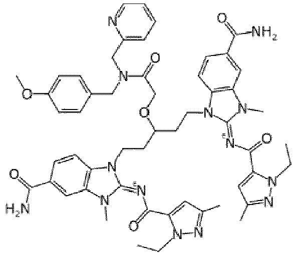

Prepared similarly to Example 5 from Building Block G-4 and the corresponding commercially available amine ([(p-methoxyphenyl)methyl][(2-pyridyl)methyl]amine).
LCMS (UPLC_pH2_MeCN_QC_V3): Rt = 3.59 min, m/z 1005.7 [M+H]⁺

### Example 20

### 1-(3-{2-[(R)-2-(p-Methoxyphenyl)-1-pyrrolidinyl]-2-oxoethoxy}-5-(5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

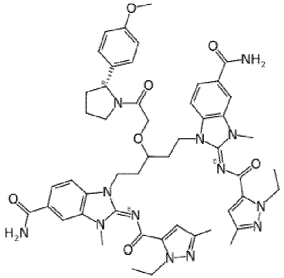

Prepared similarly to Example 5 from Building Block G-4 and the corresponding commercially available amine ((R)-2-(4-methoxyphenyl)pyrrolidine).
LCMS (UPLC_pH2_MeCN_QC_V2): Rt = 3.81 min, m/z 954.6 [M+H]⁺

### Example 21

### 1-(3-{[Bis(isopropyl)carbamoyl]methoxy}-5-(5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide

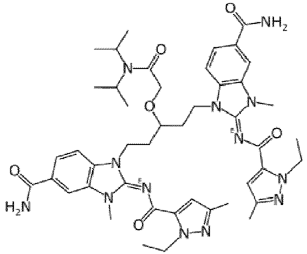

Prepared similarly to Example 5 from Building Block G-4 and the corresponding commercially available amine (bis(isopropyl)amine).
LCMS (UPLC_pH2_MeCN_QC_V2): Rt = 3.85 min, m/z 878.6 [M+H]⁺

### Example 22

### 1-{3-[(N-5-Methyl-3-pyrazolylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

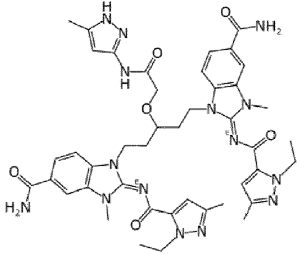

Prepared similarly to Example 5 from Building Block G-4 and 3-amino-5-methylpyrazole, first of three isomers isolated by preparative HPLC.
LCMS (UPLC_pH2_MeCN_QC_V2): Rt = 3.29 min, m/z 874.5 [M+H]⁺
¹H-NMR (400 MHz, DMSO-d₆): δ 12.01 (br s, 1H), 9.79 (s, 0.5H), 8.41 (s, 0.5H; formate), 8.03 (d, *J* = 1.6 Hz, 2H), 8.00 (s, 2H), 7.82 (dd, *J* = 8.4, 1.6 Hz, 2H), 7.56 (d, *J* = 8.8 Hz, 2H), 7.40 (s, 2H), 6.47 (s, 2H), 6.23 (s, 1H), 4.48 (q, *J* = 7.1 Hz, 4H), 4.23-4.25 (m, 4H), 3.95 (s, 2H), 3.56-3.62 (m, 1H), 3.53 (s, 6H), 2.18 (s, 3H), 1.98-2.06 (m, 10H), 1.24 (t, *J* = 7.2 Hz, 6H)

### Example 23

### 1-{3-[2-(3-Amino-5-methyl-1-pyrazolyl)-2-oxoethoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

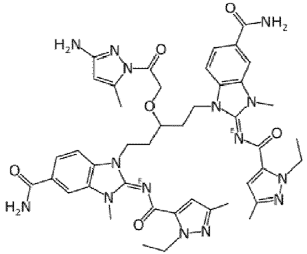

Prepared similarly to Example 5 from Building Block G-4 and 3-amino-5-methylpyrazole, second of three isomers isolated by preparative HPLC.
LCMS (UPLC_pH2_MeCN_QC_V2): Rt = 3.40 min, m/z 874.5 [M+H]⁺
   NMR?
¹H-NMR (400 MHz, DMSO-d₆): δ 8.05 (d, *J* = 1.6 Hz, 2H), 8.03 (s, 2H), 7.83 (dd, *J* = 8.8, 1.2 Hz, 2H), 7.58 (d, *J* = 8.4 Hz, 2H), 7.43 (s, 2H), 6.45 (s, 2H), 5.67 (s, 1H), 5.48 (s, 2H), 4.63 (s, 2H), 4.46 (q, *J* = 7.1 Hz, 4H), 4.18-4.33 (m, 4H), 3.64-3.70 (m, 1H), 3.54 (s, 6H), 2.37 (s, 3H), 2.04 (s, 6H), 1.96-2.01 (m, 4H), 1.23 (t, *J* = 7.0 Hz, 6H)

### Example 24

### 1-{3-[2-(5-Amino-3-methyl-1-pyrazolyl)-2-oxoethoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide

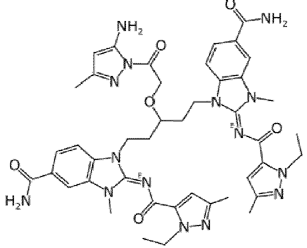

Prepared similarly to Example 5 from Building Block G-4 and 3-amino-5-methylpyrazole, third of three isomers isolated by preparative HPLC.
LCMS (UPLC_pH2_MeCN_QC_V2): Rt = 3.45 min, m/z 874.5 [M+H]⁺
¹H-NMR (400 MHz, DMSO-d₆): δ 8.05 (d, *J* = 1.2 Hz, 2H), 8.01 (s, 2H), 7.83 (dd, J = 8.4, 1.6 Hz, 2H), 7.56 (d, J = 8.4 Hz, 2H), 7.40 (s, 2H), 6.50 (s, 2H), 6.46 (s, 2H), 5.16 (s, 1H), 4.70 (s, 2H), 4.47 (q, J = 7.2 Hz, 4H), 4.18-4.33 (m, 4H), 3.67-3.73 (m, 1H), 3.54 (s, 6H), 1.95-2.08 (m, 13H), 1.24 (t, J = 7.0 Hz, 6H)

### Example 25

### 1-(3-{2-[(S)-2-Phenyl-1-pyrrolidinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino}-3-methyl-2,3-dihydro-1H-1,3-benzimidazole-5-carboxamide

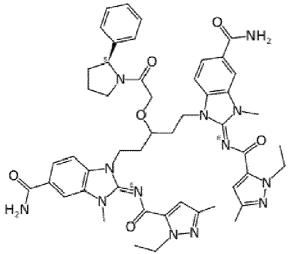

Prepared similarly to Example 5 from Building Block G-4 and the corresponding commercially available amine ((S)-2-phenylpyrrolidine).
LCMS (UPLC_pH2_MeCN_QC_V2): Rt = 3.89 min, m/z 924.6 [M+H]⁺

### Example 26

### 1-[3-(2-{(S)-2-[(6-Methoxy-1,2,3,4-tetrahydro-2-isoquinolyl)carbonyl]-1-pyrrolidinyl}-2-oxoethoxy)-5-{5-carbamoyl-2-[(1-ethyl-3-methyl-5-pyrazolyl)carbonylimino]-3-methyl-2,3-dihydro-1H-1,3-benzimidazol-1-yl}pentyl]-2-[(1-ethyl-3-methyl-5-pyrazolyl)carbonylimino]-3-methyl-2,3-dihydro-1H-1,3-benzimidazole-5-carboxamide

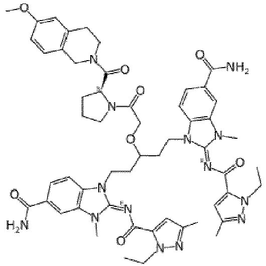

### Synthesis:

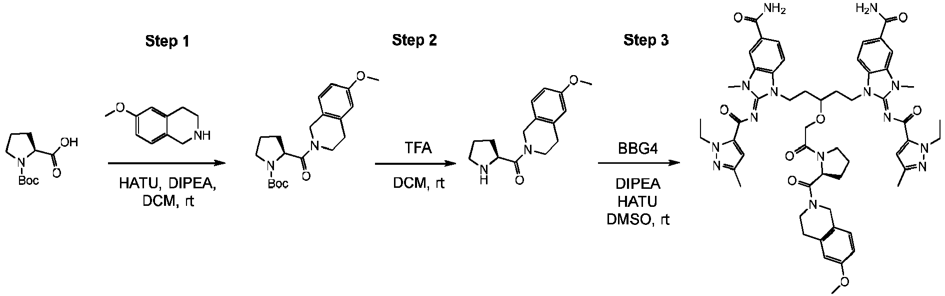

**Step 1:** Boc-(L)-proline (100 mg, 0.460 mmol) and 6-methoxy-1,2,3,4-tetrahydroisoquinoline (75 mg, 0.460 mmol) were dissolved in dry DCM (3.7 mL). DIPEA (0.24 mL, 1.38 mmol) and HATU (210 mg, 0.552 mmol) were added. The reaction mixture was stirred at rt overnight. The reaction was quenched with saturated aq NaHCOs solution and extracted with DCM three times. The combined DCM extract was passed through a hydrophobic frit and concentrated under reduced pressure to give tert-butyl (S)-2-[(6-methoxy-1,2,3,4-tetrahydro-2-isoquinolyl)carbonyl]-1-pyrrolidinecarboxylate as a yellow oil. Assumed quantitative yield (166 mg).
LCMS (UPLC_pH9_MeCN_2min): Rt = 1.76 min, m/z 361.2 [M+H]⁺

The crude material was used for the next step without purification.

**Step 2:** The Boc-protected amine obtained from step 1 (166 mg, 0.46 mmol) was dissolved in DCM (5.5 mL) and TFA (2.2 mL) was added. The reaction mixture was stirred at rt for 30 min and then concentrated under reduced pressure. The resulting oil was dissolved in a minimum amount of MeOH, loaded onto a SCX cartridge (1 g) and eluted with MeOH (3 CV) followed by 3.5 M NH₃ in MeOH (3 CV). The product was concentrated under reduced pressure, affording [(S)-2-pyrrolidinyl]-(6-methoxy-1,2,3,4-tetrahydro-2-isoquinolyl)methanone (86 mg, 72 %) as a light yellow gum.
LCMS (UPLC_pH9_MeCN_2min): Rt = 1.39 min, m/z 261.1 [M+H]⁺

**Step 3:** (3-{5-Carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetic acid (Building Block G-4; 30 mg, 0.0377 mmol) was dissolved in dry dimethyl sulfoxide (0.30 mL). [(S)-2-Pyrrolidinyl]-(6-methoxy-1,2,3,4-tetrahydro-2-isoquinolyl)methanone (8.8 mg, 0.0340 mmol) was added followed by DIPEA (0.020 mL, 0.113 mmol) and HATU (15 mg, 0.0377 mmol). The mixture was stirred at rt for 3 h. The solution was diluted with DMSO (1 mL) and purified by preparative HPLC (column: SunFire C18 250 mm x 19 mm, 5 µm; method: 30-70% MeCN in water containing 0.1% formic acid at pH 2). Fractions containing the desired product were combined and concentrated under reduced pressure. The resulting material was redissolved in MeCN and water and lyophilised overnight, affording 1-[3-(2-{(S)-2-[(6-methoxy-1,2,3,4-tetrahydro-2-isoquinolyl)carbonyl]-1-pyrrolidinyl}-2-oxoethoxy)-5-{5-carbamoyl-2-[(1-ethyl-3-methyl-5-pyrazolyl)carbonylimino]-3-methyl-2,3-dihydro-1H-1,3-benzimidazol-1 -yl}pentyl]-2-[(1 -ethyl-3-methyl-5-pyrazolyl)carbonylimino]-3-methyl-2,3-dihydro-1H-1,3-benzimidazole-5-carboxamide (24 mg, 68%) as a white solid.
LCMS (UPLC_pH2_MeCN_QC_V2): Rt = 3.83 min, m/z 1037.8 [M+H]⁺

### Example 27

### 1-(3-{2-[(R)-2-(2,5-Difluorophenyl)-1-pyrrolidinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide

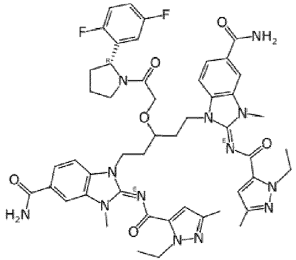

Prepared similarly to Example 5 from Building Block G-4 and the corresponding commercially available amine ((R)-2-(2,5-difluorophenyl)pyrrolidine).
LCMS (UPLC_pH2_MeCN_QC_V2): Rt = 3.98 min, m/z 960.6 [M+H]⁺

### Example 28

### 1-(3-{2-[(S)-2-(1H-Tetrazol-5-yl)-1-pyrrolidinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide

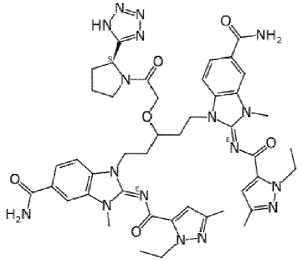

Prepared similarly to Example 5 from Building Block G-4 and the corresponding commercially available amine (5-[(S)-2-pyrrolidinyl]-1H-tetrazole).
LCMS (UPLC_pH2_MeCN_QC_V3): Rt = 3.31 min, m/z 916.6 [M+H]⁺

### Example 29

### 1-{3-[(N-Ethylcarbamoyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1, 3-benzimidazole-5-carboxamide

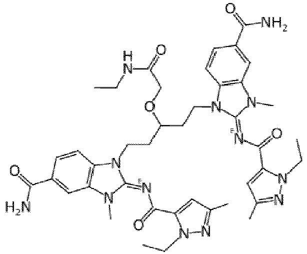

Prepared similarly to Example 5 from Building Block G-4 and the corresponding commercially available amine (ethanamine).
LCMS (UPLC_pH2_MeCN_QC_V2): Rt = 3.39 min, m/z 822.5 [M+H]⁺

### Example 30

### 1-(3-{2-[(S)-3-Phenyl-4-morpholinyl]-2-oxoethoxy}-5-(5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide

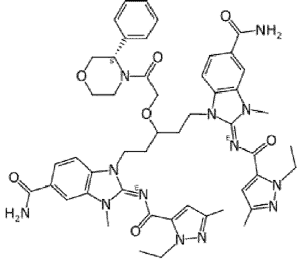

Prepared similarly to Example 5 from Building Block G-4 and the corresponding commercially available amine ((S)-3-phenylmorpholine).
LCMS (UPLC_pH9_MeCN_QC_V2): Rt = 3.77 min, m/z 940.5 [M+H]⁺

### Example 31

### Ethyl (3-{5-carbamoyl-3-ethyl-2-[(1-ethyl-3-methyl-5-pyrazolyl)carbonylimino]-2,3-dihydro-1H-1,3-benzimidazol-1-yl}-1-(2-{5-carbamoyl-3-ethyl-2-[(1-ethyl-3-methyl-5-pyrazolyl)carbonylimino]-2,3-dihydro-1H-1, 3-benzimidazol-1-yl)ethyl)propoxy)acetate

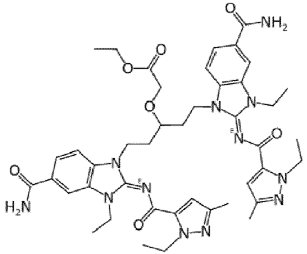

Prepared similarly to Example 2 from Building Block G-1 and iodoethane.
LCMS (UPLC_pH9_MeCN_QC_V2): Rt = 3.82 min, m/z 851.5 [M+H]⁺

### Example 32

### Isopropyl (3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl)ethyl)propoxy)acetate

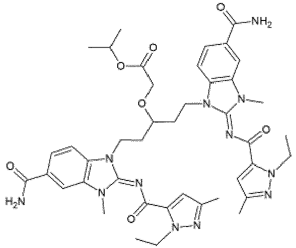

The compound is prepared from Building Block G4 by reaction with thionylchloride followed by removal of thionylchloride under reduced pressure and treatment with isopropanol.

### Example 33

### 1-(3-{2-[(S)-2-(Methoxymethyl)-1-pyrrolidinyl]-2-oxoethoxy)-5-(5-carbamoyl-2-[(1-ethyl-3-methyl-5-pyrazolyl)carbonylimino]-3-methyl-2,3-dihydro-1H-1,3-benzimidazol-1-yl)pentyl)-2-[(1-ethyl-3-methyl-5-pyrazolyl)carbonylimino]-3-methyl-2,3-dihydro-1H-1,3-benzimidazole-5-carboxamide

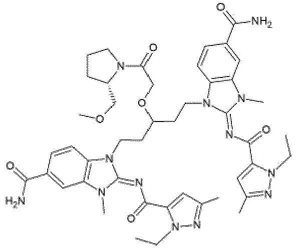

The compound is prepared similarly to Example 5 from Building Block G-4 and the corresponding commercially available amine ((S)-2-(methoxymethyl)pyrrolidine).

### Example 34

### 1-(5-{5-Carbamoyl-2-[(1-ethyl-3-methyl-5-pyrazolyl)carbonylimino]-3-methyl-2,3-dihydro-1H-1,3-benzimidazol-1-yl}-3-[2-oxo-2-(1-pyrrolidinyl)ethoxy]pentyl)-2-[(1-ethyl-3-methyl-5-pyrazolyl)carbonylimino]-3-methyl-2,3-dihydro-1H-1,3-benzimidazole-5-carboxamide

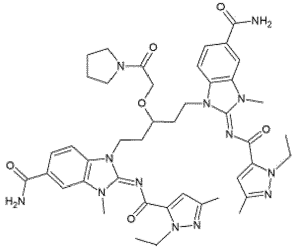

The compound is prepared similarly to Example 5 from Building Block G-4 and the corresponding commercially available amine (pyrrolidine).

### Example 35

### N-3,6,9-Trioxadecyl(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)acetamide

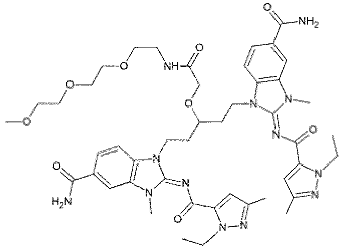

The compound is prepared similarly to Example 5 from Building Block G-4 and the corresponding commercially available amine (3,6,9-trioxa-1-decanamine).

### Example 36

### 1-{3-[(N,N-Diethylcarbamoyl)methoxy]-5-{5-carbamoyl-2-[(1-ethyl-3-methyl-5-pyrazolyl)carbonylimino]-2,3-dihydro-1H-1,3-benzimidazol-1-yl}pentyl}-2-[(1-ethyl-3-methyl-5-pyrazolyl)carbonylimino]-2,3-dihydro-1H-1,3-benzimidazole-5-carboxamide

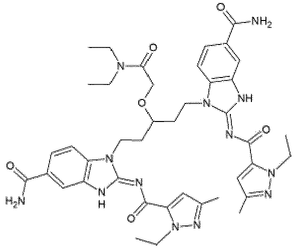

The compound is prepared similarly to Example 5 from Building Block G-3 and the corresponding commercially available amine (N-ethylethanamine).

### Biological assays and data

As stated above, the compounds of the present invention are inhibitors of STING. Biological activities of the compounds of the present invention can be determined using any suitable *in vitro* assay as well as tissue and *in vivo* models.

### STING binding FP assay I

The interaction between molecules and the C-terminal domain (CTD) of human STING was evaluated using a competition binding assay. In this assay, recombinant human STING protein (155-379) and a STING binding fluorescent probe were employed. Upon binding of the probe to STING, the rotation of the fluorescent probe is slowed due to the larger molecular size of the protein-bound complex. As a result, emitted light retains its plane of polarization, leading to an increase in fluorescence polarization. Molecules competing for the probe binding site displace the probe, which leads to rapid randomization of the polarization plane of the emitted light and a subsequent decrease in measured fluorescence polarization signal. Change in polarization was measured, allowing a comparative evaluation of the binding affinities of different compounds to the target protein.

The assay was performed in 96-well plates (ThermoFisher, cat.# 237108) containing 100 µl : 10 µL test compound in 10% DMSO and buffer A (150 mM NaCl, 50 mM HEPES pH 7.5, 1 mM DTT) + 90 µL prepared master mix solution of: 4 nM fluorescein-labelled STING ligand (Probe 1; synthesis described above) in buffer A + 0.055% Tween-20 (v/v) and 35 nM STING in buffer A. Post mixing, plates were agitated at 600 rpm and incubated for 20 min at room temperature. Subsequent measurements of the fluorescence polarization signal were made on a BioTek Synergy-H1 plate reader, using a polarization filter; excitation: 485/20 nm, emission: 528/20 nm) with a gain-setting of 75. IC50 values were determined using a standard non-linear regression four-parameter curve fit in Graphpad Prism.

Using the STING binding FP assay I described above, the following IC50 values of Example compounds were determined:

| **Example number** | **STING binding FP assay I, IC50 (nM)** |
|---|---|
| 2 | <30 |
| 3 | <30 |
| 5 | <30 |
| 6 | <30 |
| 7 | <30 |
| 8 | <30 |
| 9 | <30 |

### STING binding FP assay II

The procedure for STING binding FP assay II is the same as described for STING binding FP assay I, except for a lower STING concentration. In STING binding FP assay II, a concentration of 7 nM STING in buffer A was used.

Using the STING binding FP assay II described above, the following IC50 values of Example compounds were determined:

| **Example number** | **STING binding FP assay II, IC50 (nM)** |
|---|---|
| 10 | 3.5 |
| 11 | 2.0 |
| 12 | 1.7 |
| 13 | 2.0 |
| 21 | 4.8 |
| 22 | 12.8 |
| 23 | 6.3 |
| 24 | 4.8 |
| 25 | 4.6 |
| 26 | 3.5 |
| 27 | 3.7 |
| 28 | 8.4 |
| 29 | 7.1 |
| 30 | 3.7 |

### STING binding TR-FRET assay

The affinity of molecules to the C-terminal domain (CTD) of human STING was determined using a commercial Human STING WT Binding Kit (CisBio, cat.# 64BDSTGPEG). In this time-resolved FRET assay, a recombinant human STING protein construct with a His-tag was employed. When bound to STING, a D2-labeled active site probe accepts the 620 nm emission from Anti-HIS-Tb-STING and an increase in fluorescence is measured at 665 nm. Molecules that compete for the probe binding site will result in a low 665 nm signal. The assay was run in CisBio low-volume white 96-well plates (catalog# 66PL96005) containing 5 µL compounds in Diluent Buffer (supplied with the CisBio kit). A solution of 1xSTING, 1x Anti-HIS-Tb and 1X D2-probe in TB detection buffer (kit supplied) was added to the plate. Plates were centrifuged for 2 min at 500 rpm, incubated for 60 min at room temperature in the dark, and then fluorescence emission at 665 nm following 330 nm xenon flash excitation on an BioTek synergy H1 multimode reader (Agilent) was measured. The plC50 values were determined using a standard four parameter curve fit in GraphPad Prism.

Using the STING binding TR-FRET assay described above, the following IC50 values of Example compounds were determined:

| **Example number** | **STING binding TR-FRET assay, IC50 (nM)** |
|---|---|
| 3 | 0.452 |
| 5 | 0.380 |
| 6 | 0.600 |
| 7 | 0.267 |
| 9 | 0.665 |
| 10 | 0.869 |
| 11 | 0.862 |
| 14 | 0.659 |
| 17 | 0.540 |
| 19 | 1.110 |
| 20 | 0.950 |

### Human FB STING antagonist assay

Human skin fibroblasts were pre-treated for 2h with a serial dilution of test compound followed by stimulation with mock or 300 nM GSK STING agonist (CAS number: 2138299-34-8). Supernatants were harvested after 20 h of STING agonist stimulation. Release of IFN-a/b was determined using HEK-Blue IFN-α/β bioassay (Invivogen). Cellular viability was assessed using Alamar Blue HS assay. IC50 values were estimated using a non-linear regression fitting, variable slope (4 parameters) in GraphPad Prism 9.3.1.

Using the Human STING FB STING antagonist assay described above, the following IC50 values of Example compounds were determined:

| **Example number** | **Human FB STING antagonist assay, IC50 (µM)** |
|---|---|
| 1 | 3.2 |
| 2 | 2.5 |
| 6 | 7.6 |
| 7 | 3.5 |
| 8 | 4.7 |
| 9 | 13.1 |
| 11 | 3.4 |
| 12 | 9.8 |
| 13 | 7.8 |
| 22 | 6.0 |
| 26 | 1.4 |
| 28 | 3.9 |
| 29 | 5.2 |
| 30 | 3.6 |

### References

A number of publications are cited above in order to more fully describe and disclose the invention and the state of the art to which the invention pertains. Full citations for these references are provided below. The entirety of each of these references is incorporated herein.

Decout et al., The cGAS-STING pathway as a therapeutic target in inflammatory diseases; Nat. Rev. Immunol., 2021, 21, 548-569

Konig et al. Familial chilblain lupus due to a gain-of-function mutation in STING; Ann. Rheum. Dis., 2017, 76, 468-472

Rodero et al., Type I interferon-mediated autoinflammation due to DNase II deficiency; Nat. Comms., 2017, 8, 2176

Siu et al., Discovery of a Novel cGAMP Competitive Ligand of the Inactive Form of STING; ACS Med. Chem. Lett. 2019, 10, 1, 92-97

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press J. M. Ramanjulu et al., Design of amidobenzimidazole STING receptor agonists with systemic activity, Nature (2018), 564(7736), 439-443.

## Claims

1. The compound of Formula (I-b): 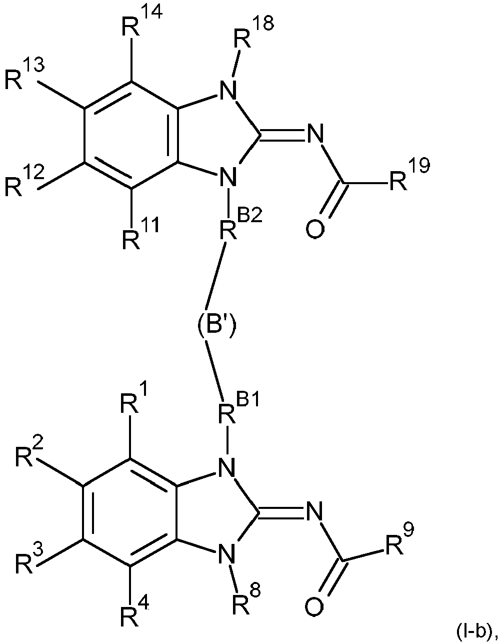 or a tautomer, prodrug or pharmaceutically acceptable salt thereof,
wherein R⁹ and R¹⁹ are each independently selected from an optionally substituted C₅₋₁₀ cycloalkyl, optionally substituted C₆₋₁₀ carboaryl, optionally substituted C₅₋₁₀ heteroaryl or optionally substituted C₅₋₁₀ heterocyclyl, wherein the optional substituents are independently selected from H, halogen, C₃₋₅ cycloalkyl, optionally substituted C₁₋₄ alkyl wherein said optionally substituted C₁₋₄ alkyl is optionally substituted by one or more substituents independently selected from halogen, halo(C₁₋₄ alkyl), OH, NH₂, C₁₋₄ alkoxy, CO₂H, CO₂(C₁₋₆ alkyl), N(C₁₋₆ alkyl)₂, OC(=O)NH₂ and C(=O)NH₂;
R¹ and R¹¹ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are independently selected from OH, -O-P(O)(OH)₂, C₁₋₄ alkoxy, NH₂, N(C₁₋₆ alkyl)₂, CO₂(C₁₋₆ alkyl) and halogen;
R⁶ and R¹³ are each independently selected from H, C(=O)NH₂; optionally substituted -C(=O)NH(C₁₋₆ alkyl), optionally substituted -C(=O)N(C₁₋₆ alkyl)₂, optionally substituted C₁₋₆ alkoxy or optionally substituted CO₂C₁₋₆ alkyl, wherein the optional substituents are independently selected from OH, -OP(O)(OH)₂, C₁₋₄ alkoxy, N(C₁₋₆ alkyl)₂, NH₂, CO₂(C₁₋₆ alkyl) and halogen;
R⁴ and R¹⁴ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are selected from OH, -O-P(O)(OH)₂, C₁₋₄alkoxy, N(C₁₋₆ alkyl)₂, NH₂, CO₂(C₁₋₆ alkyl) and halogen;
R² and R¹² are each independently selected from H, halogen, OH, optionally substituted C₁₋₆ alkyl, optionally substituted C₁₋₆ alkoxy, optionally substituted CO₂(C₁₋₆ alkyl), and optionally substituted C₁₋₆ alkylamino wherein the optional substituents are selected from OH, -O-P(O)(OH)₂, C₁₋₄ alkoxy, N(C₁₋₆ alkyl)₂, NH₂, CO₂(C₁₋₆ alkyl) and halogen;
R⁸ and R¹⁸ are each independently selected from H, and C₁₋₄ alkyl;
R^{B1} and R^{B2} are each independently selected from (-CH₂-)₁₋₂ or a bond and B' taken together with R^{B1} and R^{B2} forms a linking group, wherein B' is: 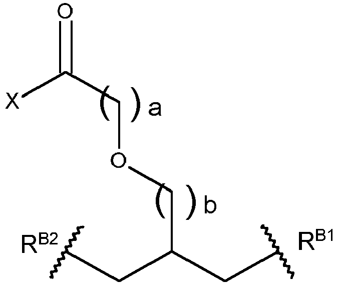
wherein a is 0, 1, or 2;
b is 0 or 1;
wherein X is -NR^{B3}R^{B4} or X is -OR^{B5},
wherein R^{B3}, R^{B4} and R^{B5} are each independently selected from:
i) hydrogen;
ii) optionally substituted C₁₋₈ alkyl;
iii) -(CH₂-(CH₂)₁₋₂₋O)₁₋₃-(CH₂)₁₋₃-H;
iv) optionally substituted C₂₋₈ alkenyl;
v) optionally substituted C₂₋₈ alkynyl;
vi) optionally substituted C₆₋₁₀ carboaryl;
vii) optionally substituted C₃₋₁₀ cycloalkyl;
viii) optionally substituted C₅₋₁₂ heteroaryl;
ix) optionally substituted C₄₋₁₂ heterocyclyl;
x) optionally substituted C₃₋₁₀ heteroalkyl;
xi) optionally substituted C₃₋₁₀ heteroalkenyl;
xii) optionally substituted C₃₋₁₀ heteroalkynyl;
or wherein R^{B3} and R^{B4} together with N form an optionally substituted C₄₋₁₂ heterocyclyl or C₅₋₁₂ heteroaryl;
wherein the optional substituents at R^{B3}, R^{B4} and R^{B5} are each R^{B6},
wherein each R^{B6} is independently selected from:
i) hydroxy;
ii) oxo;
iii) carboxy;
iv) halogen;
v) trifluoromethyl;
vi) trifluoromethoxy;
vii) -NH₂;
viii) optionally substituted C₆₋₁₀ aryl;
ix) optionally substituted C₃₋₆ cycloalkyl;
x) optionally substituted C₄₋₁₀ heterocyclyl;
xi) optionally substituted C₅₋₁₂ heteroaryl;
xii) optionally substituted C₁₋₄ alkyl;
xiii) optionally substituted C₂₋₄ alkenyl;
xiv) optionally substituted C₂₋₄ alkynyl;
xv) optionally substituted C₁₋₄ alkoxy;
xvi) optionally substituted C₃₋₄ alkenoxy;
xvii) optionally substituted C₃₋₄ alkynoxy;
xviii) optionally substituted -N(C₁₋₄ alkyl)₂,
xix) optionally substituted -NH(C₁₋₄ alkyl);
xx) optionally substituted C₃₋₆ heteroalkyl;
xxi) optionally substituted C₄₋₆ heteroalkenyl;
xxii) optionally substituted C₄₋₆ heteroalkynyl;
xxiii) optionally substituted -C(=O)-(C₆₋₁₀ aryl);
xxiv) optionally substituted -C(=O)-(C₃₋₆ cycloalkyl);
xxv) optionally substituted -C(=O)-(C₄₋₁₀ heterocyclyl);
xxvi) optionally substituted -C(=O)-(C₄₋₁₀ heteroaryl);
xxvii) optionally substituted -C(=O)-(C₁₋₄ alkyl);
xxviii) optionally substituted -C(=O)-(C₂₋₄ alkenyl);
xxix) optionally substituted -C(=O)-(C₂₋₄ alkynyl);
xxx) optionally substituted -C(=O)-(C₁₋₄ alkoxy);
xxxi) optionally substituted -C(=O)-(C₃₋₄ alkenoxy);
xxxii) optionally substituted -C(=O)-(C₃₋₄ alkynoxy);
xxxiii) optionally substituted -C(=0)-N(C₁₋₄ alkyl)₂,
xxxiv) optionally substituted -C(=O)-NH(C₁₋₄ alkyl);
xxxv) optionally substituted -C(=O)-(C₃₋₆ heteroalkyl);
xxxvi) optionally substituted -C(=O)-(C₄₋₆ heteroalkenyl);
xxxvii) optionally substituted -C(=O)-(C₄₋₆ heteroalkynyl);
xxxviii) optionally substituted -CH₂-(C₆₋₁₀ aryl);
xxxix) optionally substituted -CH₂-(C₄₋₁₀ heterocyclyl);
xi) optionally substituted -(C=O)NH-CH₂-(C₆₋₁₀ aryl); and
xii) optionally substituted -(C=O)NH-CH₂-(C₅₋₁₂ heteroaryl);
wherein the optional substituents at R^{B6} are each independently selected from hydroxy, oxo, carboxy, halogen, trifluoromethyl, trifluoromethoxy, -NH₂, -NH(C₁₋₂ alkyl), -N(C₁₋₂ alkyl)₂, methyl, ethyl, CH₂OCH₃, methoxy, and ethoxy.

2. The compound of claim 1, or a tautomer, or a pharmaceutically acceptable salt thereof, wherein R⁹ and R¹⁹ are each independently selected from an optionally substituted C₅₋₆ heteroaryl or optionally substituted C₅₋₆ heterocyclyl, wherein the optional substituents are independently selected from H, halogen, C₃₋₅ cycloalkyl, optionally substituted C₁₋₄ alkyl wherein said optionally substituted alkyl is optionally substituted by one or more substituents independently selected from halogen, halo(C₁₋₄ alkyl), OH, NH₂, C₁₋₄ alkoxy, CO₂H, CO₂C₁₋₆ alkyl, N(C₁₋₆ alkyl)₂, OCONH₂ and CONH₂.

3. The compound of claims 1 or 2, or a tautomer, or a pharmaceutically acceptable salt thereof, wherein the compound of Formula (I-b) is (I-b1): 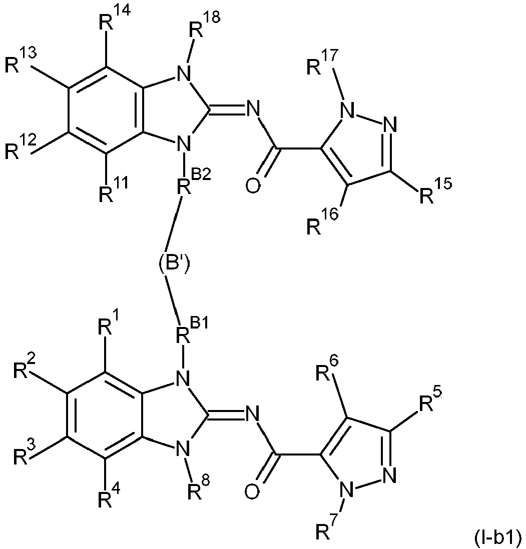
wherein R¹, R², R³, R⁴, R⁸, R¹¹, R¹², R¹³, R¹⁴, R¹⁸, R^{B1}, R^{B2} and B' are as defined in claim 1,
wherein R⁷ and R¹⁷ are each independently selected from optionally substituted C₁₋₄ alkyl wherein the optional substituents are independently selected from halogen, -halo(C₁₋₄ alkyl), OH, and C₁₋₄ alkoxy,;
R⁶ and R¹⁶ are each independently selected from H, halogen or optionally substituted C₁₋₄alkyl or C₁₋₄ alkoxy wherein the optional substituents are independently selected from halogen, halo(C₁₋₄ alkyl), OH, and C₁₋₄ alkoxy;
R⁵ and R¹⁵ are each independently selected from H, cyclopropyl and C₁₋₄ alkyl.

4. The compound of any of claims 1 to 3, or a tautomer, or a pharmaceutically acceptable salt thereof, wherein R¹⁴, R², R¹², R¹ and R¹¹ are each independently selected from H, OH, OCH₃, halogen or C₁₋₃ alkyl.

5. The compound of claims 3 or 4 or a pharmaceutically acceptable salt thereof, wherein R⁶ and R¹⁶ are each independently selected from H, OCH₃, halogen or C₁₋₃ alkyl.

6. The compound of any of claims 1 to 5, or a tautomer, or a pharmaceutically acceptable salt thereof, wherein R¹ and R¹¹ are H.

7. The compound of any of claims 1 to 6, or a tautomer, or a pharmaceutically acceptable salt thereof, wherein R⁶ and R¹⁶ are H.

8. The compound of any one of claims 1 to 7, or a tautomer, or a pharmaceutically acceptable salt thereof, wherein R⁴ and R¹⁴ are H.

9. The compound of any one of claims 1 to 8, or a tautomer, or a pharmaceutically acceptable salt thereof, wherein R² and R¹² are H.

10. The compound of any one of claims 3 to 9, or a tautomer, or a pharmaceutically acceptable salt thereof, wherein each of R⁵ and R¹⁵, are C₁₋₄ alkyl or C₁₋₄ alkoxy.

11. The compound of any of claims 1 to 10, or a tautomer, or a pharmaceutically acceptable salt thereof, wherein R⁷, R⁸, R¹⁷ and R¹⁸ are C₁₋₄ alkyl.

12. The compound of any of claims 1 to 11, or a tautomer, or a pharmaceutically acceptable salt thereof, wherein R⁷ and R¹⁷ are ethyl.

13. The compound of any one of claims 3 to 12, or a tautomer, or a pharmaceutically acceptable salt thereof, wherein R⁵ and R¹⁵ are methyl.

14. The compound of any one of claims 1 to 13, or a tautomer, or a pharmaceutically acceptable salt thereof, wherein R⁸ and R¹⁸ are methyl.

15. The compound of any one of claims 1 to 14, or a tautomer, or a pharmaceutically acceptable salt thereof, wherein R⁶ and R¹³ are selected from -C(=O)NH(C₁₋₃ alkyl) and -C(=O)NH₂.

16. The compound of any one of claims 1 to 15, or a tautomer, or a pharmaceutically acceptable salt thereof, wherein R³ and R¹³ are -C(=O)NH₂.

17. The compound of any one of claims 1 to 16, or a tautomer, or a pharmaceutically acceptable salt thereof, wherein:
R¹ and R¹¹ are selected from H, -OH or C₁₋₃ alkoxy;
R³ and R¹³ are selected from -C(=O)N(H)(C₁₋₃alkyl) or -C(=O)NH₂;
R⁴ and R¹⁴ are selected from H, halogen, OH or C₁₋₄ alkyl;
R⁷ and R¹⁷ are C₁₋₄ alkyl;
R⁵ and R¹⁵ are selected from H or C₁₋₄ alkyl;
R⁶ and R¹⁶ are H; and
R⁸ and R¹⁸ are methyl.

18. The compound of claim 17, or a tautomer, or a pharmaceutically acceptable salt thereof, wherein:
R¹ and R¹¹ are H;
R³ and R¹³ are -C(=O)NH₂ or -C(=O)-NH-CH₃;
R⁷ and R¹⁷ are ethyl; and
R⁵ and R¹⁵ are methyl.

19. The compound of any one of claims 1 to 18, or a tautomer, or a pharmaceutically acceptable salt thereof, wherein R¹ is identical to R¹¹, R² is identical to R¹², R⁶ is identical to R¹³, R⁴ is identical to R¹⁴, R^{B1} is identical to R^{B2}, R⁸ is identical to R¹⁸ and R⁹ is identical to R¹⁹.

20. The compound any one of claims 3 to 18, or a tautomer, or a pharmaceutically acceptable salt thereof, wherein R¹ is identical to R¹¹, R² is identical to R¹², R³ is identical to R¹³, R⁴ is identical to R¹⁴, R⁵ is identical to R¹⁵, R^{B1} is identical to R^{B2}, R⁸ is identical to R¹⁸, R⁵ is identical to R¹⁵, R⁶ is identical to R¹⁶ and R⁷ is identical to R¹⁷.

21. The compound of any one of claims 1 to 20, or a tautomer, or a pharmaceutically acceptable salt thereof, wherein compound (I-b) is of the Formula (I-2b): 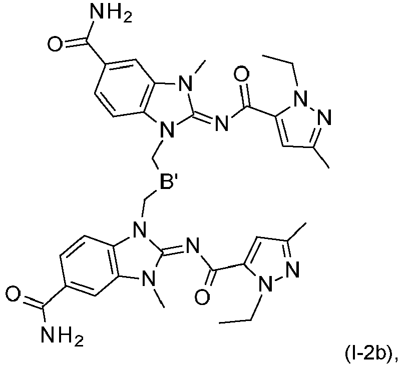 or a tautomer, or a pharmaceutically acceptable salty thereof wherein B' is as defined in any of the preceding claims.

22. The compound of any one of claims 1 to 21, or a tautomer, or a pharmaceutically acceptable salt thereof, wherein B' has the formula: 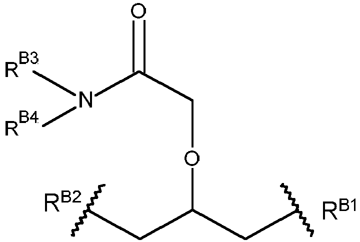
wherein R^{B3} and R^{B4} are each independently selected from:
i) hydrogen;
ii) optionally substituted C₁₋₈ alkyl;
iii) -(CH₂CH₂O)₁₋₅-(CH₂)₁₋₃-H;
iv) optionally substituted C₆₋₁₀ carboaryl;
v) optionally substituted C₃₋₁₀ cycloalkyl;
vi) optionally substituted C₅₋₁₂ heteroaryl;
vii) optionally substituted C₄₋₁₂ heterocyclyl;
viii) optionally substituted C₃₋₁₀ heteroalkyl;
or wherein R^{B3} and R^{B4} together with N form an optionally substituted C₄₋₁₂ heterocyclyl or optionally substituted C₅₋₁₂ heteroaryl;
wherein the optional substituents are R^{B6} as defined in claim 1.

23. The compound of claim 22, or a tautomer, or a pharmaceutically acceptable salt thereof, wherein R^{B3} and R^{B4} are independently selected from:
i) hydrogen;
ii) optionally substituted C₁₋₆ alkyl wherein the optional substituent is selected from methoxy, - (C=O)NH-CH₂-phenyl, C(=O)NH-phenyl, phenyl optionally substituted by methoxy, or a C₅₋₆ heteroaryl optionally substituted by methoxy or methyl;
iii) -(CH₂CH₂O)₁₋₅-(CH₂)₁₋₃-H;
iv) optionally substituted C₆ carboaryl wherein the optional substituent is C₁₋₃ alkyl;
v) optionally substituted C₅₋₁₂ heteroaryl wherein the optional substituent is C₁₋₃ alkyl;
vi) optionally substituted C₄₋₁₂ heterocyclyl wherein the optional substituent is C₁₋₃ alkyl;
or wherein R^{B3} and R^{B4} together with N form an optionally substituted C₄₋₁₂ heterocyclyl or optionally substituted C₅₋₁₂ heteroaryl wherein the optional substituents are selected from C(=O)C₁₋₆ alkyl, C₁₋₆ alkyl, NH₂, C₁₋₆ alkoxy, C₄₋₅ heteroaryl, C₄₋₅ heterocyclyl, or optionally substituted phenyl wherein the optional substituents are F and OMe.

24. The compound of claim 22 or 23, or a tautomer, or a pharmaceutically acceptable salt thereof, wherein R^{B3} and R^{B4} are independently selected from:
i) H;
ii) isopropyl;
iii) optionally substituted ethyl;
iv) optionally substituted methyl
v) CH₂CH₂OCH₂CH₂OCH₂CH₂OCH₃;
vi) optionally substituted C₆carboaryl;
vii) optionally substituted C₅₋₆ heteroaryl;
viii) optionally substituted C₅₋₆ heterocyclyl;
wherein the optional substituents are R^{B6} as defined in claim 1,
or wherein R^{B3} and R^{B4} together with N form an optionally substituted C₄₋₁₂ heterocyclyl or optionally substituted C₅₋₁₂ heteroaryl wherein the optional substituents are R^{B6} as defined in claim 1.

25. The compound of any one of claims 22, or a tautomer, or a pharmaceutically acceptable salt thereof, wherein one of R^{B6} and R^{B4} is H and the other is independently selected from:
i) isopropyl;
ii) optionally substituted ethyl;
iii) phenyl;
iv) optionally substituted C₂₋₇ heteroalkyl;
v) optionally substituted C₅₋₆ heteroaryl;
vi) optionally substituted C₅₋₆ heterocyclyl;
vii) -CH₂CH₂OCH₂CH₂OCH₂CH₂OCH₃.
wherein the optional substituents are R^{B6} as defined in claim 1.

26. The compound of claim 25, or a pharmaceutically acceptable salt thereof, wherein one of R^{B3} and R^{B4} is H and the other is independently selected from optionally substituted phenyl, optionally substituted pyrimidine, optionally substituted piperidine and optionally substituted pyrazole, wherein the optional substituents are C₁₋₃ alkyl or NH₂.

27. The compound of any of the preceding claims, or a tautomer or pharmaceutically acceptable salt thereof, wherein the number of substituents R^{B6} on R^{B3} or R^{B4} is 0, 1 or 2.

28. The compound any of the preceding claims, or a tautomer or pharmaceutically acceptable salt thereof, wherein the number of substituents on R^{B6} is 0, 1 or 2.

29. The compound of any one of claims 1 to 21, or a tautomer or pharmaceutically acceptable salt thereof, wherein the number of substituents R^{B6} on R^{B5} is 0 or 1.

30. The compound of any one of claims 1 to 24, or a tautomer, or a pharmaceutically acceptable salt thereof, wherein R^{B6} and R^{B4} together with N form an optionally substituted C₄₋₁₂heterocyclyl or optionally substituted C₅₋₁₂ heteroaryl, wherein the optional substituents are R^{B6} as defined in claim 1.

31. The compound of claim 30, or a tautomer, or a pharmaceutically acceptable salt thereof, wherein R^{B3} and R^{B4} together form an optionally substituted piperazine, an optionally substituted pyrazole, an optionally substituted morpholine, an optionally substituted pyrrolidine, an optionally substituted piperidine, an optionally substituted oxazine, an optionally substituted pyrimidine, an optionally substituted 1,2,3,4,-tetrahydroquinolone, an optionally substituted 6,7-dihydro-4H-thiazolo[5,4-c]pyridine-5-yl, or an optionally substituted 1,2,3,4-tetrahydroisoquinoline, wherein the optional substituents are R^{B6} as defined in claim 1.

32. The compound of claim 30 or 31, or a tautomer, or a pharmaceutically acceptable salt thereof wherein, R^{B3} and R^{B4} together with N form an optionally substituted piperazine, an optionally substituted pyrazole, an optionally substituted morpholine, an optionally substituted pyrrolidine or an optionally substituted piperidine, wherein the optional substituents are R^{B6} as defined in claim 1.

33. The compound of any of the preceding claims, or a tautomer, or a pharmaceutically acceptable salt thereof, wherein each R^{B6} is independently selected from:
i) hydroxy;
ii) oxo;
iii) carboxy;
iv) halogen;
v) trifluoromethyl;
vi) trifluoromethoxy;
vii) -NH₂;
viii) optionally substituted C₆₋₁₀ aryl;
ix) optionally substituted C₃₋₆ cycloalkyl;
x) optionally substituted C₄₋₁₀ heterocyclyl;
xi) optionally substituted C₅₋₁₂ heteroaryl;
xii) optionally substituted C₁₋₄ alkyl;
xiii) optionally substituted C₂₋₄ alkenyl;
xiv) optionally substituted C₂₋₄ alkynyl;
xv) optionally substituted C₁₋₄ alkoxy;
xvi) optionally substituted -C(=O)-(C₆₋₁₀ aryl);
xvii) optionally substituted -C(=O)-(C₃₋₆ cycloalkyl);
xviii) optionally substituted -C(=O)-(C₄₋₁₀ heterocyclyl);
xix) optionally substituted -C(=O)-(C₄₋₁₀ heteroaryl);
xx) optionally substituted -C(=O)-(C₁₋₄ alkyl);
xxi) optionally substituted -CH₂-(C₆₋₁₀ aryl);
xxii) optionally substituted -CH₂-(C₄₋₁₀ heterocyclyl);
xxiii) optionally substituted -(C=O)NH-CH₂-(C₆₋₁₀ aryl); and
xxiv) optionally substituted -(C=O)NH-CH₂-(C₅₋₁₂ heteroaryl);
wherein the optional substituents at R^{B6} are each independently selected from hydroxy, oxo, carboxy, halogen, trifluoromethyl, trifluoromethoxy, -NH₂, -NH(C₁₋₂ alkyl), -N(C₁₋₂ alkyl)₂, methyl, ethyl, CH₂OCH₃, methoxy, and ethoxy.

34. The compound of any of the preceding claims, or a tautomer, or a pharmaceutically acceptable salt thereof, wherein each R^{B6} is independently selected from:
i) hydroxy;
ii) oxo;
iii) carboxy;
iv) halogen;
v) trifluoromethyl;
vi) trifluoromethoxy;
vii) -NH₂;
viii) optionally substituted C₆₋₁₀ aryl;
ix) optionally substituted C₃₋₆ cycloalkyl;
x) optionally substituted C₄₋₁₀ heterocyclyl;
xi) optionally substituted C₅₋₁₂ heteroaryl;
xii) optionally substituted C₁₋₄ alkyl;
xiii) optionally substituted C₁₋₄ alkoxy;
xiv) optionally substituted -C(=O)-(C₁₋₄ alkyl);
xv) optionally substituted -C(=O)-(C₄₋₁₀ heterocyclyl);
xvi) optionally substituted -CH₂-(C₆₋₁₀ aryl);
xvii) optionally substituted -CH₂-(C₄₋₁₀ heterocyclyl);
xviii) optionally substituted -(C=O)NH-CH₂-(C₆₋₁₀ aryl); and
xix) optionally substituted -(C=O)NH-CH₂-(C₅₋₁₂ heteroaryl);
wherein the optional substituents at R^{B6} are each independently selected from hydroxy, oxo, carboxy, halogen, trifluoromethyl, trifluoromethoxy, -NH₂, -NH(C₁₋₂ alkyl), -N(C₁₋₂ alkyl)₂, methyl, ethyl, CH₂OCH₃, methoxy, and ethoxy.

35. The compound of any of the preceding claims, or a tautomer, or a pharmaceutically acceptable salt thereof, wherein each R^{B6} is independently selected from:
i) -NH₂;
ii) optionally substituted C₆₋₁₀ aryl;
iii) optionally substituted C₅₋₁₂ heteroaryl;
iv) optionally substituted C₁₋₄ alkyl;
v) optionally substituted C₁₋₄ alkoxy;
vi) optionally substituted -C(=O)-(C₁₋₄ alkyl);
vii) optionally substituted -(C=O)NH-CH₂-(C₆₋₁₀ aryl);
viii) optionally substituted -C(=O)-(C₄₋₁₀ heterocyclyl); and
wherein the optional substituents at R^{B6} are each independently selected from hydroxy, oxo, carboxy, halogen, trifluoromethyl, trifluoromethoxy, -NH₂, -NH(C₁₋₂ alkyl), -N(C₁₋₂ alkyl)₂, methyl, ethyl, CH₂OCH₃, methoxy, and ethoxy.

36. The compound of any of the preceding claims, or a tautomer, or a pharmaceutically acceptable salt thereof, wherein each R^{B6} is independently selected from:
xi) -NH₂;
xii) C₁₋₃ alkyl, optionally substituted with -OMe;
xiii) C(=O)CH₃;
xiv) OCH₃,
xv) halogen;
xvi) C₅₋₆ heterocyclyl;
xvii) C₅₋₆ heteroaryl;
xviii) optionally substituted -C(=O)-(C₄₋₁₀ heterocyclyl);
xix) phenyl which phenyl is optionally substituted by 1-2 substituents selected from -OCH₃ or halogen;
xx) optionally substituted -(C=O)NH-CH₂-(C₆₋₁₀ aryl); or
xxi) 1,2,3,4-tetrahydro-2-isoquinolinecarbonyl substituted by methoxy.

37. The compound of any of the preceding claims, or a tautomer, or a pharmaceutically acceptable salt thereof, wherein R^{B6} is selected from: -NH₂; optionally substituted C₆₋₁₀ aryl; optionally substituted C₅₋₁₂ heteroaryl; optionally substituted C₁₋₄ alkyl; optionally substituted C₁₋₄alkoxy; optionally substituted - C(=O)-(C₁₋₄ alkyl); optionally substituted C(=O)C₁₀ heterocyclyl, optionally substituted -(C=O)NH-CH₂-(C₆₋₁₀ aryl); wherein the optional substituents at R^{B6} are each independently selected from hydroxy, oxo, carboxy, halogen, trifluoromethyl, trifluoromethoxy, -NH₂, methyl, ethyl, CH₂OCH₃, methoxy, and ethoxy.

38. The compound of claim 24, or a tautomer, or a pharmaceutically acceptable salt thereof, wherein when R^{B3} and R^{B4} are independently selected from
i) H;
ii) isopropyl;
iii) optionally substituted ethyl;
iv) optionally substituted methyl,
v) CH₂CH₂OCH₂CH₂OCH₂CH₂OCH₃;
vi) optionally substituted C₆ carboaryl;
vii) optionally substituted C₅₋₆ heteroaryl or
viii) optionally substituted C₅₋₆ heterocyclyl,
R^{B6} is selected from C₁₋₃ alkyl, methoxy, -(C=O)NH-CH₂-phenyl, C(=O)NH-phenyl, C₅₋₆ heteroaryl, or phenyl which phenyl is optionally substituted by methoxy or fluorine, or
wherein when R^{B6} and R^{B4} together with N form an optionally substituted C₄₋₁₂ heterocyclyl or optionally substituted C₅₋₁₂ heteroaryl, the optional substituents are R^{B6} and R^{B6} is selected from C₁₋₃ alkyl, C(=O)CH₃, CH₂OCH₃, OCH₃, NH₂, halogen, C₅₋₆ heteroaryl, optionally substituted -C(=O)-(C₁₀ heterocyclyl) wherein the optional substituent is methoxy; or phenyl wherein said phenyl is optionally substituted by methoxy or halogen.

39. The compound of any of claims 1 to 38, or a tautomer, or a pharmaceutically acceptable salt thereof, wherein R^{B1} and R^{B2} are both -CH₂- or both -CH₂-CH₂- or R^{B1} is -CH₂- and R^{B2} is -CH₂-CH₂-.

40. The compound of any of the preceding claims, or a tautomer, or a pharmaceutically acceptable salt thereof wherein R^{B1} is a bond and R^{B2} is -CH₂-.

41. The compound of any of claim 1 to 40 or a tautomer, or a pharmaceutically acceptable salt thereof, wherein R^{B1} and R^{B2} are both -CH₂-.

42. The compound of any one of claims 1 to 21, or a tautomer, or a pharmaceutically acceptable salt thereof, wherein B' is of the formula: 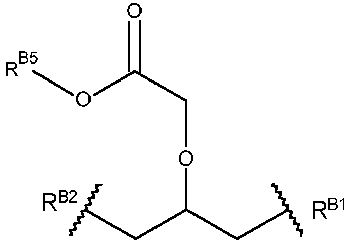 wherein R^{B5} is selected from H, C₁₋₆ alkyl, C₂₋₆ alkenyl or C₂₋₆ alkylnyl.

43. The compound of claim 42, or a tautomer, or a pharmaceutically acceptable salt thereof, wherein R^{B5} is H, isopropyl or ethyl.

44. The compound according to any of the preceding claims, or a tautomer, or a pharmaceutically acceptable salt thereof wherein B' is selected from one of the following groups:

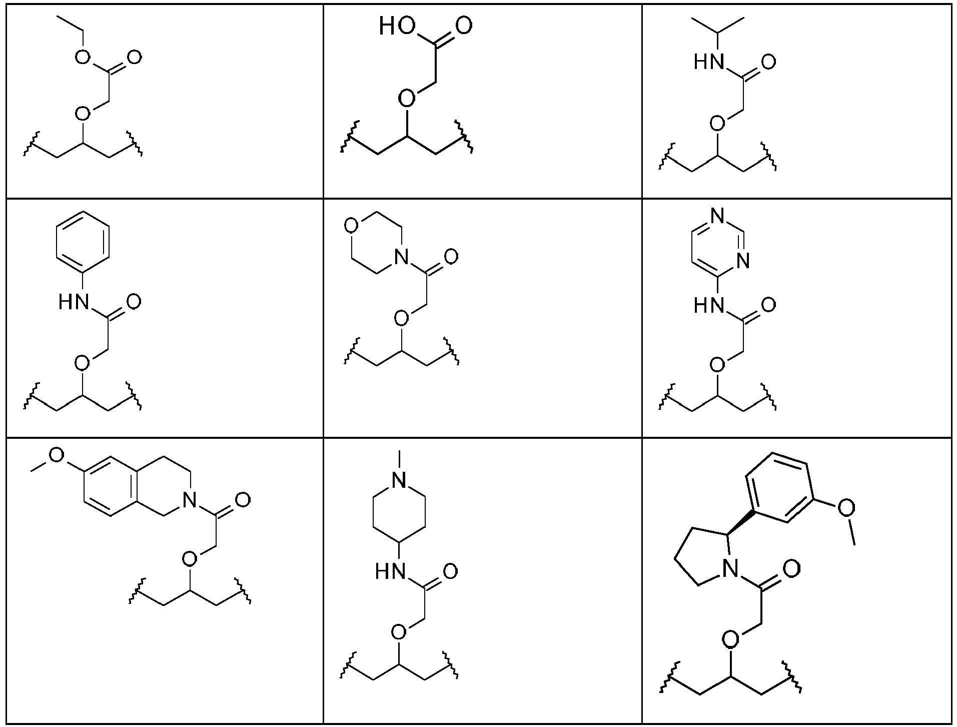

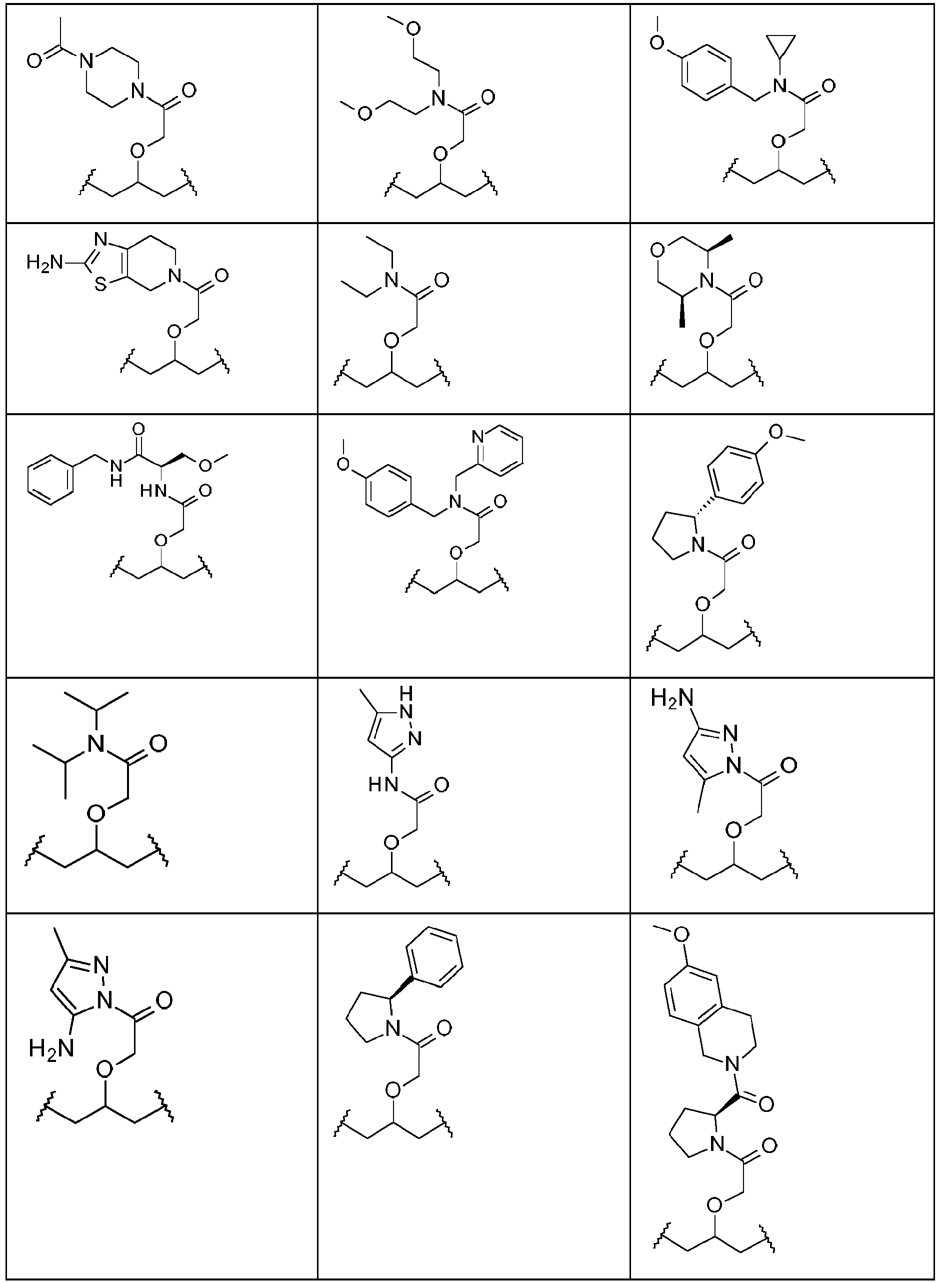

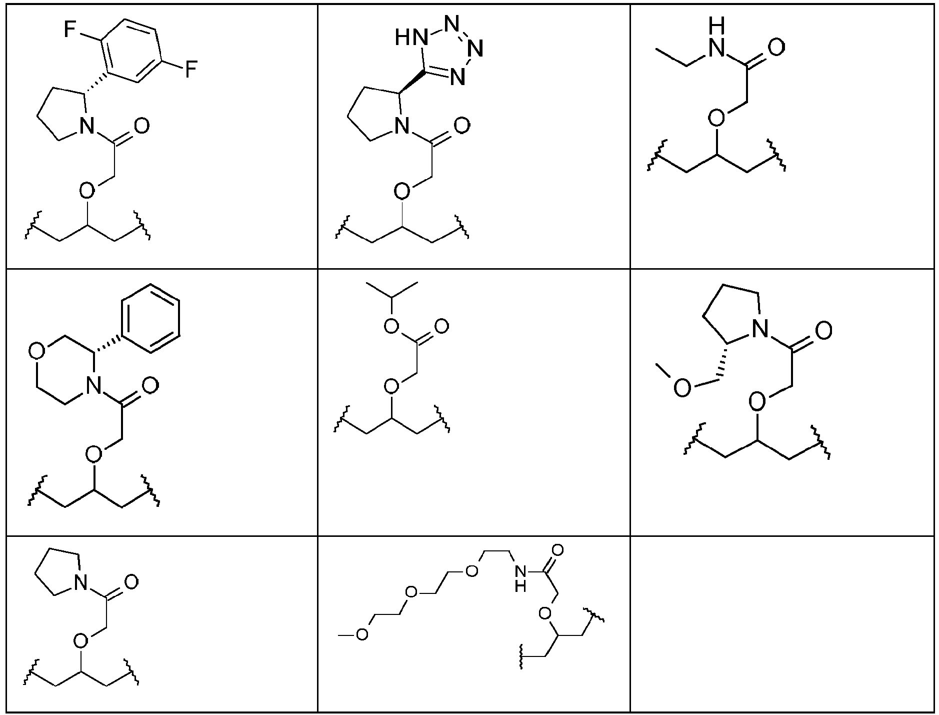

45. The compound of claim 44, or a tautomer, or pharmaceutically acceptable salt thereof wherein B' is selected from one of the following groups:

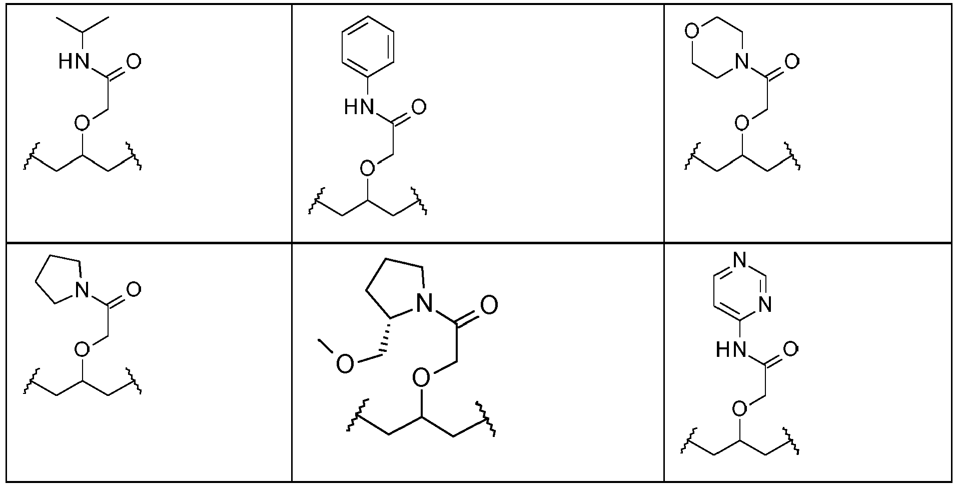

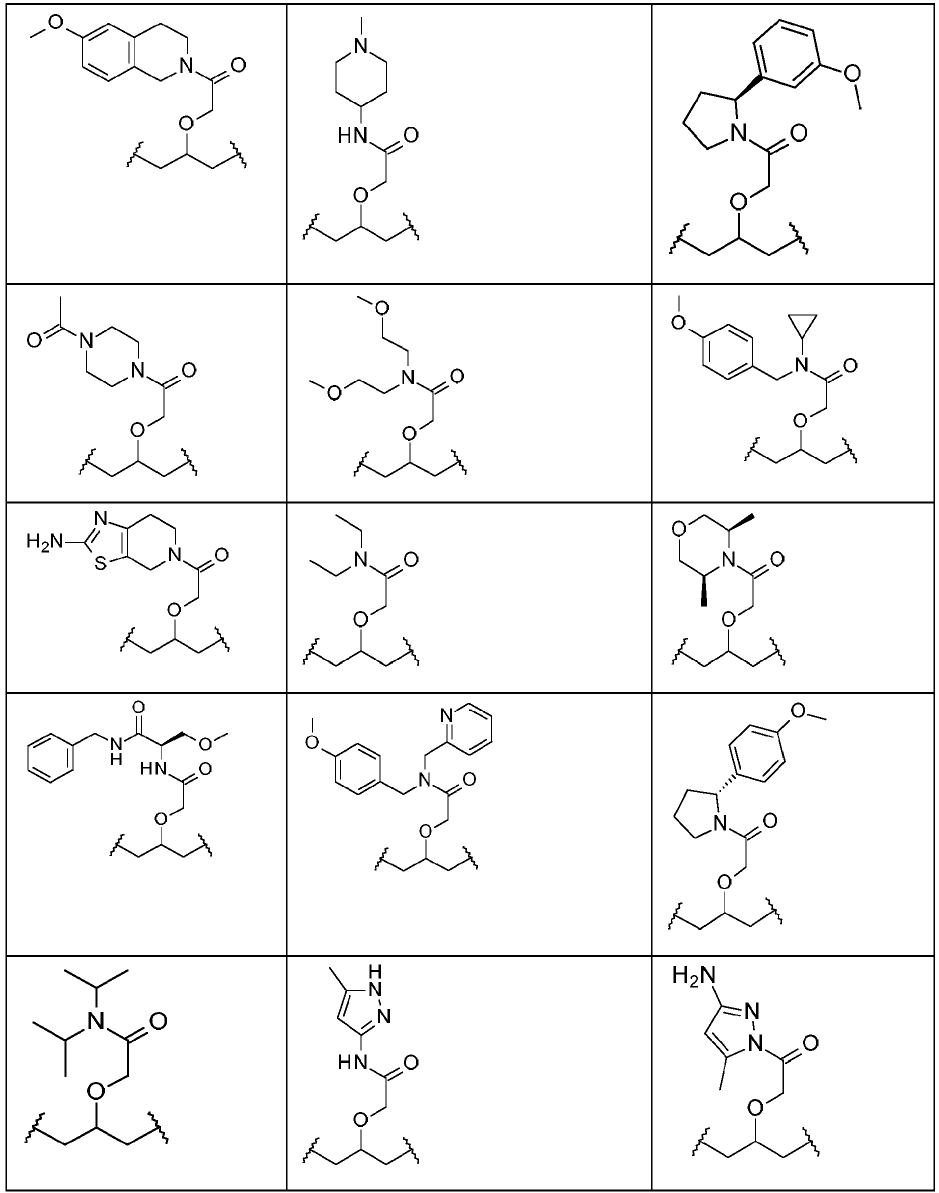

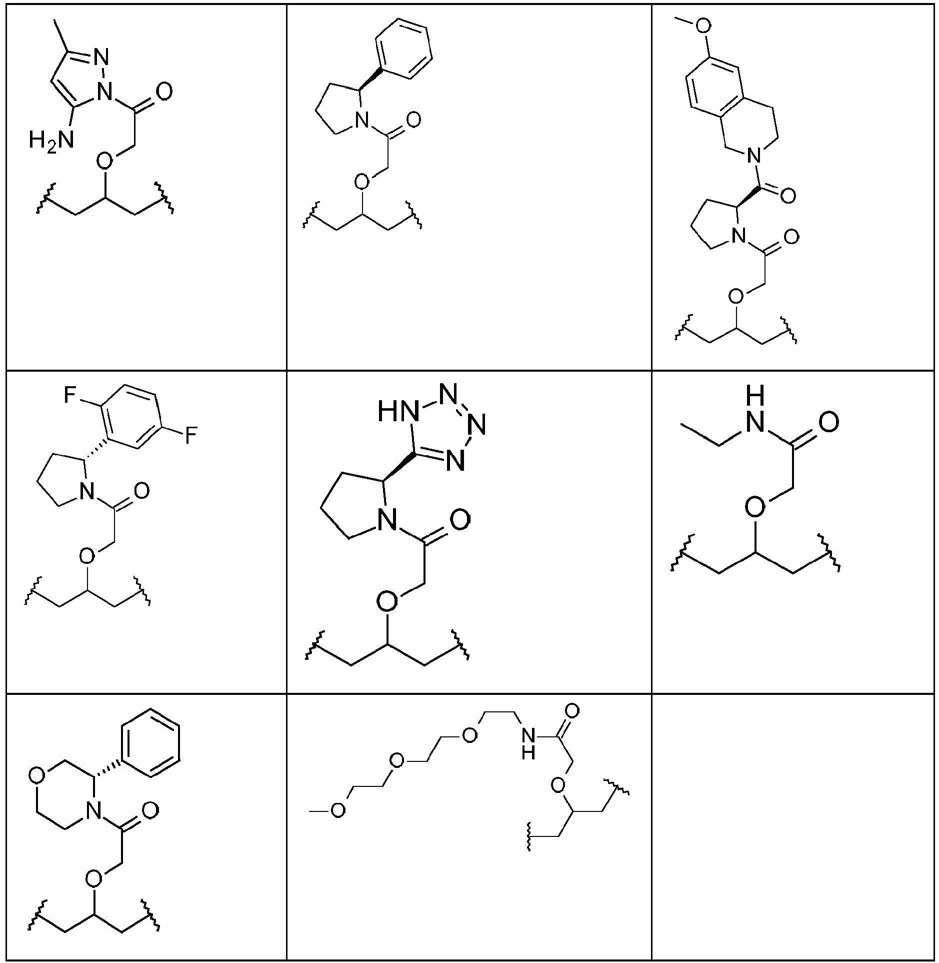

46. The compound according to any of the preceding claims, or a tautomer, or a pharmaceutically acceptable salt thereof wherein: R¹, R¹¹, R⁶, R¹⁶, R¹⁴, R² and R¹² are H
R⁷ and R¹⁷ are ethyl;
R⁵, R¹⁵, R⁸ and R¹⁸ are methyl;
R³ and R¹³ are -C(=O)NH₂;
R^{B1} and R^{B2} are both -CH₂-;
b is 0;
a is 1;
X is -NR^{B3}R^{B4} or ethoxy
R^{B3} and R^{B4} are selected from H, ethyl, phenyl, pyrimidine, -CH₂CH₂-O-CH₃, pyrazole substituted by methyl,
or R^{B3} and R^{B4} together form a morpholine which is optionally substituted by phenyl, a 1,2,3,4-tetrahydroquinoline substituted with methoxy, a pyrrolidine substituted with a tetrazole or phenyl which phenyl is substituted with methoxy or a 2-[(1 ,2,3,4-tetrahydro-2-isoquinolyl)carbonyl]pyrrolidine substituted with methoxy or a piperazine substituted with C(=O)CH₃.

47. The compound according to any of the preceding claims, or a tautomer, or a pharmaceutically acceptable salt thereof wherein the compound is selected from the following:
| **Example number** | **Structure** |
|---|---|
| 1 | 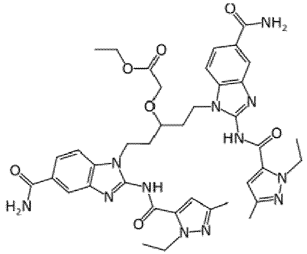 |
| 2 | 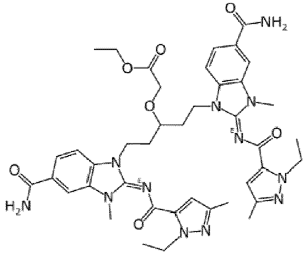 |
| 3 | 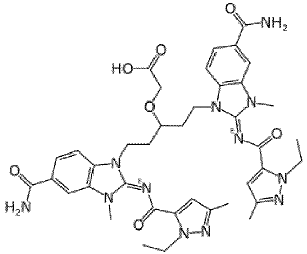 |
| 4 | 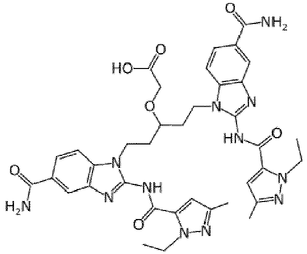 |
| 5 | 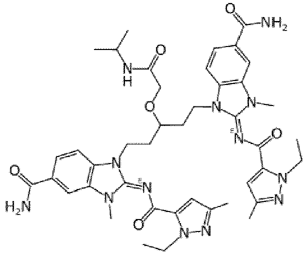 |
| 6 | 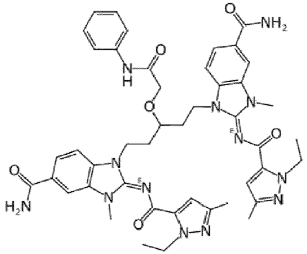 |
| 7 | 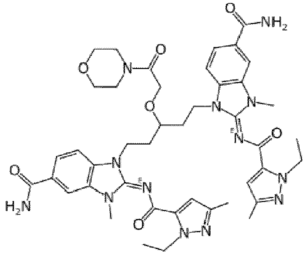 |
| 8 | 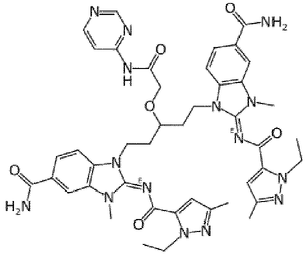 |
| 9 | 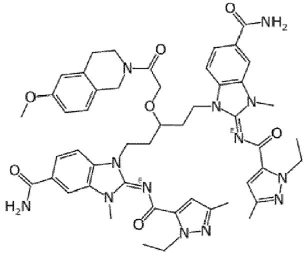 |
| 10 | 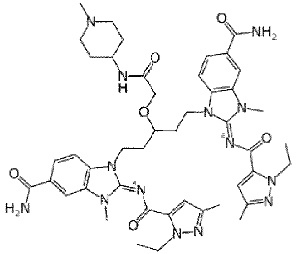 |
| 11 | 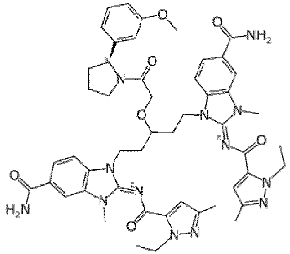 |
| 12 | 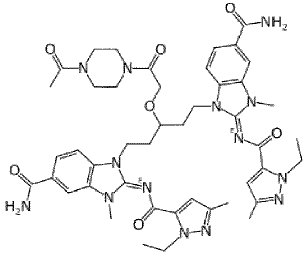 |
| 13 | 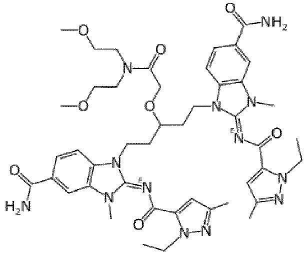 |
| 14 | 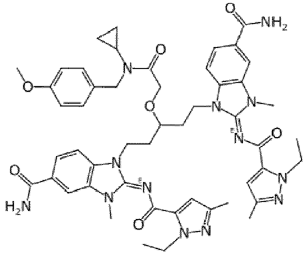 |
| 15 | 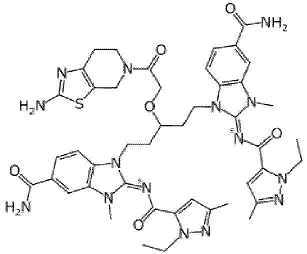 |
| 16 | 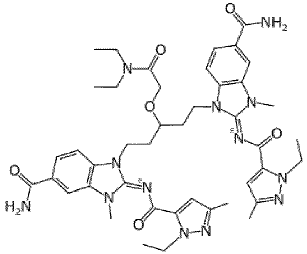 |
| 17 | 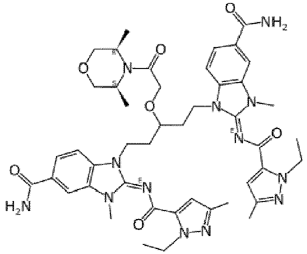 |
| 18 | 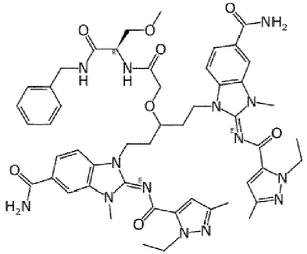 |
| 19 | 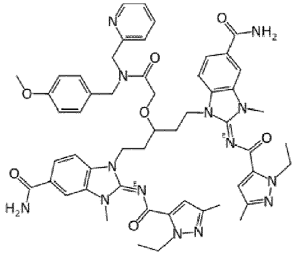 |
| 20 | 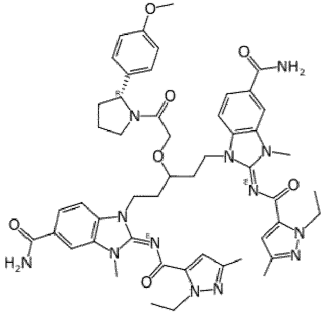 |
| 21 | 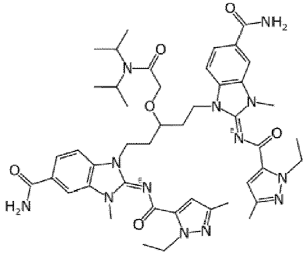 |
| 22 | 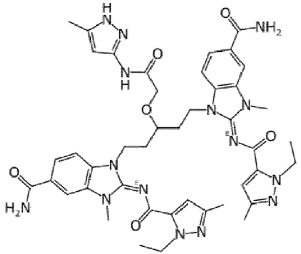 |
| 23 | 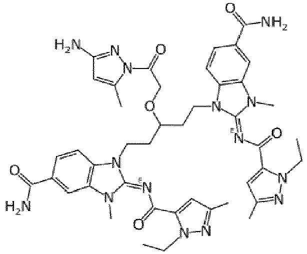 |
| 24 | 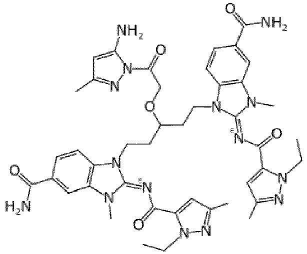 |
| 25 | 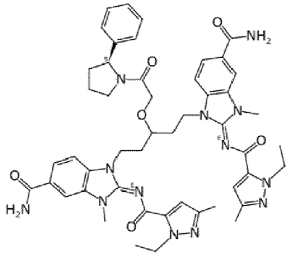 |
| 26 | 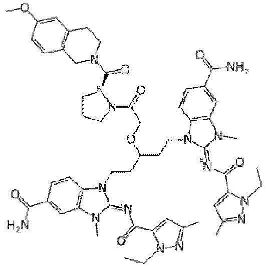 |
| 27 | 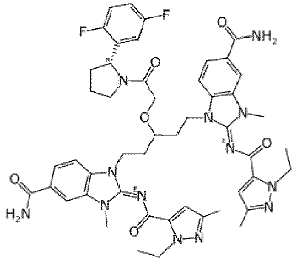 |
| 28 | 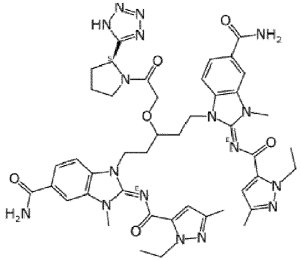 |
| 29 | 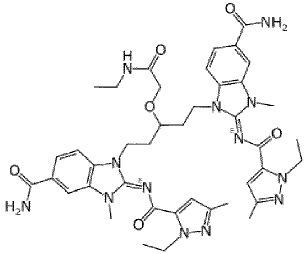 |
| 30 | 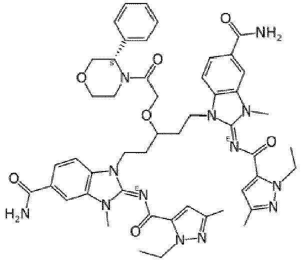 |
| 31 | 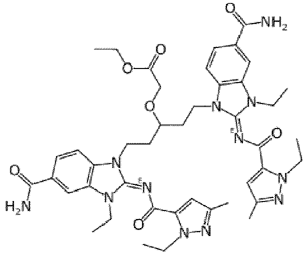 |
| 32 | 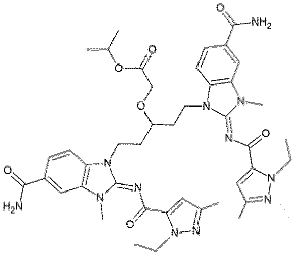 |
| 33 | 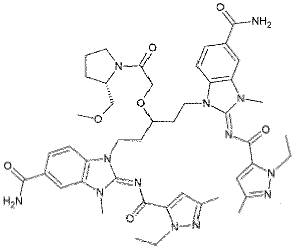 |
| 34 | 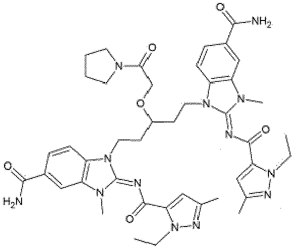 |
| 35 | 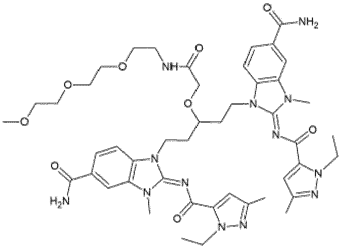 |
| 36 | 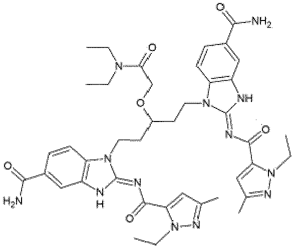 |

48. The compound of any one of the preceding claims, or a tautomer, or a pharmaceutically acceptable salt thereof, for use in a method of treatment of a disorder or condition in a subject, the method comprising administering a therapeutically effective amount of the compound to a subject in need thereof.

49. The compound for the use according to claim 48, wherein the disorder or condition is associated with overactivation of STING.

50. The compound for the use according to claim 48 or claim 49, wherein the disorder or condition is a monogenic autoinflammatory syndrome.

51. The compound for the use according to claim 50, wherein the disorder or condition is STING-associated vasculopathy with onset in infancy (SAVI), Aicardi-Goutieres Syndrome, COPA Syndrome, Familial Chilblain Lupus, DNase II deficiency, or TREX1 deficiency.

52. The compound for the use according to claim 48 or claim 49, wherein the disorder or condition is an autoimmune disease.

53. The compound for the use according to claim 52, wherein the disorder or condition is systemic lupus erythematosus (SLE) or Sjögren's syndrome.

54. The compound for the use according to claim 48 or claim 49, wherein the disorder or condition is an inflammatory disease.

55. A compound according to any one of claims 1 to 47 for use as a medicament.

56. Use of a compound according to any one of claims 1 to 47 for the manufacture of a medicament for the treatment of a disease or condition a subject in need thereof.

57. A method of treatment of a disease or condition in a subject comprising administering a therapeutically effective amount of a compound according to any one of claims 1 to 47 to a subject in need thereof.
